# FASCICULE DE BREVET EUROPEEN

(11) **EP 0 742 284 B1**
(45) Date de publication et mention de la délivrance du brevet: **17.07.2002**
(21) Numéro de dépôt: 96440034.5
(22) Date de dépôt: 29.04.1996
(51) Int. Cl.: C12M 1/34, C12M 1/26

(54) **Procédé d'identification bactérienne et de détermination de la sensibilité de bactéries à des antibiotiques et appareil et supports de mesure pour la mise en oeuvre de ce procédé**
Verfahren zur Identifizierung von Bakterien und zur Bestimmung von deren Empfindlichkeit gegen Antibiotika sowie Vorrichtung und Messträger dafür
Method for bacterial identification and determination of bacterial sensitivity to antibiotics as well as the apparatus and measurement device used

(30) Priorité: 12.05.1995 FR 9505817
(43) Date de publication de la demande: 13.11.1996
(73) Titulaire: PASTEUR SANOFI DIAGNOSTICS, 92430 Marnes La Coquette (FR)
(72) Inventeur: Bajard, Jean, 67370 Griesheim-sur-Souffel (FR)
(74) Mandataire: Nuss, Pierre

(56) Documents cités:
- EP-A- 0 211 334
- US-A- 3 832 532
- US-A- 4 812 411
- US-A- 5 314 825

## Description

La présente invention concerne le domaine de la microbiologie et plus particulièrement des analyses bactériologiques, notamment l'identification automatique de bactéries et la détermination automatique de la résistance de bactéries à différents antibiotiques, ou antibiogramme automatique, et a pour objet un procédé d'identification bactérienne et de détermination de la sensibilité des bactéries à des antibiotiques.

La présente invention a pour but, en partant d'une colonie, notamment d'un inoculum ou suspension bactérienne, d'effectuer, d'une part, une identification de bactéries à partir d'un ensemble de caractères mesurés par photométrie, ainsi que, d'autre part, des essais de croissance en présence d'un nombre optimum d'antibiotiques pour constater par néphélémétrie leur action sur lesdites bactéries, en vue de l'obtention d'un antibiogramme.

A cet effet, il est intéressant de réaliser, lors d'un même essai, un maximum de mesures avec des antibiotiques différents.

L'inoculum est une suspension de bactéries associée à un échantillon et à un patient. Sa préparation s'effectue manuellement par transfert dans une solution aqueuse d'une ou de plusieurs colonies cultivées dans un milieu de culture sous forme d'un gel ou d'éléments préparés à partir d'urine ou d'une hémoculture. En effet, après détection de présence de bactéries par contrôle, par exemple de la variation du CO₂ au-dessus d'une culture de l'échantillon à analyser, il est procédé à un repiquage de ce dernier dans une deuxième culture sous forme d'un gel, ce repiquage ayant pour effet un isolement d'un type spécifique de bactéries au détriment des autres et permettant l'obtention d'une souche monobactérienne. Cette souche est utilisée ensuite pour réaliser l'inoculum par mise en suspension dans une solution aqueuse.

Généralement, les bactéries en suspension dans les inoculums présentent une virulence relativement réduite, due au traitement de préparation, ce qui entraîne des difficultés pour une expérimentation rapide, les différentes mises en culture avec les réactifs se développant relativement lentement.

On connaît actuellement différents procédés et dispositifs permettant la réalisation d'un antibiogramme mettant en oeuvre en tant que support où s'effectuent les mesures, soit des récipients transparents individuels de réception de culture bactérienne (FR-A-2 091 133), soit des récipients transparents multiples disposés sous forme d'alvéoles sur des plaques planes (FR-A-2 449 891, EP-A-0 253 685, DE-A-25 21 025 et US-A-3 942 899).

Ces procédés et dispositifs consistent généralement à déplacer le ou les supports de mesure devant une source de rayonnement lumineux, et à mesurer dans une ou plusieurs directions l'intensité lumineuse du faisceau après qu'il ait traversé le ou les récipients et leur contenu.

L'alimentation du ou des récipients en solution bactérienne est effectuée de manière connue, soit au moyen de pipettes manuelles, soit au moyen d'un dispositif de pipetage automatique selon EP-A-0 522 602, les différents antibiotiques étant mis en place manuellement dans les différents récipients avant leur remplissage.

Par ailleurs, FR-A-2 354 554 décrit un récipient compartimenté tubulaire permettant la dispersion automatique d'une culture bactérienne dans différents compartiments périphériques. Ces compartiments périphériques peuvent être munis chacun, préalablement à leur alimentation en culture bactérienne, d'antibiotiques disposés dans leur fond sous forme de réactif déshydraté.

On connaît également, par FR-A-2 280 895, un dispositif de fractionnement adapté à une utilisation en néphélémétrie ou en photométrie, se présentant sous forme d'un disque muni sur sa périphérie d'alvéoles de réception d'un réactif, sous forme solide et d'une solution bactérienne, la solution bactérienne étant transférée à partir d'une chambre centrale vers les différentes alvéoles, par centrifugation.

Ces différents procédés et dispositifs connus permettent, par séries de mesures distinctes, au moyen de contenants distincts, d'effectuer des mesures en photométrie et en néphélémétrie.

Cependant, ces dispositifs et procédés connus ne permettent pas d'effectuer rapidement et successivement ces mesures sans intervention manuelle, ni avec une alimentation automatique des supports de mesure à partir d'un même contenant, tout en autorisant également la réalisation d'opérations simultanées d'identification et d'antibiogramme, même à partir d'un inoculum initial très peu concentré.

La présente invention a pour but de pallier ces inconvénients en proposant un procédé et des moyens permettant des identifications et des déterminations automatiques sans intervention manuelle sur les milieux.

A cet effet, elle a pour objet un procédé d'identification bactérienne et de détermination de la sensibilité de bactéries à des antibiotiques, sous forme d'antibiogramme, caractérisé en ce qu'il consiste à introduire manuellement, au moyen d'un outil de prélèvement et de transfert, un volume donné de colonie bactérienne dans un récipient primaire, à réaliser automatiquement la dispersion de cette colonie bactérienne au sein d'un liquide pour constituer un inoculum au moins précalibré dans ledit récipient primaire, à effectuer également automatiquement des transferts totaux ou partiels de cet inoculum précalibré entre ledit récipient primaire et un ou plusieurs supports de mesure destinés à réaliser, respectivement, soit l'identification, soit l'antibiogramme ou encore ces deux déterminations simultanément en une seule opération combinée, lesdits transferts étant réalisés sans que l'inoculum précalibré soit mis en contact avec un élément autre que, d'une part, un outil de prélèvement et de transfert et/ou le récipient primaire et, d'autre part, son ou ses supports finaux et de telle manière que les quantités de bactéries transférées correspondent aux quantités requises pour les analyses à réaliser, à effectuer automatiquement la répartition de l'inoculum précalibré, éventuellement après l'avoir convenablement dilué pour aboutir à une calibration définitive, dans une ou plusieurs alvéoles desdits supports de mesure contenant pour la plupart des réactifs appropriés, à réaliser, dans certains cas, notamment pour l'antibiogramme, à l'intérieur d'un support de mesure, une opération dite de pré-croissance destinée à mettre les bactéries en état de se multiplier rapidement avant de les soumettre à analyse et à faire des mesures sur le contenu desdites alvéoles pendant ou à la fin d'une ou plusieurs incubations subies par l'inoculum dans lesdits supports de mesure, les mesures relevées étant enregistrées par un moyen informatique et traitées en vue de la caractérisation de la croissance des bactéries présentes dans l'inoculum, de leur identification et/ou de la détermination de leur sensibilité à différents antibiotiques.

L'invention a également pour objet des appareils et des supports de mesure, sous forme de consommables, pour la mise en oeuvre de ce procédé et de variantes de réalisation de ce dernier, qui seront décrits en détail en regard des dessins énumérés ci-après.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :
la figure 1 est une vue de dessus de l'intérieur d'un appareil pour la mise en oeuvre du procédé conforme à l'invention, selon un premier mode de réalisation ;
la figure 2 est une vue en élévation latérale et en coupe de l'appareil suivant la figure 1 ;
la figure 3 est une vue en élévation latérale et en coupe d'un support de mesure selon l'invention ;
la figure 4 est une vue en perspective et partiellement en coupe d'un support de mesure représenté à la figure 3 ;
la figure 5 est une vue de détail à une échelle différente d'une partie du support de mesure représenté à la figure 4 ;
la figure 6 est une vue simplifiée de dessus et par transparence d'un support de mesure selon l'invention ;
les figures 6A et 6B sont des vues de détail et en perspective, à une échelle différente, de deux parties distinctes du support de mesure représenté à la figure 6 ;
la figure 7 est une demi-vue en coupe transversale et en élévation latérale d'un support de mesure monté dans un poste de mesure et de centrifugation ;
la figure 8 est une vue partielle en coupe et en perspective d'un support de mesure monté dans un poste de mesure et de centrifugation ;
la figure 9 est une vue en perspective et partiellement en coupe d'un couvercle formant la paroi supérieure d'un support de mesure représenté à la figure 3 ;
la figure 9A est une vue en détail, selon une autre perspective, d'une partie du couvercle représenté à la figure 9 ;
la figure 10 est une vue simplifiée en perspective d'un corps principal faisant partie du support de mesure représenté à la figure 3 ;
la figure 11 est une vue en élévation latérale et en coupe d'un ensemble récipient primaire / outil de prélèvement et de transfert ;
la figure 12 est une vue en coupe d'un outil de prélèvement et de transfert ;
les figures 13 et 14 sont des vues respectivement en élévation latérale et en perspective d'un outil de prélèvement et de transfert ;
les figures 15A à 15J montrent différentes phases de manipulation successives de la préparation d'un inoculum précalibré dans le récipient primaire et de son transfert au moins partiel dans un support de mesure ;
la figure 16 est une vue en élévation et en coupe d'un appareil pour la mise en oeuvre d'une variante de réalisation du procédé conforme à l'invention ;
la figure 17 est une vue en plan et en coupe, au niveau inférieur, de l'appareil suivant la figure 16 ;
la figure 18 est une vue analogue à celle de la figure 17 de l'appareil au niveau supérieur ;
la figure 19 est une vue en perspective éclatée d'un récipient primaire selon une variante de réalisation de l'invention, correspondant à l'appareil de la figure 16 ;
la figure 20 est une vue en perspective par le dessous du récipient primaire de la figure 19 ;
les figures 21 à 23 sont des vues schématiques en coupe représentant le récipient primaire de la figure 19 pendant les étapes successives de préparation de l'inoculum ;
la figure 24 est une vue en perspective éclatée, partiellement arrachée, d'un support de mesure pour l'identification destiné à être mis en oeuvre dans l'appareil des figures 16 à 18 ;
la figure 25 est une vue en coupe du support de mesure de la figure 24 pour l'identification muni d'un récipient primaire associé ;
la figure 26 est une vue analogue à celle de la figure 24 représentant un support de mesure pour antibiogramme destiné à être mis en oeuvre dans l'appareil des figures 16 à 18 ;
la figure 27 est une vue en perspective partiellement arrachée du support suivant la figure 26 avant inoculation ;
la figure 28 est une vue analogue à celle de la figure 25 représentant le support de mesure pour antibiogramme en position de répartition de l'inoculum dans les alvéoles, et
la figure 29 est une vue en perspective et en coupe d'une variante de réalisation du récipient primaire de la figure 19 pour le traitement d'un matériel bactérien de faible volume, sans calibration automatique.

Conformément à l'invention, le procédé d'identification bactérienne et de détermination de la sensibilité de bactéries à des antibiotiques, sous forme d'antibiogramme, consiste à introduire manuellement, au moyen d'un outil 101' de prélèvement et de transfert, un volume donné de colonie bactérienne dans un récipient primaire 1 ; 101 à réaliser automatiquement la dispersion de cette colonie bactérienne au sein d'un liquide pour constituer un inoculum au moins précalibré dans ledit récipient primaire 1 ; 101, à effectuer également automatiquement des transferts totaux ou partiels de cet inoculum précalibré entre ledit récipient primaire 1 ; 101 et un ou plusieurs supports de mesure 4, 5 ; 104, 105 destinés à réaliser, respectivement, soit l'identification, soit l'antibiogramme ou encore ces deux déterminations simultanément en une seule opération combinée, lesdits transferts étant réalisés sans que l'inoculum précalibré soit mis en contact avec un élément autre que, d'une part, un outil de prélèvement et de transfert 101'; 1, 101 et/ou le récipient primaire 1 ; 101 et, d'autre part, son ou ses supports de mesure finaux 4, 5 ; 104, 105 et de telle manière que les quantités de bactéries transférées correspondent aux quantités requises pour les analyses à réaliser, à effectuer automatiquement la répartition de l'inoculum précalibré, éventuellement après l'avoir convenablement dilué pour aboutir à une calibration définitive, dans une ou plusieurs alvéoles 32, 37, 45 ; 132, 137, 145 desdits supports de mesure 4, 5 ; 104, 105 contenant pour la plupart des réactifs appropriés, à réaliser, dans certains cas, notamment pour l'antibiogramme, à l'intérieur d'un support de mesure 4 ; 104 une opération dite de pré-croissance destinée à mettre les bactéries en état de se multiplier rapidement avant de les soumettre à analyse et à faire des mesures sur le contenu desdites alvéoles 32, 37, 45 ; 132, 137, 145 pendant ou à la fin d'une ou plusieurs incubations subies par l'inoculum dans lesdits supports de mesure 4, 5 ; 104, 105, les mesures relevées étant enregistrées par un moyen informatique 8 ; 108 et traitées en vue de la caractérisation de la croissance des bactéries présentes dans l'inoculum, de leur identification et/ou de la détermination de leur sensibilité à différents antibiotiques.

Selon un premier mode de réalisation de l'invention, le prélèvement d'un volume donné de colonie bactérienne et les transferts des volumes d'inoculum précalibré dans les supports de mesure 104, 105 sont effectués au moyen d'un outil unique de prélèvement et de transfert 101' coopérant, par exemple par emboîtement étanche, avec le récipient primaire 101, les volumes d'inoculum précalibré à transférer étant déterminés à partir de mesures optiques de la concentration bactérienne dudit inoculum, effectuées dans le récipient primaire 101, et calculés en fonction des quantités de bactéries nécessaires.

Le prélèvement et l'introduction dans le récipient primaire 101 d'un volume donné de colonie bactérienne sont effectués au moyen de l'outil 101' de prélèvement et de transfert après sa mise en conformation en vue de prélever le volume souhaité (sélection d'un volume parmi deux volumes différents disponibles) et les transferts d'inoculum précalibré du récipient primaire 101 vers les supports de mesure 104, 105 sont réalisés par pipetage de volumes d'inoculum prédéterminés.

Le transfert de l'inoculum à l'intérieur des supports de mesure 104, 105, notamment vers les alvéoles 132, 145, est contrôlé dynamiquement ou activement par commande de l'ouverture ou du blocage étanche de passages successifs conduisant progressivement vers lesdites alvéoles 132, 145.

Dans le cadre de la mise en oeuvre de supports de mesure servant à la détermination des antibiogrammes ou à réaliser des déterminations combinées d'antibiogramme et d'identification, la pré-croissance de l'inoculum calibré est réalisée par une première incubation, dite pré-incubation, pendant laquelle les bactéries présentes dans l'inoculum sont incubées sans être mises en présence de réactifs et ce jusqu'à ce qu'un début de croissance bactérienne soit mesurable ou jusqu'à ce que la concentration bactérienne atteigne un niveau prédéfini, cette pré-croissance étant suivie de la mise en contact de l'inoculum avec les réactifs et d'une incubation finale pendant laquelle leurs interactions sont observées.

Conformément à un mode de réalisation pratique avantageux de l'invention, la répartition de l'inoculum dans les alvéoles 32, 37, 45 ; 132, 137, 145 situées en périphérie des supports de mesure 4, 5 ; 104, 105 est réalisée par centrifugation, les mesures et les observations effectuées au niveau desdites alvéoles 32, 37, 45 ; 132, 137, 145 étant effectuées par mise en rotation des supports de mesure 4, 5 ; 104, 105 autour de l'axe central de centrifugation desdits supports.

De plus, il peut être prévu, conformément à l'invention, que la mesure de la concentration de l'inoculum bactérien et la détermination du début de croissance, pendant la pré-croissance dans un support de mesure 4 ; 104 d'antibiogramme, sont réalisées par des mesures photométriques effectuées sur une partie aliquote de l'inoculum transférée à cet effet, par une première centrifugation, dans une alvéole périphérique spécifique 37 ; 137 dédiée à la caractérisation de la croissance pendant la pré-incubation et ne contenant pas de réactifs, le reste d'inoculum restant bloqué pendant la pré-incubation dans une chambre concentrique centrale 27 ; 127 dudit support 4 ; 104, avant d'être transféré, après achèvement de la pré-croissance dans les autres alvéoles périphériques 32 ; 132 par une deuxième centrifugation, précédée d'une action d'autorisation ou de déblocage appropriée ouvrant le passage pour le transfert du reste de l'inoculum vers lesdites autres alvéoles 32 ; 132 contenant des réactifs.

En vue d'éviter toute contamination de l'inoculum calibré ou non présent dans le support de mesure d'antibiogramme 104, le passage de l'étape de pré-incubation à l'étape d'incubation est effectué sans rupture du confinement de l'intérieur du support rotatif de mesure concerné 104 par rapport à l'extérieur, ledit support restant clos et ledit passage étant réalisé par interruption d'une action sur un élément obturateur participant à la réalisation de ce confinement.

Selon un mode de réalisation préférentiel de l'invention, les mesures photométriques sont réalisées par néphélémétrie pour les supports de mesure 4 ; 104 servant à l'antibiogramme et par détermination d'absorbance ou de densité optique pour les supports de mesure 5 ; 105 servant à l'identification ou aux déterminations combinées identification/antibiogramme. Il est également possible, par addition d'un marqueur fluorogène au réactif, de réaliser des mesures fluorimétriques.

L'invention a également pour objet un support de mesure en forme de disque, pouvant notamment être avantageusement mis en oeuvre dans le cadre du procédé décrit ci-dessus, mais qui peut également trouver une application plus générale dans d'autres procédés d'analyse ou d'identification mettant en oeuvre des supports de mesure rotatifs et réalisant des transferts contrôlés des substances à analyser à l'intérieur desdits supports de mesure.

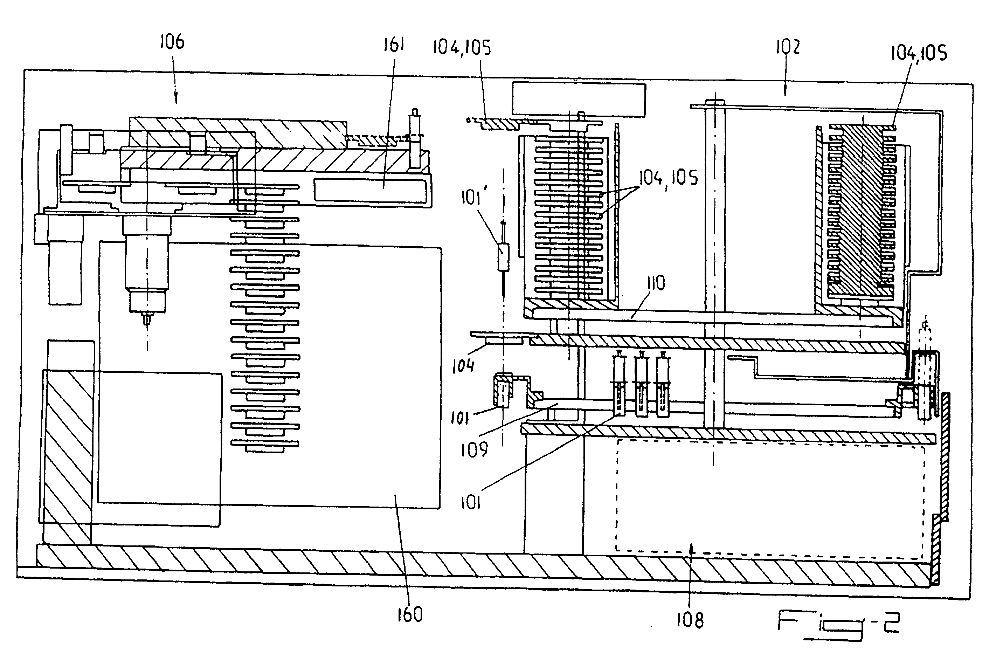

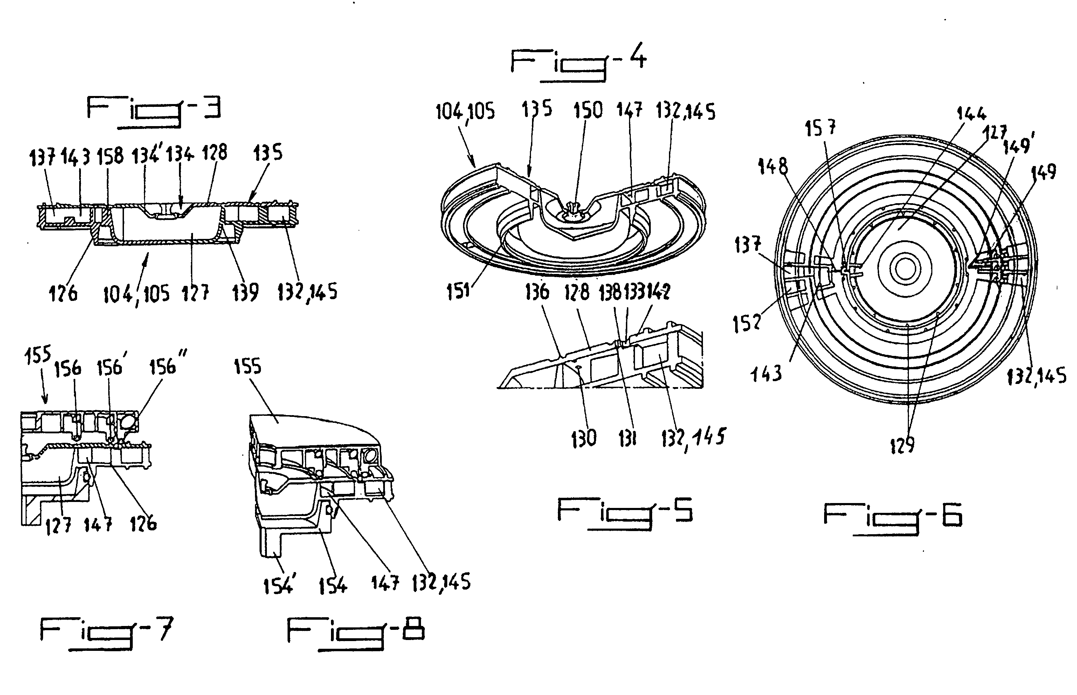

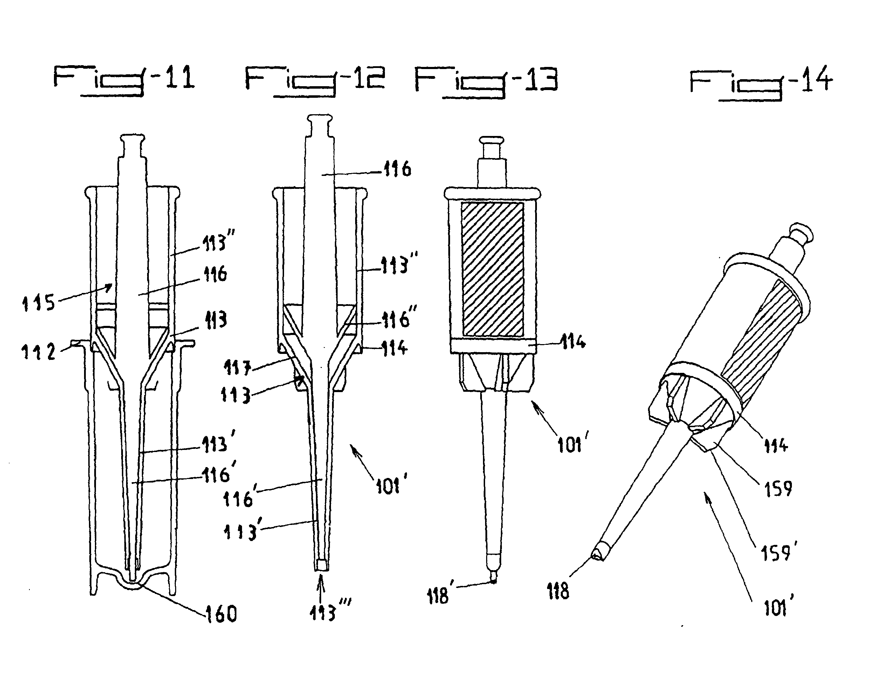

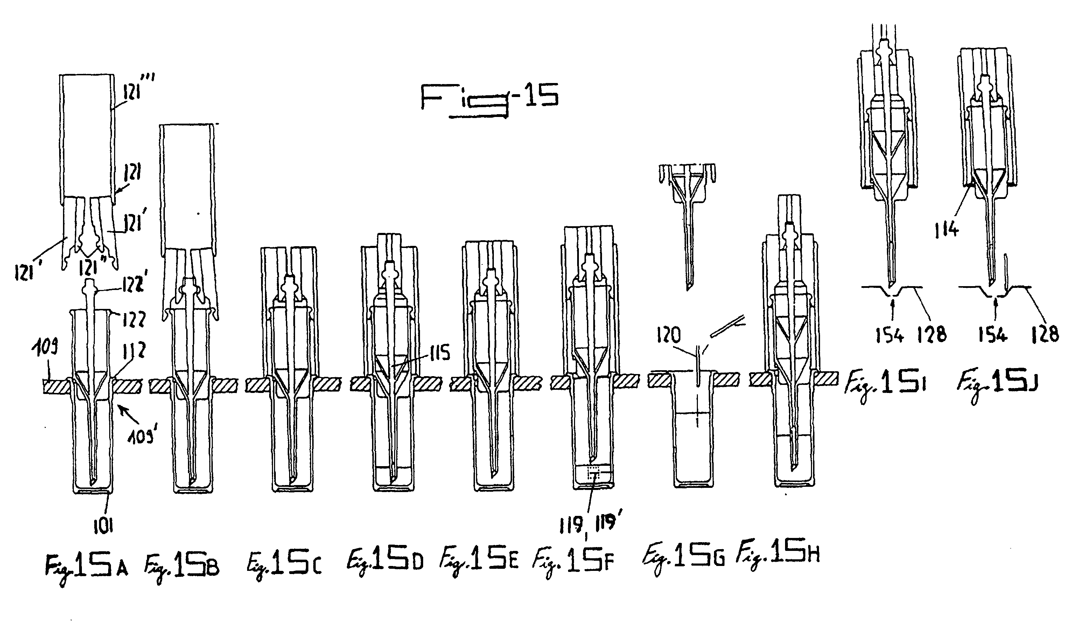

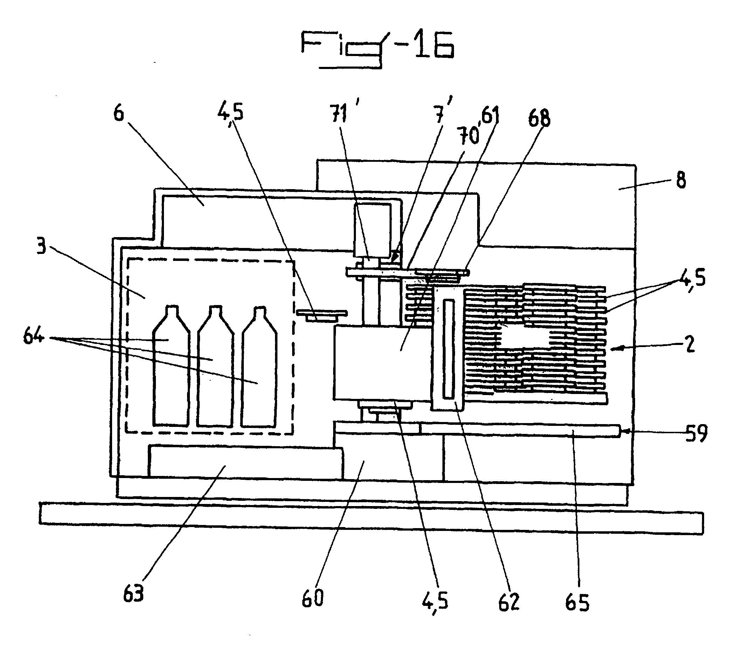

Comme le montrent les figures 3 à 15 des dessins annexés, le support de mesure 104, 105 comprend un corps principal rigide 126 délimitant, sous forme de compartiments non communiquants, une chambre centrale 127 et plusieurs alvéoles périphériques 132, 145 associés, chacune, à une niche de captage 146.

Conformément à l'invention, le corps principal 126 présente une paroi supérieure 128 pourvue de plusieurs ensembles d'orifices de transfert traversants 129, 130, 131, 133 débouchant respectivement dans la chambre centrale cylindrique 127, dans chaque niche de captage 146 affectée à chaque alvéole 132, 145 et dans chacune desdites alvéoles 132, 145 et recouverte, à l'exception d'une portion centrale entourant l'orifice de chargement 134 de ladite chambre centrale 127 et de la zone des fenêtres de mesure des alvéoles 132, 145, par une membrane flottante étanche affleurante 135 fixée localement et hermétiquement notamment à proximité de la périphérie externe du support de mesure et autour de l'orifice de chargement 134 de ladite paroi supérieure 128 et pouvant être pressée intimement contre la surface supérieure de ladite paroi 128 pour former des zones de contact temporaires étanches s'étendant entre ou recouvrant certains desdites ensembles d'orifices traversants 129, 130, 131, 133, notamment au cours de la rotation dudit support de mesure.

On notera que, grâce à cette disposition, le transfert de la substance à analyser ou à identifier, telle qu'un inoculum bactérien, injectée initialement dans la chambre centrale 127, vers les alvéoles de mesure 132, 145 situées en périphérie dudit support 104, 105, peut être réalisé progressivement et de manière parfaitement contrôlée en fonction des opérations successives à effectuer.

Selon un mode de réalisation préférentiel de l'invention, la paroi supérieure 128 comporte, au niveau de sa face supérieure, des rainures ou des renfoncements annulaires concentriques 140, 140' dans lesquel(le)s débouchent les orifices traversants 129, 130, 131, 133 disposés selon des cercles concentriques espacés, la rainure intérieure 140 comprenant une nervure 136 formant surface de contact annulaire et séparant les orifices de sortie 129 de la chambre centrale 127 et les orifices d'entrée 130 de la chambre concentrique annulaire 147 comprenant les niches radiales de captage 146, et les orifices de sortie 131 des niches de captage 146 débouchant au niveau de la face supérieure d'une nervure circulaire concentrique ou de structures proéminentes distinctes 138, par exemple sous forme de dômes, disposée(s) dans la rainure extérieure 140', les extrémités supérieures de la nervure 136 et des structures 138 étant situées dans un plan ou des plans s'étendant légèrement en retrait du plan de collage de la membrane étanche affleurante 135.

La prévision de points ou de zones d'appui de la membrane 135, proéminent(e)s par rapport à la surface avoisinante des fonds de rainures annulaires 140, 140' ménagées dans la face supérieure de la paroi supérieure 128, mais s'arrêtant au moins légèrement en dessous du plan de collage de la membrane affleurante 135, et conformés avec une face supérieure restreinte et délimités par rapport à ladite surface avoisinante, permet d'obtenir une étanchéité reproductible et augmentée, de localiser précisément les zones de contact étanches et d'aboutir à une résistance accrue de ces dernières à la pression exercée par la substance ou l'inoculum lors de la rotation des supports de mesures 104, 105, lesdites zones de contact étant situées aux sommets d'élévation formant obstacles à la progression centripète de la substance ou de l'inoculum.

En vue de faciliter les transferts et le vidage complet de la chambre centrale 127 et des niches de captage 146, il peut être prévu que la chambre centrale 127 comporte une paroi latérale périphérique 139 au moins légèrement inclinée vers l'extérieur en direction de la paroi supérieure 128, les orifices de sortie 129 de la chambre 127 centrale et les orifices de sortie 131 des niches de captage et d'équirépartition 146 étant situés sensiblement dans l'alignement des faces internes des parois latérales périphériques 139', 146' de ces dernières.

Afin de diriger préférentiellement le flux de substance ou d'inoculum vers les orifices d'entrée au cours de la rotation des supports de mesure 104, 105, lesdits orifices d'entrée 130 de la chambre concentrique 147 comprenant les niches de captage et d'équirépartition 146 débouchent sur la face supérieure de la paroi supérieure 128 au pied d'un décrochement 141 délimitant latéralement et du côté externe le renfoncement annulaire 140 interne et les orifices d'entrée 133 des alvéoles de mesure 132, 145 débouchent et les orifices d'entrée 133 des alvéoles à la base ou dans un flanc incliné 141' d'un décrochement circulaire débouchant sur une surface proéminente extérieure 142 pouvant former une zone de contact ou de collage externe de la membrane flottante affleurante 135.

Cette dernière présente, par exemple, une structure composite comprenant, par exemple, une couche externe en papier (servant de support d'impression), une couche intermédiaire en aluminium et une couche interne de colle thermofusible.

La fixation de la membrane 135 au niveau de ses zones de solidarisation avec la paroi supérieure 128 peut alors être réalisée en appuyant intimement ladite membrane 135 sur ladite paroi 128 au niveau desdites zones et en induisant un courant de chauffage dans la couche en aluminium, qui aboutit à la fusion de la couche de colle interne.

La nervure 136 et les structures saillantes ou proéminentes 138 présentent des hauteurs telles que leurs faces supérieures sont situées légèrement en dessous du plan de collage de la membrane 135, il en résulte que cette dernière n'adhérera pas à leur niveau lors de la fusion de la couche de colle thermofusible interne.

Conformément à une autre caractéristique de l'invention, représentée notamment aux figures 3, 6, 6A et 10 des dessins annexés, le support de mesure 104, 105 peut comporter une alvéole de pré-incubation 137 identique aux autres alvéoles périphériques 132, 145, alignée circulairement avec ces dernières et communiquant avec une pré-chambre close 143 formée dans la chambre concentrique 147 renfermant les niches de captage et d'équirépartition 146 et alimentée en inoculum à partir d'une niche de captage interne 144 située au niveau d'une portion de la paroi latérale périphérique 139 de la chambre centrale 127, par l'intermédiaire d'un passage non obturable par les zones de contact étanches 136, 138, 142 pouvant être formées entre la membrane affleurante 135 et la paroi supérieure 128.

De manière avantageuse, le passage d'alimentation de la pré-chambre 143, communiquant librement avec la chambre de pré-incubation 137 et formant également trop-plein pour les niches de captage 146, est constitué par un conduit 157, débutant dans la partie supérieure de la portion de paroi latérale périphérique 139 délimitant la niche de captage interne 144, formé par coopération d'une rainure ménagée dans la partie supérieure d'un prolongement saillant radial 158 de la paroi latérale périphérique 139 de la chambre centrale 127 et de la paroi supérieure 128 et débouchant dans ou à l'entrée d'une rainure 148 à extension radiale centripète, creusée dans la face supérieure de la paroi supérieure 128, formant un conduit étanche en coopération avec la membrane (135) contrecollée de part et d'autre de ladite rainure 148, dont l'extrémité distale se termine au niveau d'un orifice traversant 148' débouchant dans la préchambre 143.

La préchambre 143 fait saillie dans la chambre concentrique annulaire 147 d'une distance sensiblement égale à la longueur des portions de paroi délimitant les niches de captage 146 et présente, au niveau de la partie supérieure de sa paroi parallèle à la paroi latérale périphérique 139 de la chambre centrale 127, un orifice capillaire très fin 143' formant passage d'évacuation du trop plein durant la phase d'équirépartition.

Bien que les figures 6 et 10 notamment ne représentent qu'un nombre limité d'alvéoles 132, 145, il est bien évidemment entendu que ces dernières sont disposées sur toute la périphérie des supports de mesure 104 et 105 et sont, par exemple, au nombre d'environ cinquante.

Conformément à un mode de réalisation préférentiel du support de mesure 104, 105, la paroi supérieure 128 peut consister en un couvercle rapporté, fixé par emboîtement et collage ou soudure au niveau de la paroi périphérique externe du corps principal 126 et comportant des orifices supplémentaires 149 et des rainures superficielles radiales 149' assurant le retour d'air au cours des transferts de la substance à analyser injectée dans la chambre centrale 127 vers la périphérie dudit support 104, 105.

Une telle réalisation du support 104, 105 permet de fabriquer indépendamment ses trois composantes, à savoir le corps principal 126, le couvercle 128 et la membrane 135, puis de les assembler de manière adaptée, le corps principal et le couvercle 128 étant obtenus, par exemple, par moulage par injection d'un matériau thermoplastique, préférentiellement transparent.

Dans le cas d'une mise en oeuvre de deux supports de mesure rotatifs 104, 105 de natures différentes, tels que des supports rotatifs 104 pour antibiogramme et des supports rotatifs 105 pour l'identification, ces derniers pourront présenter la même constitution et se différencier uniquement par la nature des réactifs disposés dans leurs alvéoles de mesure respectives 132, 145 et par les manipulations dont ils feront l'objet au cours de leur mise en oeuvre (prévision ou non d'une phase de préincubation).

Une telle similarité de structure des supports de mesure 104, 105, destinés à des applications différentes, facilite sensiblement la standardisation de leur fabrication, aboutit à un prix de revient plus faible que pour des supports différenciés, facilite leur manipulation par l'appareil de traitement et d'analyse et permet de simplifier la constitution de ce dernier.

Comme le montrent notamment les figures 3 et 4 des dessins annexés, l'orifice de chargement 134 de la chambre centrale 127 est situé dans un renfoncement 134' de la paroi supérieure 128 et est obturable au moyen d'un bouchon 150 dont la partie supérieure est sensiblement de niveau avec la face supérieure de la paroi supérieure 128.

Ainsi aucune partie ne dépassera du plan contenant la face supérieure de la paroi supérieure 128.

Le renfoncement 134' pourra être délimité latéralement par des parois formant une structure cylindrique tronconique dirigé vers l'orifice de chargement 134 (figures 3 et 4).

En vue de la manipulation et du stockage par empilement des supports de mesure 104, 105, le fond de la chambre centrale 127 est entouré d'un manchon concentrique 151 de préhension et de rigidification, saillant au niveau de la face inférieure du support 104, 105. De plus, lesdits supports pourront également comporter des nervures de rigidification et de guidage au niveau de leurs alvéoles périphériques.

En outre, afin de faciliter le réglage et le calage des dispositifs de mesures photométriques, les supports de mesures 104, 105 peuvent comporter, chacun, une ouverture traversante 152, ayant des dimensions identiques à celles d'une alvéole 132, 145, située en alignement circulaire avec ces dernières et qui est ménagée dans le corps principal 126 et dans la paroi supérieure 128, ladite ouverture 152 étant préférentiellement située adjacente à l'alvéole de pré-incubation 137, de manière à permettre un repérage aisé de cette dernière.

Enfin, la paroi supérieure 128 est munie, au dessus de chaque alvéole 132, 137, 145 et près de son bord extérieur, d'une fenêtre de mesure, une fenêtre analogue étant prévue, dans chacune des portions de paroi du corps principal 126 constituant respectivement les fonds desdites alvéoles, en regard de chaque fenêtre de mesure précitée et au moins une zone réfléchissante d'indexation, formée sur la face externe de la paroi périphérique externe du corps principal 126, étant associée à chaque alvéole 132, 137, 145 avec un décalage angulaire fixe par rapport à cette dernière.

Les zones réfléchissantes inclinées créent des repères optiques décalés pour les différentes alvéoles de mesure 132, 137, 145, et permettent un centrage guidé des dispositifs de mesures photométriques au niveau de chacune desdites alvéoles.

Ainsi, les supports de mesure 104, 105 comprennent un corps fermé compartimenté (corps principal 126 + paroi supérieure 128) percé en des emplacements déterminés par des orifices 129, 130, 131, 133 de faible dimension et surmonté d'une membrane étanche souple affleurante 135, permettant par action contrôlée sur ladite membrane et dans le cadre du procédé décrit précédemment :
- soit de conserver la substance à analyser au centre du support de mesure considéré, pendant une phase d'homogénéisation et le cas échéant, de pré-incubation,
- soit de garder la substance à analyser dans la zone de la chambre concentrique annulaire 147 et des niches radiales de captage 146, en isolant celles-ci des alvéoles de mesure 132, 145, pendant la phase d'équirépartition de la substance à analyser par centrifugation, cette dernière pouvant être réalisée pendant la durée nécessaire à l'obtention d'une équirépartition parfaite de la substance à analyser dans lesdites niches, moyennant une fonction de trop-plein assurée par la préchambre 143 et l'alvéole de pré-incubation 137 par l'intermédiaire de l'orifice capillaire 143',
- soit d'ouvrir le passage entre les niches radiales de captage 146 et les alvéoles de mesure 132, 145, de manière à réaliser le transfert de la substance à analyser desdits niches vers lesdites alvéoles au cours d'une opération de centrifugation.

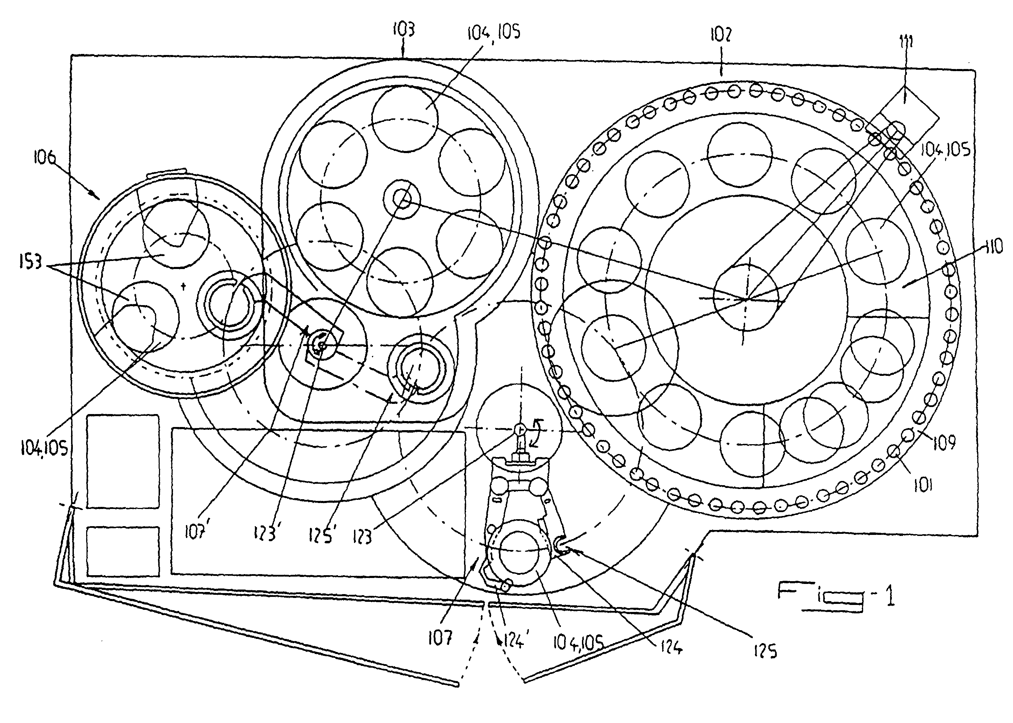

L'invention a également pour objet un appareil pour la mise en oeuvre du procédé décrit ci-dessus, représenté aux figures 1 et 2 des dessins annexés, ledit appareil étant essentiellement constitué par un premier module 102 de support et de stockage de supports de mesure 104, 105 frais, tels que décrits précédemment, et de récipients primaires 101 coopérant chacun avec un outil de prélèvement et de transfert 101' correspondant, par un deuxième module 103 d'incubation des supports 104 et 105, après injection de l'inoculum bactérien, par un troisième module 106 de centrifugation et de mesures néphélémétriques et/ou photométriques et/ou fluorimétriques, par un ou plusieurs dispositifs 107, 107' de transfert desservant lesdits modules 102, 103 et 106 et par un moyen informatique 108 de contrôle et de commande des différentes étapes du procédé, et d'acquisition, d'enregistrement, de traitement, d'évaluation et/ou de restitution de données, les deuxième et troisième modules 103 et 106 étant disposés dans une enceinte de confinement.

Conformément à l'invention, les récipients primaires 101 sont portés par un moyen support 109 en forme de couronne, s'étendant concentriquement autour et sous un moyen support 110 en forme de disque portant les supports de mesure 104, 105, les deux moyens supports 109, 110 étant montés avec faculté de rotation autour d'un même axe, chaque récipient primaire 101 pouvant être amené, par simple rotation dudit moyen support 109 en forme de couronne, au niveau d'un poste 111 de manipulation de l'outil 101' de prélèvement et de transfert associé et de mesure de densité optique du contenu dudit récipient primaire 101.

Comme le montrent notamment les figures 11 à 14 des dessins annexés, chaque récipient primaire 101 de réception d'un volume donné de colonie bactérienne et de préparation d'un inoculum au moins précalibré se présente sous la forme d'un récipient cylindrique ouvert au niveau de sa partie supérieure, laquelle est munie d'un rebord radial 112 pour le maintien dudit récipient primaire 101 dans un des logements correspondants 109' ménagé(s) dans le support mobile 109 en forme de couronne.

L'outil de prélèvement et de transfert 101' peut avantageusement consister en une seringue dont le corps 113 comporte une partie avant de prélèvement et d'éjection effilée 113' et une partie arrière 113" de stockage et de préhension sensiblement cylindrique, ladite partie arrière 113" présentant au moins un décrochement radial extérieur 114 au niveau de son extrémité contiguë à la paroi avant 113' en vue d'un emboîtement partiel avec blocage dudit corps 113 dans le récipient primaire 101, l'extrémité libre avant de la partie avant 113' étant alors située à faible distance du fond dudit récipient primaire 101.

La partie arrière 113" peut comporter des prolongements inférieurs 159 sous forme d'ailettes radiales, s'étendant depuis le décrochement 114 et pourvues chacune d'un chanfrein latéral inférieur 159', ces prolongements aidant au centrage de l'emboîtement de l'outil 101' dans le récipient primaire 101 et au blocage de son positionnement radial dans ce dernier.

Selon une caractéristique de l'invention, représentée également sur les figures 11 à 14 des dessins annexés, la seringue 101' comporte, au niveau de son extrémité avant, des moyens 118, 118' pour prélever, en volume, une quantité réduite ou une quantité normale de colonie bactérienne, en fonction du positionnement de son piston 115.

Le piston 115 de la seringue 101' est constitué par une tige d'actionnement effilée 116 dont la partie avant 116' coopère avec la partie avant 113' du corps 113 et peut dépasser de l'ouverture de prélèvement et d'éjection 113"' de ladite partie avant 113' du corps 113 par son extrémité libre, lorsque le piston 115 est totalement enfoncé dans le corps 113, ladite tige d'actionnement 116 présentant un décrochement radial 116" venant buter avec conjugaison de forme sur le fond 117 du volume interne de la partie arrière cylindrique 113" du corps 113, lorsque ledit piston 115 est entièrement enfoncé dans ledit corps 113.

Le fond du récipient primaire 101 pcut présenter, comme le montre la figure 11 des dessins annexés, un renfoncement central 160 pour la réception de l'extrémité libre avant de la tige d'actionnement 116 lorsqu'elle dépasse l'extrémité avant de la partie avant 113' du corps 113.

Afin de pouvoir obtenir dans le récipient primaire 101 un inoculum au moins précalibré par une détermination au moins grossière de la quantité d'éléments de colonie bactérienne prélevée (prélèvement manuel ou micro-prélèvement), fonction notamment du type d'analyse envisagé, les extrémités libres des parties avant 113', 116' du corps 113 et de la tige d'actionnement 116 du piston 115 sont pourvus d'un décrochement ou d'un renfoncement 118, 118' formant des surfaces ou des volumes de prélèvement de quantités déterminées différentes de colonie bactérienne, l'extrémité libre de la partie avant 116' de la tige d'actionnement 116 étant opérationnelle pour le prélèvement lorsque le piston 115 est entièrement enfoncé et l'extrémité libre de la partie avant 113' du corps 113 étant opérationnelle lorsque le piston 115 est au moins légèrement rétracté dans ladite partie avant 113".

L'inoculum précalibré est constitué par injection d'une quantité fixe de liquide de dilution, liquide injecté par un moyen 120.

En vue de déterminer la concentration bactérienne de l'inoculum précalibré, chaque récipient primaire 101 comporte dans sa paroi latérale, à proximité de son fond, deux fenêtres de mesure 119, 119' en regard, adaptées pour la mesure de la densité optique du contenu dudit récipient primaire 101 au niveau du poste 111, ce dernier comportant également le moyen 120 d'injection de liquide de dilution dans ledit récipient primaire 101.

A cela s'ajoute que la quantité d'inoculum prélevée dans le récipient primaire 101 et injectée dans les supports de mesure 104, 105 destinataires peut être aisément réglée, en calculant le volume à transférer compte tenu de la concentration bactérienne mesurée par densité optique, du fait de la mise en oeuvre d'un outil de prélèvement et de transfert 101' sous forme de seringue, manipulé automatiquement par un moyen adapté au niveau du poste 111 notamment.

Ce moyen de manipulation 121 de la seringue 101' peut, par exemple, consister, comme le montrent les figures 15A à 15J des dessins annexés en deux ensembles de mâchoires 121' et 121" comprenant deux mâchoires externes 121' destinées à venir en prise rigide et bloquante avec un rebord radial 128 au niveau de l'ouverture de la partie arrière 113" du corps 113 et deux mâchoires internes 121", disposées concentriquement entre les deux mâchoires externes 121', destinées à venir en prise rigide et bloquante avec un renflement 122' de la tige d'actionnement 116, la fermeture des deux ensembles de mâchoires 121' et 121" étant réalisée séparément ou, de manière préférentielle, simultanément par coulissement d'un manchon 121"' autour et le long des mâchoires externes 121', les mâchoires internes 121" pouvant coulisser par rapport aux mâchoires extemes 121', notamment en position de fermeture, et au manchon 121"' et ce dernier pouvant prendre appui sur le support mobile 109 en forme de couronne et sur le bord radial 112 de l'ouverture supérieure du récipient primaire 101 considéré, de manière à bloquer ce dernier en position dans son logement 109' lors du coulissement de l'un quelconque des deux ensembles de mâchoires 121', 121".

La préparation d'un inoculum au moins précalibré dans un récipient primaire 101 et son transfert au moins partiel et en quantité contrôlée dans un support de mesure 104,105 va à présent être décrit plus en détail en se référant aux figures 15A et 15J des dessins annexés.

Tout d'abord, l'opérateur prélève manuellement au moyen de l'outil 101' un volume déterminé de colonie bactérienne, ce volume étant défini par réglage dudit outil 101' en déplaçant le piston 115 dans une position correspondant à un enfoncement total (dépassement de l'extrémité avant de la partie avant 116' de la tige d'actionnement 116 au niveau de l'ouverture 113"' du corps 113) ou à une légère rétraction de ladite tige d'actionnement. En position rétractée du piston 115, l'outil 101' collecte une quantité de bactéries constituant un prélèvement dit normal, alors qu'en position enfoncée du piston, l'outil 101' collecte un prélèvement dit réduit ou micro-prélèvement (environ 1/100 du précédent).

L'outil 101' portant une quantité déterminée de colonie bactérienne est ensuite emboîté dans un récipient primaire 101 et l'ensemble ainsi formé est disposé dans un logement 109' du moyen support 109.

En début de préparation de l'inoculum, l'outil de prélèvement est saisi par le moyen de manipulation 121 (figures 15A à 15C), et est soulevé par rapport au récipient primaire 101 dans lequel on injecte une quantité déterminée de liquide de dilution.

Puis, l'outil 101' est réemboîté dans le récipient primaire 101 et une homogénéisation de l'inoculum est réalisée dans le récipient primaire 101 par des opérations successives d'aspiration et d'éjection obtenus par coulissement alternatif de l'ensemble de mâchoires internes 121" (figures 15C, 15D et 15E). Après achèvement de cette opération, le récipient primaire 101 contient un inoculum précalibré.

L'outil 101' est ensuite extrait du récipient primaire 101 et une mesure de densité optique est effectuée pour déterminer la concentration bactérienne de l'inoculum précalibré ce qui permet de déterminer, par calcul, le volume d'inoculum à transférer dans les supports de mesure 104, 105 pour y amener la quantité de bactéries nécessaire aux analyses prévues (figures 15F et 15G).

Après une nouvelle phase éventuelle d'homogénéisation, il est procédé à un prélèvement de la quantité ainsi calculée d'inoculum précalibré dans le récipient primaire 101 au moyen de l'outil 101' (figure 15H), ce dernier étant ensuite déplacé par le dispositif de manipulation 121 jusqu'à être positionné au-dessus de ou partiellement dans l'orifice de chargement 134 d'un support de mesure 104, 105 dont le bouchon obturateur 150 a été préalablement enlevé, ledit support 104, 105 étant maintenu par un dispositif de manipulation et de transfert 107 correspondant, qui a extrait ledit support de mesure du moyen de support et de stockage 110.

Le contenu de l'outil 101' est alors injecté dans la chambre centrale 127 du support de mesure 104, 105 considéré, par coulissement adapté de l'ensemble de mâchoires internes 121", aboutissant à un enfoncement total du piston 115 dans le corps 113 et donc à un vidage complet de ce dernier.

Avant remise en place du bouchon obturateur 150, il est procédé à l'injection d'une quantité complémentaire déterminée de liquide de dilution, de manière à obtenir, dans la chambre centrale 127, un inoculum parfaitement calibré en fonction de l'application envisagée et ayant le volume requis.

L'ensemble récipient primaire 101 / outil de prélèvement et de transfert 101' permet ainsi de réaliser les différentes opérations suivantes :
- prélèvement d'une partie de colonie bactérienne, selon une quantité relativement bien ajustée (deux volumes de prélèvement dans un rapport d'environ cent),
- dispersion et homogénéisation automatisée de cette colonie bactérienne dans un volume d'eau pour constituer un inoculum précalibré, dont la concentration bactérienne est supérieure à la concentration souhaitée pour l'inoculum calibré,
- mesure de la concentration de l'inoculum précalibré (nombre de bactéries par unité de volume),
- pipetage d'une partie de l'inoculum précalibré et transfert de cette dernière dans un support de mesure, l'inoculum transféré comprenant le nombre voulu de bactéries, le volume d'inoculum transféré étant complémenté par addition d'eau dans la chambre centrale 127 du support de mesure 104, 105 considéré, pour aboutir à un inoculum calibré.

En vue de la manipulation des supports de mesure 104, 105 et, le cas échéant, de leur évacuation dans un contenant à déchets 160, ainsi que de celle des outils 101' et des récipients primaires 101, l'appareil selon l'invention comporte, d'une part, un premier dispositif de manipulation et de transfert 107, desservant les supports 109 et 110 et se présentant sous la forme d'un bras manipulateur monté avec faculté de rotation et de coulissement sur un premier axe vertical 123 et comprenant deux mâchoires de préhension pivotantes 124, 124' dont la première 124 est munie d'un moyen de prise 125 d'un récipient primaire 101 et dont la seconde 124' présente une forme courbée ou un crochet destiné à venir en prise en deux endroits distincts d'un support de mesure 104, 105, de manière à maintenir ce dernier par soutien inférieur et par pincement en coopération avec la première mâchoire de préhension 124 et, d'autre part, un second dispositif de manipulation et de transfert 107', desservant le module d'incubation 103 et le module 104 de centrifugation et de mesure se présentant sous la forme d'un bras support monté avec faculté de rotation et de coulissement sur un second axe vertical 123' et comportant à son extrémité libre un organe de réception 125' coopérant avec conjugaison de forme avec une partie au moins de la face inférieure des supports de mesure 104, 105, les deux dispositifs de manipulation et de transfert 107, 107' présentant une zone de chevauchement de leurs espaces ou zones de manoeuvre respectivement accessibles, au niveau de laquelle peut notamment s'effectuer le transfert des supports de mesure 104, 105 de l'un à l'autre.

En vue de la préhension d'un récipient primaire 101 par le moyen de prise 125 de la première mâchoire 124, l'ensemble emboîté seringue 101' / récipient primaire 101 est d'abord soulevé par les ou une des paires de mâchoires 121, 121'.

Les supports de mesure 104, 105 chargés en inoculum calibré au niveau de leur chambre centrale 127 sont amenés par le second dispositif de manipulation et de transfert 107' dans l'enceinte de confinement renfermant les deuxième et troisième modules 103 et 106, à travers une ouverture obturable 161.

Durant les phases opératoires suivantes, les supports de mesure 104, 105 sont à plusieurs reprises transférés entre les modules 103 et 106 au moyen du dispositif 107', ce jusqu'à ce que les analyses soient achevées et les résultats enregistrés par l'unité informatique 108.

Les supports de mesure 104, 105 peuvent ensuite, après exploitation, être évacués dans le contenant à déchets 160, en utilisant successivement les deux dispositifs de manipulation et de transfert 107 et 107'.

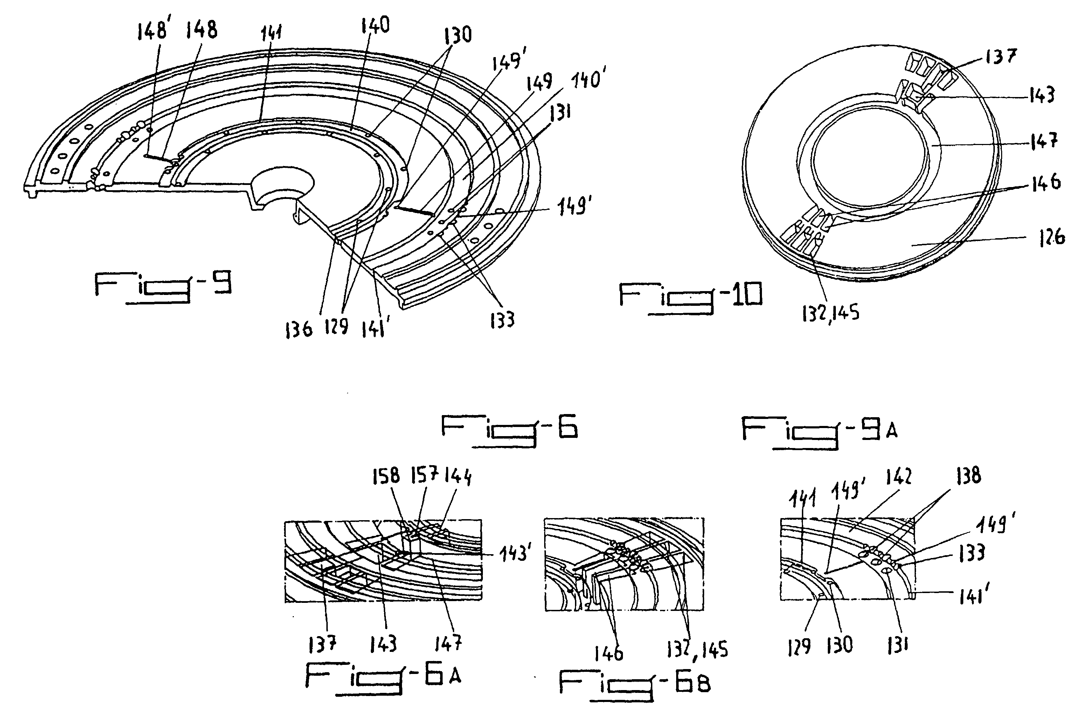

Conformément à une caractéristique de l'invention, représentée partiellement sur les figures 7 et 8 des dessins annexés, le troisième module 106 comporte plusieurs postes de travail 153, notamment de mesure et de centrifugation, comprenant chacun, en plus des différents moyens de mesure par néphélémétrie, par photométrie et/ou par fluorimétrie, d'une part, une embase support 154 en forme de disque, portée par l'extrémité supérieure d'un arbre vertical 154' entraîné en rotation et pouvant s'engager avec enclenchement ou avec maintien par friction radiale dans le manchon concentrique 151 d'un support de mesure 104, 105 et, d'autre part, un dispositif presseur supérieur 155 de forme sensiblement discoïdale et entraîné en rotation autour de son axe de symétrie par friction sur le support 104, 105 disposé dans le poste de travail 153 considéré, chaque dispositif presseur 155 comprenant un ou plusieurs organes presseurs 156, 156', 156" de forme annulaire pouvant être déplacés, de manière réversible, d'une position haute dans laquelle lesdits organes presseurs ne sont pas en contact avec le support de mesure 104, 105 disposé sur l'embase support 154, vers plusieurs positions plus basses dans lesquelles les organes presseurs 156, 156', 156" sont mis successivement en appui sur la membrane flottante affleurante 135 selon un ordre prédéterminé 156", 156', 156, cette dernière prenant elle-même appui au niveau de zones de contact étanches, circulaires ou annulaires, concentriques, ou disposées selon des configurations circulaires ou annulaires concentriques, avec la paroi supérieure 128 du support de mesure 104, 105 considéré.

En fonction du type de poste de travail 153 considéré, le dispositif presseur 155 associé pourra comporter un nombre variable d'organes presseurs 156, 156', 156".

Ainsi dans les postes de mesure 153, l'organe presseur extérieur 156" sera absent du dispositif presseur 155 associé.

La sélection d'une, de deux ou de trois zones d'appui au niveau de la membrane 135, est effectuée en réalisant un déplacement vers le bas plus ou moins important du dispositif presseur 155.

En effet, les organes presseurs extérieurs 156' et 156" consistent en des structures annulaires concentriques et indépendantes du disque central formant l'organe presseur 156, tout en étant reliées à ce dernier par des moyens de liaisonnement élastiques qui entraînent lesdits organes presseurs 156' et 156" en appui contre la membrane 135 lorsque l'organe presseur 155 est moyennement descendu.

Selon l'invention, une variante de réalisation du procédé d'identification bactérienne et de détermination de la sensibilité de bactéries à des antibiotiques, tel que décrit ci-dessus, peut consister à introduire manuellement au moyen d'un outil de prélèvement et de transfert (non représenté) un volume donné d'un inoculum bactérien initial dans un récipient primaire 1, à effectuer automatiquement une calibration définitive dudit inoculum dans ledit récipient primaire 1 en amenant sa concentration bactérienne et son volume à des valeurs imposées, à effectuer également automatiquement des transferts totaux ou partiels de cet inoculum calibré entre ledit récipient primaire 1 et un ou plusieurs supports de mesure 4, 5 destinés à réaliser, respectivement, soit l'identification, soit l'antibiogramme ou encore ces deux déterminations simultanément en une seule opération combinée, lesdits transferts étant réalisés sans que l'inoculum initial soit mis en contact avec un élément autre que son récipient initial et son ou ses récipients finaux et de manière à ce qu'à leur issue l'inoculum calibré reste confiné à l'intérieur desdits supports de mesure 4, 5 qui sont alors clos, à effectuer automatiquement la répartition de l'inoculum dans une ou plusieurs alvéoles 32, 45 desdits supports de mesure 4, 5 contenant pour la plupart des réactifs appropriés, à réaliser, dans certains cas, notamment pour l'antibiogramme, à l'intérieur d'un support de mesure 4, une opération dite de pré-croissance destinée à mettre les bactéries en état de se multiplier rapidement avant de les soumettre à analyse et à faire des mesures photométriques sur le contenu desdites alvéoles 32, 45 pendant ou à la fin d'une ou de plusieurs incubations subies par l'inoculum dans lesdits supports de mesure 4, 5, les mesures relevées étant enregistrées par un moyen informatique 8 et traitées en vue de la caractérisation de la croissance des bactéries présentes dans l'inoculum, de leur identification et/ou de la détermination de leur sensibilité à différents antibiotiques.

La concentration ou densité de l'inoculum préparé manuellement présente une valeur telle qu'elle soit suffisante, c'est-à-dire qu'elle dépasse une valeur minimale pouvant être facilement vérifiée par l'homme du métier. En effet, ce dernier réalisera une solution aqueuse dont il vérifiera la densité par simple constatation de l'aspect de ladite solution, l'expérience lui permettant de constater que la densité ou concentration est suffisante ou non. Une telle préparation de l'inoculum est facilement accessible à l'homme du métier et est destinée à permettre un ajustement ou une calibration ultérieur à une valeur finale inférieure, par dilution.

Selon une première caractéristique de l'invention, la calibration automatique de l'inoculum dans le récipient primaire 1 consiste à réaliser, à partir d'un inoculum initial contenant une quantité de bactéries en excès par rapport aux besoins réactionnels, excès vérifié par observation visuelle de la turbidité de l'inoculum initial, une injection d'eau de dilution dans ledit récipient primaire 1, de manière à créer un volume augmenté d'inoculum plus dilué, puis à éliminer une partie de l'inoculum ainsi dilué par aspiration jusqu'à obtention du volume final d'inoculum calibré requis, ces opérations étant conduites et éventuellement répétées sur la base de mesures optiques faites au travers du récipient primaire 1 sur l'inoculum pour en déterminer la concentration, jusqu'à obtenir à la fois la concentration et le volume recherchés pour l'inoculum calibré.

Conformément à un mode de réalisation préférentiel de l'invention, le transfert de l'inoculum calibré entre le récipient primaire 1 et le ou les supports de mesure 4, 5 est effectué par emboîtement de tout ou partie 11 du récipient primaire 1 contenant l'inoculum dans une cavité prévue à cet effet dans chaque support 4, 5, de manière à y transférer la quantité d'inoculum calibré voulue, contenue dans la partie emboîtée dudit récipient primaire, cet emboîtement assurant l'isolement nécessaire au confinement de l'inoculum dans l'ensemble formé par le support de mesure 4, 5 et le récipient primaire 1 ou la partie 11 du récipient primaire 1 emboîté dans ledit support 4, 5, ainsi que la fermeture de cet ensemble.

En fonctionnement automatique et sans manipulation intermédiaire par un opérateur du récipient primaire 1 et des supports 4 et/ou 5, tous ces éléments sont introduits au départ par l'opérateur dans un premier module de stockage et de préparation 2, dans lequel les supports 4 et/ou 5 sont alimentés en inoculum, chacun par l'intermédiaire d'au moins une chambre de réception prévue dans lesdits supports, à partir du récipient primaire 1 ou directement au moyen de ce dernier.

Après cette alimentation des supports 4 et/ou 5, il est effectué une équirépartition de l'inoculum dans les alvéoles de ces derniers par centrifugation, puis lesdits supports 4 rotatifs et/ou 5 sont transférés dans un deuxième module 3 d'incubation, dont ils sont périodiquement extraits pour être soumis dans le module 6 à des mesures néphélémétriques et/ou photométriques et/ou fluorimétriques en vue de l'identification et/ou de la réalisation d'antibiogrammes.

Dans le cadre de la mise en oeuvre de cette variante de réalisation du procédé selon l'invention, les opérations de précroissance de l'inoculum, de répartition de l'inoculum dans les alvéoles de mesures, de détermination du début de la précroissance, de transfert de l'inoculum après achèvement de la précroissance et de mesures photométriques sur les supports de mesures, sont effectuées d'une manière analogue à celle décrite ci-dessus en regard de la première variante de réalisation, leur mise en oeuvre pratique et les moyens, dispositifs et appareil utilisés étant toutefois différentes, comme va le montrer la description qui suit.

En vue d'éviter toute contamination de l'inoculum calibré ou non présent dans le support de mesure d'antibiogramme 4, le passage de l'étape de pré-incubation à l'étape d'incubation est effectué sans rupture du confinement de l'intérieur du support rotatif de mesure concerné 4 par rapport à l'extérieur, ledit support restant clos et ledit passage étant réalisé par une action, telle qu'une poussée, sur un élément obturateur participant à la réalisation de ce confinement.

En outre, pour les cas où l'inoculum initial ne comporte pas suffisamment de matériel bactérien, il peut être prévu, selon une réalisation légèrement modifiée de la variante de réalisation du procédé décrite ci-dessus, d'omettre l'étape de calibration automatique.

On introduit alors automatiquement un inoculum bactérien initial, mis en place manuellement, au moyen d'un outil de prélèvement et de transfert adapté, dans un récipient primaire 1', dans un support de mesure tel que mentionné ci-dessus et destiné à réaliser soit un antibiogramme, soit une analyse combinée simultanée d'identification et d'antibiogramme, ladite introduction automatique étant réalisée ou achevée de telle manière qu'à son issue le support de mesure soit fermé et isolé de l'extérieur.

Le récipient primaire 1' présentera une conformation extérieure et un encombrement identiques au récipient primaire 1 et comportera un contenant de stockage et de manipulation de l'inoculum bactérien initial appelé macropipette 73 permettant le transfert de son contenu dudit récipient primaire 1' dans le support de mesure d'antibiogramme ou de détermination combinée destinataire.

Après cette introduction de l'inoculum bactérien initial dans ledit support de mesure, le procédé consiste à répartir automatiquement, comme décrit précédemment, une partie aliquote de l'inoculum initial dans une ou plusieurs alvéoles 37, 45, ne contenant pas de réactifs, desdits supports de mesure 4, 5, puis à effectuer à l'intérieur du support de mesure 4, 5 une pré-croissance de cet inoculum initial, comme indiqué précédemment, consistant à mettre les bactéries de l'inoculum en état de se multiplier rapidement par une première incubation ou pré-incubation lors de laquelle des mesures photométriques, visant à mesurer la concentration de l'inoculum bactérien et à déterminer le début de croissance, sont réalisées au niveau de la ou des alvéoles 37, 45 contenant ladite première partie aliquote de l'inoculum, à répartir ensuite automatiquement par centrifugation le reste de l'inoculum dans les autres alvéoles 32, 45 contenant des réactifs, et à effectuer des mesures photométriques sur l'inoculum au niveau desdites alvéoles 32, 45, tel que décrit ci-dessus, pendant une nouvelle phase d'incubation subie par l'inoculum dans lesdits supports de mesure 4, 5, les mesures relevées étant enregistrées par un moyen informatique 8 et traitées en vue de la caractérisation de la croissance des bactéries présentes dans l'inoculum, de leur identification et/ou de la détermination de leur sensibilité à différents antibiotiques.

La variante de réalisation du procédé conforme à l'invention sera à présent décrite de manière détaillée à propos d'un exemple de mise en oeuvre pratique et en relation avec une description des matériels utilisés.

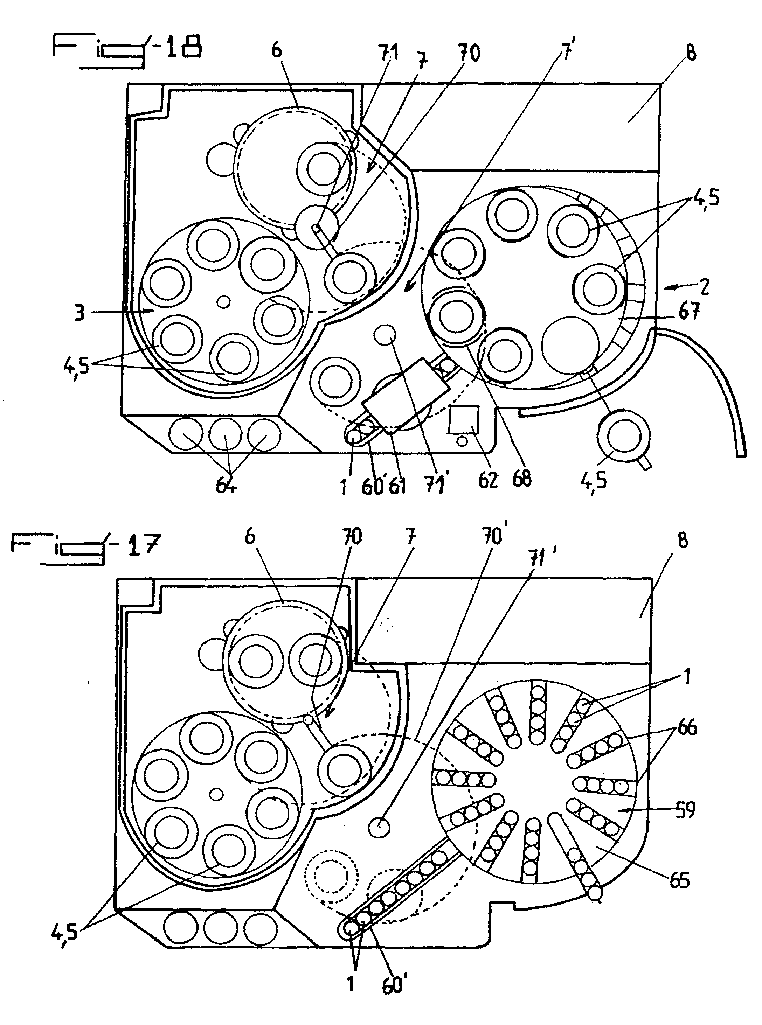

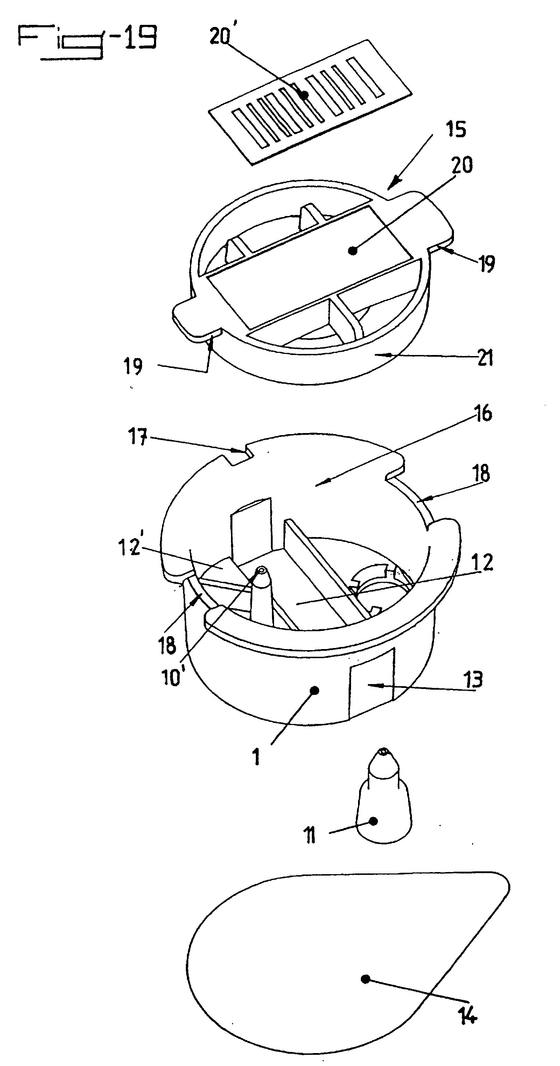

Un appareil pour la mise en oeuvre de la variante de réalisation du procédé est représenté plus particulièrement sur les figures 16 à 18 des dessins annexés, et est essentiellement constitué par un premier module 2 de réception, de stockage et de préparation de supports rotatifs de mesure 4 et/ou 5 frais et usagés, par un deuxième module 3 d'incubation des supports rotatifs 4 et/ou 5, par un troisième module 6 de mesures néphélémétriques et/ou photométriques, par un dispositif 7, 7' de transfert desservant les modules 2, 3 et 6 et par un moyen informatique 8 de contrôle et de commande des différents étapes de procédé et d'acquisition, d'enregistrement, de traitement, d'évaluation et/ou de restitution de données.

Conformément à l'invention, le premier module 2 de réception, de stockage et de préparation des supports rotatifs de mesure 4 et/ou 5 frais et usagés comporte, en outre, au moins un moyen 59 de réception et de stockage de récipients primaires 1 contenant de l'inoculum non calibré et un dispositif de calibration 60,61 de l'inoculum dans lesdits récipients primaires 1 et de préparation des supports rotatifs 4 et/ou 5.

Selon une caractéristique de l'invention, chaque récipient primaire 1 de réception de l'inoculum se présente avantageusement (figures 19, 20) sous forme d'un récipient cylindrique muni sur son fond d'au moins un tube d'évent 9, d'une conduite d'évacuation de trop-plein 10 éventuellement d'une conduite 10' de mise à un niveau inférieur du volume d'inoculum, d'un moyen de prélèvement d'une quantité prédéterminée d'inoculum, monté de manière séparable, par arrachement ou par emboîtement, dans le fond du récipient 1 et excentré par rapport à l'axe dudit récipient 1, telle qu'une pipette 11, et d'un logement transversal 12 en forme d'auge s'étendant entre les parois du récipient 1 qui est pourvu à chaque extrémité dudit logement 12 d'une fenêtre de mesure 13, ce logement 12 étant muni, en outre, d'une partie surélevée 12' communiquant avec ledit logement 12, l'évent 9, la conduite de trop-plein 10, la conduite 10' de mise à un niveau inférieur et le moyen de prélèvement 11 étant fermés de manière étanche par un film arrachable 14, un couvercle de fermeture 15 coopérant par emboîtement conique étanche avec la partie supérieure conique 16 dudit récipient 1.

Le logement transversal 12, et plus particulièrement la partie surélevée 12' de ce dernier, est destiné à la préparation manuelle de l'inoculum en solution aqueuse à partir d'une solution de culture, ladite solution étant déposée dans le fond du logement 12, sur la partie surélevée 12', au moyen d'un organe de prélèvement servant simultanément à réaliser un premier mélange de la solution de culture avec l'eau rajoutée.

La partie supérieure conique 16 du récipient primaire 1 est pourvue d'au moins une encoche 17 de faible profondeur et de deux encoches 18 de profondeur plus importante et de largeur différente, disposées en alignement entre-elles et à 90° par rapport à l'axe passant par la ou les encoches 17 de faible profondeur, cette ou ces encoches de faible profondeur 17 étant alignées dans l'axe du logement 12 du fond et les encoches 18 de profondeur plus importante coopérant avec des pattes 19 de section correspondante prévues sur le couvercle de fermeture étanche 15 qui est pourvu, d'une part, sur sa face supérieure, d'une surface 20 s'étendant dans l'axe des pattes 19 et, d'autre part, d'une paroi conique 21 de forme correspondante à celle de la partie supérieure conique 16 du récipient 1 et reliant les pattes 19.

La surface 20, légèrement en retrait et qui s'étend entre les pattes 19, et donc entre les encoches 18 lorsque le couvercle de fermeture 15 est monté sur le récipient primaire 1, est destinée à la réception d'un moyen d'identification codé 20', tel qu'une étiquette adhésive à code à barres.

L'encoche 17 de faible profondeur permet de réaliser un positionnement précis du récipient primaire 1 dans le module 2, afin de permettre notamment la lecture du moyen d'identification. Cette encoche 17 est destinée, par ailleurs, à assurer une orientation correcte du récipient primaire 1 lors de la calibration automatique de l'inoculum, ainsi que pour l'inoculation du support rotatif 4 pour la réalisation d'antibiogrammes.

La pipette 11 formant le moyen de prélèvement d'une quantité prédéterminée d'inoculum est montée de manière séparable par arrachement ou par emboîtement dans le fond du récipient 1 retourné de manière à présenter son couvercle 15 tourné vers le bas. Cette pipette 11 est pourvue, près de son extrémité dépassant sous le fond du récipient 1, d'une collerette ou d'un manchon 11', par laquelle elle est reliée avec le fond du récipient 1 au niveau de la traversée de ce fond, par exemple par une liaison étanche fragilisée ou par emboîtement. De manière connue, le fond et les parois latérales, ainsi que le couvercle 15, du récipient primaire 1 sont réalisés par moulage en un matériau synthétique. Ainsi, lors du remplissage du récipient 1 et pendant sa manipulation, l'étanchéité au niveau de la pipette 11 reste assurée, tandis que, après retournement du récipient 1 fermé par le couvercle, lors de la prise de la pipette 11 et par une légère poussée sur celle-ci, elle est désolidarisée du fond du récipient 1 et peut être transférée vers une utilisation après aspiration d'une quantité prédéterminée d'inoculum.

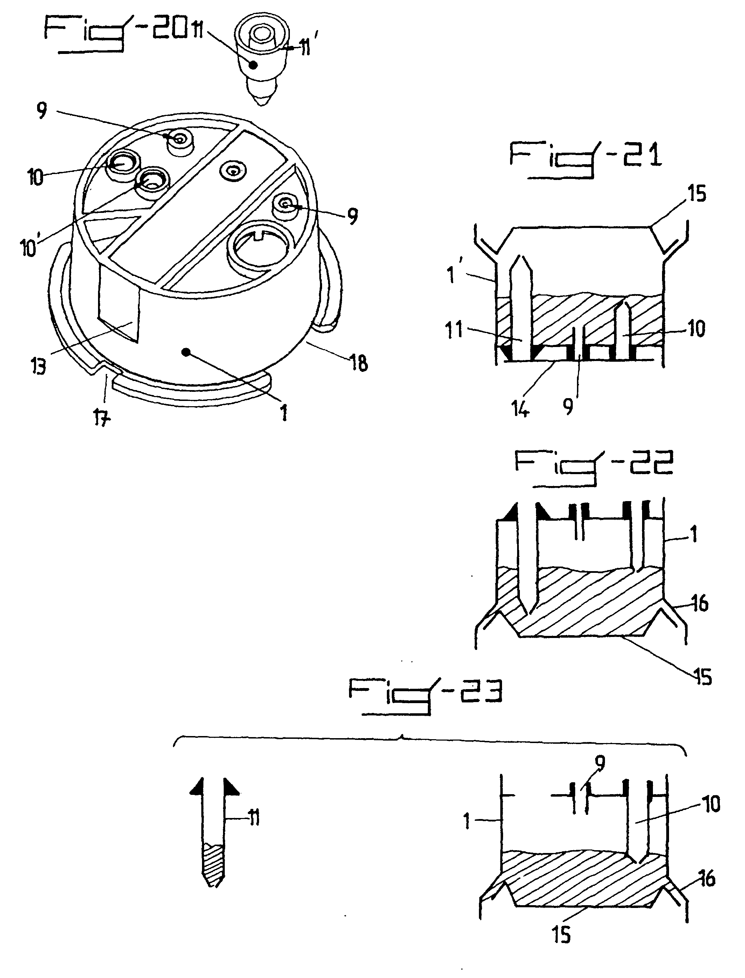

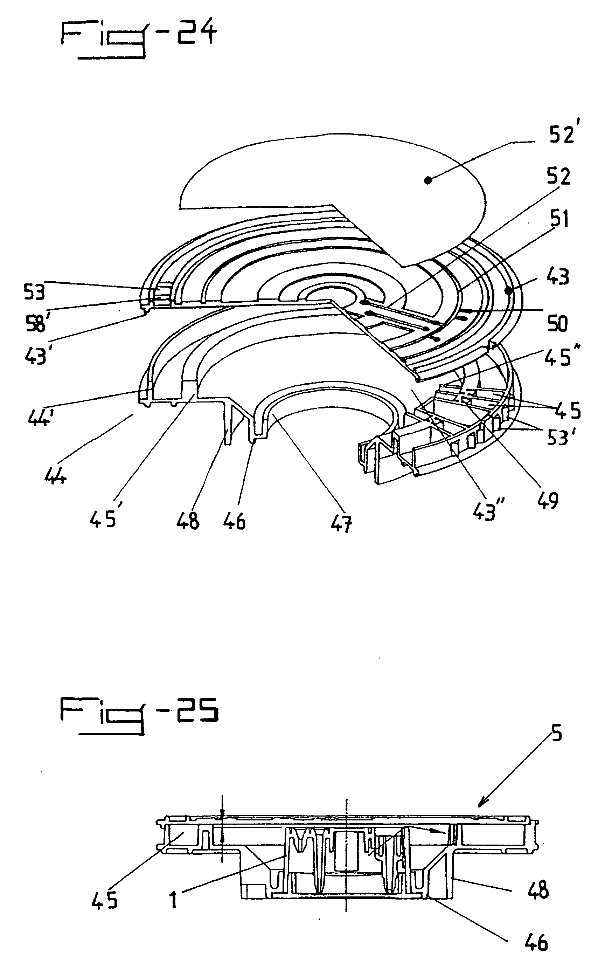

Les figures 21 à 23 représentent schématiquement les étapes successives de préparation de l'inoculum et de vérification de sa densité minimale dans le récipient 1, puis la fermeture étanche de ce dernier par le couvercle 15, le film arrachable 14 fermant hermétiquement les orifices extérieurs du tube d'évent 9, de la conduite d'évacuation 10 et de la pipette 11. Lors de l'introduction du récipient primaire 1 dans le premier module 2 de réception et de stockage ainsi que de calibration de l'inoculum, le récipient 1 est retourné de manière à présenter son fond tourné vers le haut et le film 14 est arraché, afin de permettre la calibration de l'inoculum par ajustement éventuel de son volume et/ou par élimination du trop-plein, au moyen du dispositif 60, 61 de calibration de l'inoculum et de préparation des supports rotatifs 4 et/ou 5.

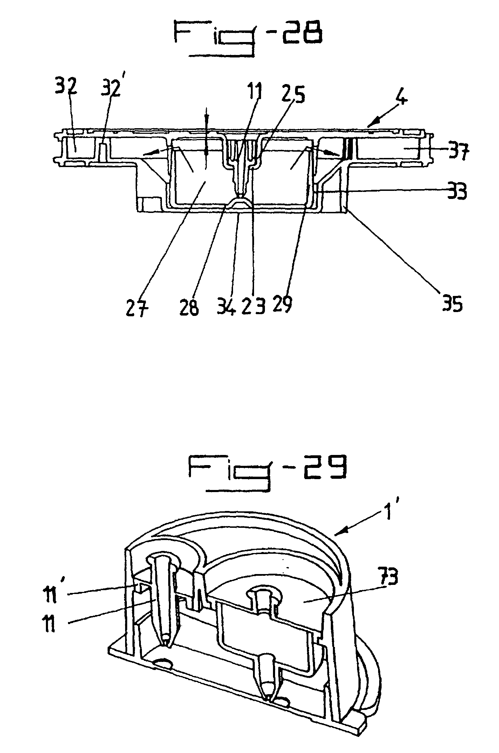

Le support rotatif 4 pour antibiogramme, représenté aux figures 26 à 28 des dessins annexés, se présente avantageusement sous forme d'un disque composite supérieur muni d'alvéoles périphériques 32 de réception de l'inoculum et de préparations antibiotiques et est caractérisé en ce qu'il est muni d'un corps cylindrique concentrique, de plus petit diamètre que le disque et lié audit disque, formant une chambre centrale 33, de réception de l'inoculum pour la distribution par centrifugation, ladite chambre 33 étant alimentée à travers un orifice 23 de la face supérieure du disque composite supérieur et étant entourée par un manchon 35 de préhension et de rigidification de l'ensemble.

Le disque composite supérieur du support rotatif pour antibiogramme 4 est constitué, d'une part, par une partie de couvercle circulaire 22 pourvue en son centre d'un orifice 23 et, d'autre part, par un plateau circulaire 31, dont la face supérieure tournée vers la face inférieure de la partie de couvercle 22 porte, en relief sur sa périphérie, les alvéoles 32 de réception de l'inoculum et de préparations antibiotiques, ce plateau circulaire 31 étant relié à la chambre concentrique 33 fermée par un fond 34, la partie de couvercle 22 étant pourvue d'un rebord 22' s'étendant concentriquement par-dessus une paroi circulaire 31' du plateau circulaire 31, délimitant les alvéoles en relief 32 vers l'extérieur, et fixé à ladite paroi circulaire 31' par collage ou soudage.

Selon une caractéristique de l'invention, le plateau circulaire 31 de la partie inférieure de corps est pourvu, entre les alvéoles en relief 32, d'au moins une alvéole de pré-incubation 37 reliée, par l'intermédiaire d'un capillaire 38, à une chambre concentrique 27 ménagée sous le plateau circulaire 22 de la partie de couvercle, dans la chambre concentrique 33 du plateau circulaire 31 formant la partie inférieure du corps.

L'orifice 23, prévu au centre de la partie de couvercle 22, est disposée dans un puits 25 s'étendant concentriquement à l'intérieur de la chambre concentrique 27 qui présente une coupelle mobile 28 pourvue d'un milieu de culture sec, ladite coupelle mobile 28 présentant une paroi de guidage étanche 29 délimitant avec ladite coupelle mobile 28 une cuvette, le puits 25 étant fermé par un film étanche 26 recouvrant la partie supérieure du plateau circulaire de couvercle et délimitant simultanément le capillaire 38.

Le guidage étanche de la coupelle 28 au moyen de sa paroi 29 peut être obtenu par un montage glissant légèrement serré de ladite coupelle 28 et de sa paroi 29 dans la chambre concentrique 27. La coupelle mobile 28 est destinée à la préparation de la culture disposée sur son fond, cette préparation étant réalisée sous forme de solution aqueuse puis séchée, la paroi 29 assurant le logement de la solution aqueuse jusqu'à dessèchement complet.

Les alvéoles en relief 32 sont délimitées sur leur face tournée vers l'intérieur du support rotatif 4 par une paroi circulaire concentrique 32' et sont pourvues chacune, sur la face de ladite paroi concentrique 32' opposée aux alvéoles 32, de chicanes verticales 32" déterminant des moyens de captage de l'inoculum lors de la centrifugation.

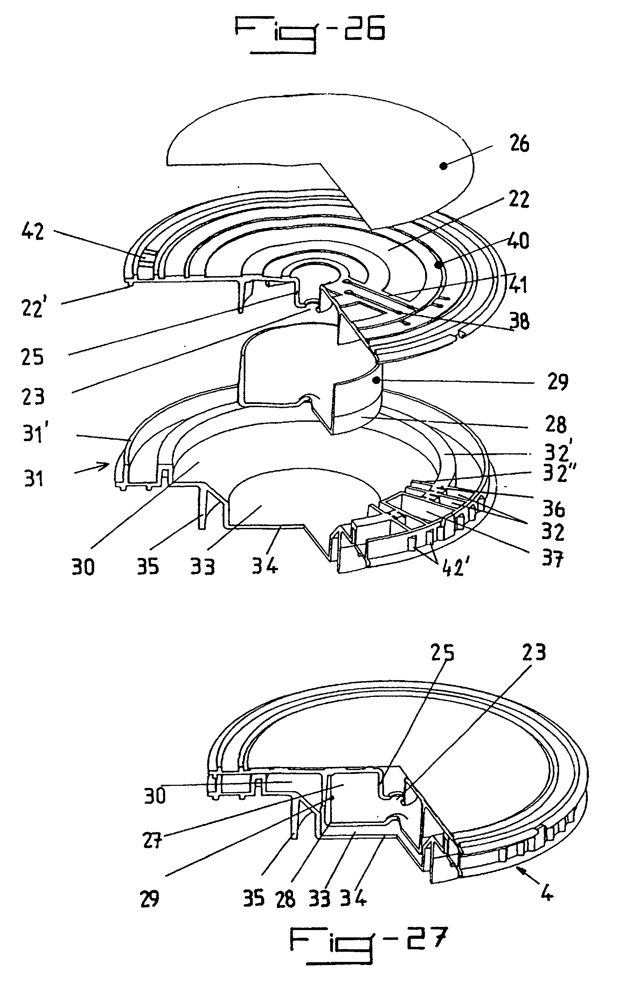

Le support rotatif 5 pour l'identification, représenté aux figures 24 et 25 des dessins annexés, se présente également avantageusement sous forme d'un disque composite supérieur muni d'alvéoles périphériques 45 de réception de l'inoculum et de réactif de caractérisation et est caractérisé en ce qu'il est muni d'un corps cylindrique concentrique, de plus petit diamètre que le disque et lié audit disque, formant un manchon concentrique 46 de réception du récipient primaire 1 en position retournée, pour la distribution par centrifugation de l'inoculum, ledit manchon 46 étant entouré par un manchon concentrique 48 de préhension et de rigidification de l'ensemble.

Le manchon concentrique 46 s'étend, au centre, sous la face inférieure du plateau circulaire 44, est relié à cette dernière et est muni à son extrémité libre d'une partie de paroi conique 47, s'étendant intérieurement audit manchon 46 et destinée à coopérer avec la paroi externe de la partie supérieure conique 16 du récipient primaire 1.

Le disque composite supérieur du support rotatif pour l'identification 5 est constitué, d'une part, par une partie de couvercle circulaire 43 et, d'autre part, par une partie inférieure sous forme d'un plateau circulaire 44 pourvu, sur sa face supérieure tournée vers la face inférieure de la partie de couvercle 43, en relief sur sa périphérie, d'alvéoles 45 de réception de l'inoculum et de réactifs de caractérisation, le couvercle 43 étant pourvu d'un rebord 43' s'étendant concentriquement par-dessus la paroi circulaire 44' du plateau circulaire 44, délimitant les alvéoles en relief 45 vers l'extérieur, et fixé à ladite paroi circulaire 44' par collage ou soudage.

Les alvéoles en relief 45 sont délimitées sur leur face tournée vers l'intérieur du support rotatif 5 par une paroi circulaire concentrique 45' et sont pourvues chacune, sur la face de ladite paroi concentrique 45' opposée aux alvéoles 45, de chicanes verticales 45" déterminant des moyens de captage de l'inoculum lors de la centrifugation.

Les moyens de captage de l'inoculum, lors de la centrifugation, prévus, vus en sens radial, devant chaque alvéole 32, 45, respectivement des supports 4 et 5, sont reliés chacun avec l'intérieur d'une alvéole correspondante 32, 45 par l'intermédiaire d'un capillaire 36, 49 et sont ouverts, par ailleurs, sur un espace intérieur 30, 43", communiquant avec la chambre concentrique 33 du plateau circulaire 31 formant la partie inférieure de corps ou avec l'espace de réception du récipient primaire 1 dans le manchon concentrique 46 s'étendant sous la face inférieure du plateau circulaire 44 formant la partie inférieure du disque composite, les alvéoles en relief 32, 45 étant reliées, par ailleurs, chacune par l'intermédiaire d'un capillaire radial 39, 50 à un capillaire circulaire 40, 51 débouchant, par l'intermédiaire d'un autre capillaire 41, 52, à l'extérieur du plateau circulaire 22 de couvercle ou de la partie de couvercle circulaire 43, l'alvéole de pré-incubation 37 prévue sur le support de mesure 4 pour antibiogramme étant reliée directement, par un trou de passage correspondant, au capillaire circulaire 40. Ces différents capillaires sont délimités dans la partie supérieure du plateau circulaire 22 et du couvercle 43 par le film étanche 26 ou 52', les capillaires 41 et 52 communiquant en leur extrémité la plus proche de l'axe central des disques 31 et 44 avec les chambres intérieures 27 et 43" par des trous percés dans les plateaux circulaires 22 et 43.

Un film étanche de scellement 26, 52 peut se présenter notamment, de manière connue, sous forme d'un film autocollant en deux matériaux, à savoir, par exemple, en aluminium et en polyéthylène. Ainsi, lors des remplissages successifs, par centrifugation, de l'alvéole de pré-incubation 37 et des alvéoles en relief 32, 45 au moyen des capillaires respectifs 38, 36 et 49, l'air contenu dans lesdites alvéoles peut s'échapper, en compensation du remplissage, à travers les capillaires 39, 50, 40, 51 et 52 vers les chambres 27 et 43".

Le plateau circulaire de couvercle 22 et le couvercle circulaire 43 sont munis, en outre, au-dessus de chaque alvéole 32, 45 et près de son bord, d'une fenêtre de mesure 42, 53, une fenêtre analogue 43, 54 étant prévue sous la face inférieure du plateau circulaire 31, 44 formant la partie inférieure du corps ou du disque composite, en regard de chaque fenêtre de mesure 42, 53 et au moins une zone réfléchissante d'indexation 42', 53', formée sur la face externe de la paroi périphérique externe des supports 4, 5, étant associée à chaque alvéole 32, 45 avec un décalage angulaire fixe par rapport à cette dernière.

Ainsi, lors des mesures, le passage de chaque alvéole devant le dispositif de mesure est parfaitement repéré au cours de la rotation du support rotatif 4 ou 5 grâce aux zones réfléchissantes ou miroirs d'indexation et la mesure correspondante peut être effectuée de manière parfaitement synchronisée.

Le récipient primaire 1' utilisé pour l'introduction automatique d'un inoculum bactérien initial dans un support de mesure pour antibiogramme ou analyse combinée simultanée se présente avantageusement, comme le montre la figure 29 des dessins annexés, sous forme d'un réceptacle muni, d'une part, d'une pipette 11 identique à celle associée au récipient primaire 1 et, d'autre part, d'une macropipette 73 de transfert de la totalité du contenu du récipient 1', cette macropipette pouvant être détachée du récipient primaire 1', utilisée pour vider son contenu dans le support 4, puis replacée dans le récipient primaire 1', la pipette 11 étant ensuite détachée et utilisée pour obturer le support 4 en étant déposée dans le puits 25. Dans un tel cas, la solution est directement disposée dans la macropipette 73 et non dans le récipient 1', la pipette 11 servant uniquement de bouchon d'obturation pour un support 4 pour antibiogramme ou éventuellement pour un rajout d'eau.

Comme il ressort des figures schématiques 21 à 23 des dessins annexés, l'inoculation d'un support rotatif 4 pour antibiogramme s'effectue par saisie, au moyen d'un organe préhenseur d'un dispositif 64 de pipetage et d'injection d'eau et de gaz de l'extrémité de la pipette 11 dépassant hors du fond du récipient 1, puis par introduction de ladite pipette 11, après perforation du film étanche 26, dans le puits 25 s'étendant concentriquement à l'intérieur de la chambre concentrique 27, de manière à dégager l'accès à cette dernière. L'inoculum peut alors être déversé dans la chambre concentrique 27 et un apport d'eau complémentaire peut être effectué. Dans la chambre 27, l'inoculum se mélange au milieu de culture sec destiné à alimenter sa croissance.

La pipette 11 est alors enfoncée à travers l'orifice 23 et le puits 25 et guidée dans ces derniers au moyen de son manchon ou collerette 11' jusqu'à arriver en butée de la coupelle mobile 28 par son extrémité libre, de manière à réaliser une étanchéité par rapport à l'extérieur. Après une agitation favorisant l'homogénéisation du contenu de la coupelle 28, une première centrifugation est alors effectuée de manière à amener une partie dudit contenu dans l'alvéole de pré-incubation 37, à travers le capillaire 38.

Dès que le début de croissance aura pu être observé par mesure néphélémétrique, le support rotatif pour antibiogramme 4 sera soumis à une nouvelle centrifugation, le fond mobile 28 de la chambre concentrique 27 étant préalablement poussé au moyen de la pipette 11 de manière à libérer entre sa paroi 29 et celle de ladite chambre 27 des passages permettant le déversement de l'inoculum dans la chambre concentrique 33. Le support rotatif 4 sera alors déplacé successivement dans le module de mesure 6 et dans le deuxième module 3 d'incubation en vue de la réalisation des mesures néphélémétriques et de l'incubation.

L'inoculation du support rotatif 5 pour l'identification peut être effectuée immédiatement après l'inoculation du support 4, par transfert du récipient primaire 1 sous un support 5 et emboîtement étanche dans ledit support 1. A cet effet, la partie de paroi conique interne 47 du manchon concentrique 46 enserre la paroi extérieure de la partie supérieure conique 16 du récipient 1 et réalise l'étanchéité du montage.

Une centrifugation du support rotatif 5 a alors pour effet d'amener l'inoculum contenu dans le récipient 1 vers l'espace délimité au-dessus de ce récipient 1 sous la partie de couvercle 43, à travers l'orifice réalisé par l'arrachage ou le retrait de la pipette 11 et de son manchon ou collerette 11' et, de là, vers les alvéoles 45, à travers les capillaires 49, l'air en excès étant évacué à travers les capillaires 50 à 52. Après incubation avec les réactifs de caractérisation, les mesures photométriques sont alors effectuées.

Comme le montrent plus particulièrement les figures 16 à 18 des dessins annexés, le dispositif de calibration automatique est constitué, d'une part, par une unité inférieure 60 comportant notamment un moyen de lecture du moyen d'identification codé rapporté sur la rainure transversale 20 du couvercle 15 des récipients primaires 1 et un moyen de mesure de densité optique de l'inoculum présent dans lesdits récipients 1 et, d'autre part, par une unité supérieure 61 comportant essentiellement un dispositif de pipetage et d'injection d'eau et d'air.

Ce dernier dispositif peut être, d'une part, muni d'un organe préhenseur mobile au moins dans une direction verticale et d'un dispositif d'évacuation du trop-plein et, d'autre part, relié à un moyen de pompage ou d'aspiration 62, à un ensemble 63 de vannes de commande des flux de liquides et d'air et à des récipients de liquides 64 et d'air.

L'organe de préhension du dispositif de pipetage pourra venir en prise étanche, avec conjugaison de forme, sur l'extrémité extérieure libre de la pipette 11 d'un récipient 1, lorsque ce dernier est disposé à un emplacement adéquat, appelé emplacement de travail.

Cet organe de préhension réalisera aussi l'injection d'eau lors de la phase de calibration automatique de même que le détachement de la pipette 11 par arrachage ou déboîtement du fond du récipient primaire 1, le maintien de la pipette 11 dans une position escamotée avec rétention de l'inoculum et l'introduction de ladite pipette dans le support rotatif 4 pour antibiogramme avec injection de l'inoculum.

Ledit dispositif de pipetage pourra être relié alternativement à un moyen d'aspiration et à un moyen d'injection contrôlée d'une quantité de fluide, notamment d'eau ou d'air, extraite de réservoirs correspondants.

L'unité supérieure 61 pourra également comporter un embout mobile pour le branchement étanche d'un tuyau d'évacuation de trop-plein ou d'une pipette relié à un moyen d'aspiration continue et à un réservoir de récupération, ledit embout mobile ou ladite pipette pouvant venir en prise sur l'extrémité libre extérieure de la conduite d'évacuation 10 du trop-plein et/ou de la conduite 10' de mise à niveau intégrée dans le fond du récipient primaire 1, lorsque ce dernier est positionné au niveau de l'emplacement de travail.

Comme le montrent également les figures 16 à 18 des dessins annexés, le moyen de réception et de stockage 59 consiste en au moins un disque 65 pouvant être actionné en rotation de manière contrôlée et pourvu d'une pluralité de rainures de stockage 66 disposées suivants des rayons dudit disque 65 et pouvant contenir chacune, soit directement, soit au moyen d'un portoir intermédiaire, une rangée alignée de récipients primaires 1 en position inversée et indexés en position angulaire autour de leur axe de symétrie central, chacune desdites rainures 66 pouvant être amenée individuellement en regard de l'ouverture du dispositif de calibration automatique 60, 61, par simple rotation dudit disque 65 en vue de l'extraction de la rangée de récipients primaires 1 concernée.

Les portoirs intermédiaires pourront avantageusement consister chacun en un réceptacle allongé profilé 60' pouvant contenir chacun simultanément une pluralité de récipients primaires 1, sous forme d'un alignement de récipients primaires 1 en positions inversées, dont chacun est indexé en position angulaire autour de son axe de symétrie central et peut être positionné individuellement à un emplacement de travail défini au niveau des unités supérieure 61 et inférieure 60 constituant le dispositif de calibration et de préparation automatique.

Chaque récipient primaire 1 pourra ainsi être amené individuellement et positionné verticalement au niveau de l'emplacement de travail, par simple coulissement du réceptacle allongé profilé 60'.

En outre, le guidage radial des réceptacles allongés profilés 60', formant portoirs pour des alignements de récipients primaires 1, dans les rainures de stockage 66 du moyen 59, permettra de réaliser une agitation des contenus de l'ensemble desdits récipients primaires 1, en soumettant successivement chacun desdits alignements, disposés chacun dans un desdits portoirs 60', à un mouvement alternatif en va-et-vient.

Conformément à une autre caractéristique de l'invention, les dispositifs de transfert desservant les différents modules 2, 3 et 6 comprennent notamment, d'une part, un premier organe transporteur 7 assurant notamment le transfert des supports rotatifs 4 et 5 entre les modules d'incubation 3 et de mesure 6 et leur prise en charge en provenance du dispositif de calibration automatique 60, 61, d'autre part, un second organe transporteur 7' assurant la prise en charge et le dépôt d'un support rotatif 4 ou 5 au niveau d'un magasin de stockage 67 correspondant, son transfert et son maintien dans le dispositif de calibration automatique 60,61 et son acheminement à un emplacement déterminé permettant sa prise en charge par le premier organe transporteur 7.

Les mouvements de transfert de supports rotatifs 4, 5 pouvant, bien évidemment, également être réalisés en sens inverse, en vue du rangement des supports 4, 5 usagés dont l'exploitation est achevée. Dans ce cas, chaque support 4, 5 usagé sera transmis par le premier organe transporteur 7 au second organe transporteur 7' qui l'acheminera vers le magasin de stockage 67 du premier module 2 où il sera déchargé et rangé à un ou plusieurs emplacements prévus à cet effet.

Comme le montrent également les figures 16 à 18 des dessins annexés, le magasin de stockage 67 est situé dans la partie supérieure du premier module 2 et consiste en un magasin circulaire pouvant être animé d'un mouvement de rotation et comportant une pluralité de piles de supports de mesures 4, 5 frais et usagés au niveau de sa périphérie.

Chaque pile peut être amenée individuellement dans la zone de préhension de l'organe transporteur 7', permettant à ce dernier de soulever le support de mesure 4, 5 supérieur d'une pile positionnée de manière adéquate, et de le transférer vers le dispositif de calibration automatique 60, 61.

Bien entendu, ledit organe transporteur 7' pourra également recevoir un support de mesure 4, 5 usagé, acheminé par le premier organe transporteur 7 à l'endroit d'échange, et le déposer dans une pile correspondante, préalablement positionnée à l'endroit adéquat desservi par ledit second organe transporteur 7'.

Selon un mode de réalisation préférentiel de l'invention, l'organe de manipulation du second organe transporteur 7' peut se présenter sous la forme d'une pince 68, ladite pince 68 pouvant notamment se positionner entre les unités inférieures 60 et supérieures 61 du dispositif de calibration automatique de telle manière que le support de mesure 4 ou 5 porté par cette dernière soit disposé au niveau de l'emplacement de travail dudit dispositif de calibration automatique.

Le maintien des supports de mesure 4 et 5 par ladite pince 68 est réalisé par pincement au niveau de leur partie supérieure.

Le positionnement des supports 4, 5 au niveau de l'emplacement de travail du dispositif de calibration automatique 60, 61 permettra soit l'introduction de la pipette 11 dans un support de mesure 4 pour antibiogramme, soit l'emboîtement d'un récipient primaire 1 dans un support de mesure 5 pour l'identification.

Les premier et second organes transporteurs 7 et 7' peuvent consister avantageusement chacun en un bras support 70, respectivement 70', monté avec faculté de rotation et de translation verticale sur un axe 71, respectivement 71', solidaire du châssis de l'appareil.

Les mouvements du premier organe transporteur 7, dont l'axe peut être fixé sur le module de mesure 6 et dont l'organe de manipulation peut consister en un plan support, permettent un chargement ou un déchargement d'un support de mesure 4 ou 5 à partir du ou vers le second organe transporteur 7', le module 3 d'incubation et le module 6 de mesure.

Ce dernier peut comporter, au niveau de son ouverture d'accès, un mécanisme échangeur transférant les supports de mesure 4,5 entre un emplacement de chargement/déchargement accessible par l'organe transporteur 7 et un emplacement de mesure, en interchangeant deux supports de mesure 4, 5 entre ces deux emplacements.

De manière préférentielle, et en vue d'augmenter l'efficacité de l'appareil, la durée nécessaire pour le replacement d'un support 4 ou 5 après la phase de mesure et l'amenée d'un nouveau support 4 ou 5 au niveau du module 6 de mesure est environ identique à la durée de la phase de mesure.

En outre, les supports de mesure 4, 5 peuvent être centrés et guidés en rotation au niveau de l'emplacement de mesure du module 6 de mesure, l'entraînement en rotation desdits supports de mesure 4, 5 étant réalisé par l'intermédiaire d'un organe escamotable supérieur.

Afin de pouvoir effectuer une centrifugation à vitesse élevée en vue d'une répartition rapide de l'inoculum dans les alvéoles périphériques des supports de mesure 4, 5, le module 6 de mesure peut comprendre un dispositif 72 de centrifugation supplémentaire distinct pouvant être desservi par le premier organe transporteur 7.

De manière avantageuse, les modules 3 et 6 sont situés dans une enceinte confinée et thermorégulée à une température favorable à l'incubation, alors que le magasin de stockage 67 des supports 4, 5 est à température ambiante ou situé dans un espace éventuellement refroidi.

L'enceinte confinée et thermorégulée pourra comporter au niveau du lien d'échange entre le premier 7 et le second 7' organes transporteurs, une ouverture obturable, éventuellement de manière étanche, commandée par le moyen informatique 8 de commande et de contrôle.

L'appareil pour la mise en oeuvre du procédé conforme à l'invention est par conséquent composé par un nombre limité de parties constitutives, dont les mouvements, notamment au niveau des moyens de transfert et du dispositif de calibration automatique, sont extrêmement simplifiés. En outre, la structure et l'agencement interne spécifique dudit appareil permet d'aboutir à un encombrement minimum pour une capacité de traitement donnée.

Bien entendu, l'invention n'est pas limitée aux modes de réalisation décrits et représentés aux dessins annexés. Des modifications restent possibles, notamment du point de vue de la constitution des divers éléments ou par substitution d'équivalents techniques, sans sortir pour autant du domaine de protection de l'invention.

## Revendications

1. Procédé d'identification bactérienne et de détermination de la sensibilité de bactéries à des antibiotiques, sous forme d'antibiogramme, **caractérisé en ce qu'**il consiste à introduire manuellement, au moyen d'un outil de prélèvement, un volume donné de colonie bactérienne dans un récipient primaire (1 ; 101), à réaliser automatiquement la dispersion de cette colonie bactérienne au sein d'un liquide pour constituer un inoculum au moins précalibré dans ledit récipient primaire (1 ; 101), à effectuer également automatiquement des transferts totaux ou partiels de cet inoculum précalibré entre ledit récipient primaire (1 ; 101) et un ou plusieurs supports de mesure (4, 5 ; 104, 105) destinés à réaliser, respectivement, soit l'identification, soit l'antibiogramme ou encore ces deux déterminations simultanément en une seule opération combinée, lesdits transferts étant réalisés sans que l'inoculum précalibré soit mis en contact avec un élément autre que, d'une part, un outil de prélèvement et de transfert (101'; 1, 101) et/ou le récipient primaire (1 ; 101) et, d'autre part, son ou ses supports de mesure finaux (4,5; 104, 105) et de telle manière que les quantités de bactéries transférées correspondent aux quantités requises pour les analyses à réaliser, à effectuer automatiquement la répartition de l'inoculum précalibré, éventuellement après l'avoir convenablement dilué pour aboutir à une calibration définitive, dans une ou plusieurs alvéoles (32, 37, 45 ; 132, 137, 145) desdits supports de mesure (4, 5 ; 104, 105) contenant pour la plupart des réactifs appropriés, à réaliser, dans certains cas, notamment pour l'antibiogramme, à l'intérieur d'un support de mesure (4 ; 104), une opération dite de pré-croissance destinée à mettre les bactéries en état de se multiplier rapidement avant de les soumettre à analyse et à faire des mesures sur le contenu desdites alvéoles (32, 37, 45; 132, 137, 145) pendant ou à la fin d'une ou plusieurs incubations subies par l'inoculum dans lesdits supports de mesure (4, 5 ; 104, 105), les mesures relevées étant enregistrées par un moyen informatique (8 ; 108) et traitées en vue de la caractérisation de la croissance des bactéries présentes dans l'inoculum, de leur identification et/ou de la détermination de leur sensibilité à différents antibiotiques.

2. Procédé selon la revendication 1, **caractérisé en ce que** le prélèvement d'un volume donné de colonie bactérienne et les transferts des volumes d'inoculum précalibré dans les supports de mesure (104, 105) sont effectués au moyen d'un outil unique de prélèvement et de transfert (101') coopérant avec le récipient primaire (101), les volumes d'inoculum précalibré à transférer étant déterminés à partir de mesures optiques de la concentration bactérienne dudit inoculum, effectuées dans le récipient primaire (101), et calculés en fonction des quantités de bactéries nécessaires.

3. Procédé selon l'une quelconque des revendications 1 et 2, **caractérisé en ce que** le prélèvement et l'introduction dans le récipient primaire (101) d'un volume donné de colonie bactérienne sont effectués au moyen de l'outil de prélèvement et de transfert (101') après sa mise en conformation en vue de prélever le volume souhaité et les transferts d'inoculum précalibré du récipient primaire (101) vers les supports de mesure (104, 105) sont réalisés par pipetage de volumes d'inoculum prédéterminés.

4. Procédé selon l'une quelconque des revendications 1 à 3, **caractérisé en ce que** le transfert de l'inoculum à l'intérieur des supports de mesure (104, 105), notamment vers les alvéoles (132, 145), est contrôlé dynamiquement par commande de l'ouverture ou du blocage étanche de passages successifs conduisant progressivement vers lesdites alvéoles (132, 145).

5. Procédé selon la revendication 1, **caractérisé en ce qu'**il consiste à introduire manuellement au moyen d'un outil de prélèvement et de transfert un volume donné d'inoculum bactérien initial dans un récipient primaire (1), à effectuer automatiquement une calibration définitive dudit inoculum dans ledit récipient primaire (1) en amenant sa concentration bactérienne et son volume à des valeurs imposées, à effectuer également automatiquement des transferts totaux ou partiels de cet inoculum calibré entre ledit récipient primaire (1) et un ou plusieurs supports de mesure (4, 5) destinés à réaliser, respectivement, soit l'identification, soit l'antibiogramme ou encore ces deux déterminations simultanément en une seule opération combinée, lesdits transferts étant réalisés sans que l'inoculum initial soit mis en contact avec un élément autre que son récipient initial et son ou ses récipients finaux et de manière à ce qu'à leur issue l'inoculum calibré reste confiné à l'intérieur desdits supports de mesure (4, 5) qui sont alors clos, à effectuer automatiquement la répartition de l'inoculum dans une ou plusieurs alvéoles (32, 45) desdits supports de mesure (4, 5) contenant pour la plupart des réactifs appropriés, à réaliser, dans certains cas, notamment pour l'antibiogramme, à l'intérieur d'un support de mesure (4), une opération dite de pré-croissance destinée à mettre les bactéries en état de se multiplier rapidement avant de les soumettre à analyse et à faire des mesures photométriques sur le contenu desdites alvéoles (32, 45) pendant ou à la fin d'une ou plusieurs incubations subies par l'inoculum dans lesdits supports de mesure (4, 5), les mesures relevées étant enregistrées par un moyen informatique (8) et traitées en vue de la caractérisation de la croissance des bactéries présentes dans l'inoculum, de leur identification et/ou de la détermination de leur sensibilité à différents antibiotiques.

6. Procédé, selon la revendication 5, **caractérisé en ce que** la calibration automatique de l'inoculum dans le récipient primaire (1) consiste à réaliser, à partir d'un inoculum initial contenant une quantité de bactéries en excès par rapport aux besoins réactionnels, excès vérifié par observation visuelle de la turbidité de l'inoculum initial, une injection d'eau de dilution dans ledit récipient primaire (1), de manière à créer un volume augmenté d'inoculum plus dilué, puis à éliminer une partie de l'inoculum ainsi dilué par aspiration jusqu'à obtention du volume final d'inoculum calibré requis, ces opérations étant conduites et éventuellement répétées sur la base de mesures optiques faites au travers du récipient primaire (1) sur l'inoculum pour en déterminer la concentration, jusqu'à obtenir à la fois la concentration et le volume recherchés pour l'inoculum calibré.

7. Procédé, selon l'une quelconque des revendications 5 et 6, **caractérisé en ce que** le transfert de l'inoculum calibré entre le récipient primaire (1) et le ou les supports de mesure (4, 5) est effectué par emboîtement de tout ou partie (11) du récipient primaire (1) contenant l'inoculum dans une cavité prévue à cet effet dans chaque support (4, 5), de manière à y transférer la quantité d'inoculum calibré voulue, contenue dans la partie emboîtée dudit récipient primaire, cet emboîtement assurant l'isolement nécessaire au confinement de l'inoculum dans l'ensemble formé par le support de mesure (4, 5) et le récipient primaire (1) ou la partie (11) du récipient primaire (1) emboîté dans ledit support (4, 5), ainsi que la fermeture de cet ensemble.

8. Procédé, selon l'une quelconque des revendications 1 et 5, **caractérisé en ce que** la pré-croissance de l'inoculum calibré est réalisée, dans les supports de mesure servant à la détermination des antibiogrammes ou dans certains supports de mesure servant à réaliser des déterminations combinées d'antibiogramme et d'identification, par une première incubation, dite pré-incubation, pendant laquelle les bactéries présentes dans l'inoculum sont incubées sans être mises en présence de réactifs et ce jusqu'à ce qu'un début de croissance bactérienne soit mesurable ou jusqu'à ce que la concentration bactérienne atteigne un niveau prédéfini, cette pré-croissance étant suivie de la mise en contact de l'inoculum avec les réactifs et d'une incubation finale pendant laquelle leurs interactions sont observées.

9. Procédé, selon l'une quelconque des revendications 1 et 5, **caractérisé en ce que** la répartition de l'inoculum dans les alvéoles (32, 37, 45 ; 132, 137, 145) situées en périphérie des supports de mesure (4, 5 ; 104, 105) est réalisée par centrifugation, les mesures et les observations effectuées au niveau desdites alvéoles (32, 37, 45 ; 132, 137, 145) étant effectuées par mise en rotation des supports de mesure (4, 5 ; 104, 105) autour de l'axe central de centrifugation desdits supports.

10. Procédé, selon l'une quelconque des revendications 1, 5, 8 et 9, **caractérisé en ce que**, pendant la pré-croissance dans un support de mesure (4 ; 104) d'antibiogramme, la mesure de la concentration de l'inoculum bactérien et la détermination du début de croissance sont réalisées par des mesures photométriques effectuées sur une partie aliquote de l'inoculum transférée à cet effet, par une première centrifugation, dans une alvéole périphérique spécifique (37 ; 137), dédiée à la caractérisation de la croissance pendant la pré-incubation et ne contenant pas de réactifs, le reste d'inoculum restant bloqué pendant la pré-incubation dans une chambre concentrique centrale (27 ; 127) dudit support (4 ; 104), avant d'être transféré, après achèvement de la pré-croissance, dans les autres alvéoles périphériques (32 ; 132) par une deuxième centrifugation, précédée d'une action d'autorisation ou de déblocage appropriée ouvrant le passage pour le transfert du reste de l'inoculum vers lesdites autres alvéoles (32 ; 132) contenant des réactifs.

11. Procédé, selon l'une quelconque des revendications 1, 5, 9 et 10, **caractérisé en ce que** le passage de l'étape de pré-incubation à l'étape d'incubation est effectué sans rupture du confinement de l'intérieur du support rotatif de mesure concerné (4 ; 104) par rapport à l'extérieur, ledit support restant clos et ledit passage étant réalisé par une action, pour le support (4), ou une interruption d'action, pour le support (104), sur un élément obturateur participant à la réalisation de ce confinement.

12. Procédé, selon l'une quelconque des revendications 1 à 11, **caractérisé en ce que** les mesures sont réalisées par néphélémétrie pour les supports de mesure (4 ; 104) servant à l'antibiogramme et par photométrie ou détermination d'absorbance ou de densité optique pour les supports de mesure (5 ; 105) servant à l'identification ou aux déterminations combinées identification/antibiogramme, ou encore par fluorimétrie, après addition préalable d'un marqueur fluorogène au réactif.

13. Support de mesure en forme de disque comprenant un corps principal rigide délimitant, sous forme de compartiments non communiquants, une chambre centrale et plusieurs alvéoles périphériques associées, chacune, à une niche de captage, **caractérisé en ce que** ledit corps principal (126) présente une paroi supérieure (128) pourvue de plusieurs ensembles d'orifices de transfert traversants (129, 130, 131, 133) débouchant respectivement dans la chambre centrale cylindrique (127), dans chaque niche de captage (146) affectée à chaque alvéole (132, 145) et dans chacune desdites alvéoles (132, 145) et recouverte, à l'exception d'une portion centrale entourant l'orifice de chargement (134) de ladite chambre centrale (127) et de la zone des fenêtres de mesure des alvéoles (132, 145), par une membrane flottante étanche affleurante (135) fixée localement et hermétiquement notamment à sa périphérie externe et autour de l'orifice de chargement (134) et pouvant être pressée intimement contre la surface supérieure de ladite paroi supérieure (128) pour former des zones de contact temporaires étanches (136, 138, 142) s'étendant entre ou recouvrant certains desdits ensembles d'orifices traversants (129, 130, 131, 133), notamment au cours de la rotation dudit support de mesure.

14. Support de mesure selon la revendication 13, **caractérisé en ce que** la paroi supérieure (128) comporte, au niveau de sa face supérieure, des rainures ou des renfoncements annulaires concentriques (140, 140') dans lesquel(le)s débouchent les orifices traversants (129, 130, 131, 133) disposés selon des cercles concentriques espacés, la rainure intérieure (140) comprenant une nervure (136) formant surface de contact annulaire et séparant les orifices de sortie (129) de la chambre centrale (127) et les orifices d'entrée (130) de la chambre concentrique annulaire (147) comprenant les niches radiales de captage (146), et les orifices de sortie (131) des niches de captage (146) débouchant au niveau de la face supérieure d'une nervure circulaire concentrique ou de structures proéminentes distinctes (138), par exemple sous forme de dômes, disposée(s) dans la rainure extérieure (140'), les extrémités supérieures de la nervure (136) et des structures (138) étant situées dans un plan ou des plans s'étendant légèrement en retrait du plan de collage de la membrane étanche affleurante (135).

15. Support de mesure selon l'une quelconque des revendications 13 et 14, **caractérisé en ce qu'**il comporte une alvéole de pré-incubation (137) identique aux autres alvéoles périphériques (132, 145), alignée circulairement avec ces dernières et communiquant avec une pré-chambre close (143) formée dans la chambre concentrique (147) renfermant les niches de captage et d'équirépartition (146) et alimentée en inoculum à partir d'une niche de captage interne (144) située au niveau d'une portion de la paroi latérale périphérique (139) de la chambre centrale (127), par l'intermédiaire d'un passage non obturable par les zones de contact étanches (136, 138, 142) pouvant être formées entre la membrane affleurante (135) et la paroi supérieure (128).

16. Support de mesure selon la revendication 15, **caractérisé en ce que** le passage d'alimentation de la pré-chambre (143), communiquant librement avec l'alvéole de pré-incubation (137) et formant également trop-plein pour les niches de captage (146), est constitué par un conduit (157), débutant dans la partie supérieure de la portion de paroi latérale périphérique (139) délimitant la niche de captage interne (144), formé par coopération d'une rainure ménagée dans la partie supérieure d'un prolongement saillant radial (158) de la paroi latérale périphérique (139) de la chambre centrale (127) et de la paroi supérieure (128) et débouchant dans ou à l'entrée d'une rainure (148) à extension radiale centripète, creusée dans la face supérieure de la paroi supérieure (128), formant conduit étanche en coopération avec la membrane (135) contrecollée de part et d'autre de ladite rainure (148) et dont l'extrémité distale se termine au niveau d'un orifice traversant (148') débouchant dans la préchambre (143).

17. Support de mesure selon l'une quelconque des revendications 13 à 16, **caractérisé en ce que** l'orifice de chargement (134) de la chambre centrale (127) est situé dans un renfoncement (134') de la paroi supérieure (128) et est obturable au moyen d'un bouchon (150) dont la partie supérieure est sensiblement de niveau avec la face supérieure de la paroi supérieure (128), **en ce que** le fond de la chambre centrale (127) est entouré d'un manchon concentrique (151) de préhension et de rigidification, saillant au niveau de la face inférieure du support (104, 105) et **en ce qu'**une ouverture traversante (152), ayant des dimensions identiques à celles d'une alvéole (132, 145) et située en alignement circulaire avec ces dernières, est ménagée dans le corps principal (126) et dans la paroi supérieure (128), ladite ouverture (152) étant préférentiellement située adjacente à l'alvéole de pré-incubation (137).

18. Support de mesure selon l'une quelconque des revendications 13 à 17, **caractérisé en ce que** la paroi supérieure (128) est munie, au dessus de chaque alvéole (132, 137, 145) et près de son bord extérieur, d'une fenêtre de mesure, une fenêtre analogue étant prévue, dans chacune des portions de paroi du corps principal (126) constituant respectivement les fonds desdites alvéoles, en regard de chaque fenêtre de mesure précitée et au moins une zone réfléchissante d'indexation, formée sur la face externe de la paroi périphérique externe du corps principal (126), étant associée à chaque alvéole (132, 137, 145) avec un décalage angulaire fixe par rapport à cette dernière.

19. Appareil pour la mise en oeuvre du procédé selon l'une quelconque des revendications 1 à 12, essentiellement constitué par un premier module (102) de support et de stockage de supports de mesure (104, 105) frais, selon l'une quelconque des revendications 13 à 18 et de récipients primaires (101) coopérant chacun avec un outil de prélèvement et de transfert (101') correspondant, par un deuxième module (103) d'incubation des supports (104 et 105), après injection de l'inoculum bactérien, par un troisième module (106) de centrifugation et de mesures néphélémétriques et/ou photométriques et/ou fluorimétriques, par un ou plusieurs dispositifs (107, 107') de transfert et de manipulation desservant lesdits modules (102, 103 et 106) et par un moyen informatique (108) de contrôle et de commande des différentes étapes du procédé, et d'acquisition, d'enregistrement, de traitement, d'évaluation et/ou de restitution de données, les deuxième et troisième modules (103) et (106) étant disposés dans une enceinte de confinement, appareil **caractérisé en ce que** les récipients primaires (101) sont portés par un moyen support (109) en forme de couronne, s'étendant concentriquement autour et sous un moyen support (110) en forme de disque portant les supports de mesure (104, 105), les deux moyens supports (109, 110) étant montés avec faculté de rotation autour d'un même axe et **en ce que** chaque récipient primaire (101) peut être amené, par simple rotation dudit moyen support (109) en forme de couronne, au niveau d'un poste (111) de manipulation de l'outil de prélèvement et de transfert (101') associé et de mesure de densité optique du contenu dudit récipient primaire (101).

20. Appareil selon la revendication 19, **caractérisé en ce que** chaque récipient primaire (101) de réception d'un volume donnée de colonie bactérienne et de préparation d'un inoculum au moins précalibré se présente sous la forme d'un récipient cylindrique ouvert au niveau de sa partie supérieure, laquelle est munie d'un rebord radial (112) pour le maintien dudit récipient primaire (101) dans un des logements correspondants (109') ménagé(s) dans le support mobile (109) en forme de couronne et **en ce que** l'outil de prélèvement et de transfert (101') consiste en une seringue dont le corps (113) comporte une partie effilée avant de prélèvement et d'éjection (113') et une partie arrière (113") de stockage et de préhension sensiblement cylindrique, ladite partie arrière (113") présentant au moins un décrochement radial extérieur (114) au niveau de son extrémité contiguë à la paroi avant (113') en vue d'un emboîtement partiel avec blocage dudit corps (113) dans le récipient primaire (101), l'extrémité libre avant de la partie avant (113') étant alors située à faible distance du fond dudit récipient primaire (101).

21. Appareil selon la revendication 20, **caractérisé en ce que** la seringue (101') comporte, au niveau de son extrémité avant, des moyens (118, 118') pour prélever, en volume, une quantité réduite ou une quantité normale de colonie bactérienne, en fonction du positionnement de son piston (115).

22. Appareil selon l'une quelconque des revendications 19 à 21, **caractérisé en ce que** chaque récipient primaire (101) comporte dans sa paroi latérale, à proximité de son fond, deux fenêtres de mesure (119, 119') en regard, adaptées pour la mesure de la densité optique du contenu dudit récipient primaire (101) au niveau du poste (111).

23. Appareil selon l'une quelconque des revendications 20 à 22, **caractérisé en ce que** le moyen de manipulation (121) de la seringue constituant l'outil de prélèvement (101'), au niveau du poste (111), consiste en deux ensembles de mâchoires (121' et 121") comprenant deux mâchoires externes (121') destinées à venir en prise rigide et bloquante avec un rebord radial (128) au niveau de l'ouverture de la partie arrière (113") du corps (113) et deux mâchoires internes (121"), disposées concentriquement entre les deux mâchoires externes (121'), destinées à venir en prise rigide et bloquante avec un renflement (122') de la tige d'actionnement (116), la fermeture des deux ensembles de mâchoires (121', 121") étant réalisée simultanément par coulissement d'un manchon (121"') autour et le long des mâchoires externes (121'), les mâchoires internes (121") pouvant coulisser par rapport aux mâchoires externes (121'), notamment en position de fermeture, et au manchon (121"') et ce dernier pouvant prendre appui sur le support mobile (109) en forme de couronne et sur le bord radial (112) de l'ouverture supérieure du récipient primaire (101) considéré, de manière à bloquer ce dernier en position dans son logement (109') lors du coulissement de l'un quelconque des deux ensembles de mâchoires (121', 121").

24. Appareil selon l'une quelconque des revendications 19 à 23, **caractérisé en ce qu'**il comporte, d'une part, un premier dispositif de manipulation et de transfert (107), desservant les supports (109 et 110) et se présentant sous la forme d'un bras manipulateur monté avec faculté de rotation et de coulissement sur un premier axe vertical (123) et comprenant deux mâchoires de préhension (124, 124') dont la première (124) est munie d'un moyen de prise (125) d'un récipient primaire (101) et dont la seconde (124') présente une forme courbée ou un crochet destiné à venir en prise en deux endroits distincts d'un support de mesure (104, 105), de manière à maintenir ce dernier par soutien inférieur et par pincement en coopération avec la première mâchoire de préhension (124) et, d'autre part, un second dispositif de manipulation et de transfert (107'), desservant le module d'incubation (103) et le module (104) de centrifugation et de mesure, se présentant sous la forme d'un bras support monté avec faculté de rotation et de coulissement sur un second axe vertical (123') et comportant à son extrémité libre un organe de réception (125') coopérant avec conjugaison de forme avec une partie au moins de la face inférieure des supports de mesure (104, 105), les deux dispositifs de manipulation et de transfert (107, 107') présentant une zone de chevauchement de leurs espaces ou zones de manoeuvre respectivement accessibles, au niveau de laquelle peut notamment s'effectuer le transfert des supports de mesure (104, 105) de l'un à l'autre.

25. Appareil selon l'une quelconque des revendications 19 à 24. **caractérisé en ce que** le troisième module (106) comporte plusieurs postes de travail (153), notamment de mesure et de centrifugation, comprenant chacun, en plus des différents moyens de mesure par néphélémétrie, par photométrie et/ou par fluorimétrie, d'une part, une embase support (154) en forme de disque, portée par l'extrémité supérieure d'un arbre vertical (154') entraîné en rotation et pouvant s'engager avec enclenchement ou avec maintien par friction radiale dans le manchon concentrique (151) d'un support de mesure (104, 105) et, d'autre part, un dispositif presseur supérieur (155) de forme sensiblement discoïdale et entraîné en rotation autour de son axe de symétrie par friction sur le support de mesure (104, 105), ledit dispositif presseur (155) comprenant un ou plusieurs organes presseurs (156, 156', 156") de forme annulaire et pouvant être déplacé, de manière réversible, d'une position haute dans laquelle lesdits organes presseurs ne sont pas en contact avec le support de mesure (104, 105) disposé sur l'embase support (154), vers plusieurs positions plus basses dans lesquelles ils sont successivement mis en appui sur la membrane flottante affleurante (135) selon un ordre prédéterminé (156", 156', 156), cette dernière prenant elle-même appui au niveau de zones de contact étanches, circulaires ou annulaires, concentriques, ou disposées selon des configurations circulaires ou annulaires concentriques, avec la paroi supérieure (128) du support de mesure (104, 105) considéré.

26. Appareil pour la mise en oeuvre du procédé, selon l'une quelconque des revendications 5 à 12, essentiellement constitué par un premier module de réception et de stockage de supports de mesure (4 et/ou 5) frais et usagés, par un deuxième module (3) d'incubation des supports (4 et/ou 5), par un troisième module (6) de mesures néphélémétriques et/ou photométriques et/ou fluorimétriques, par un ou plusieurs dispositifs (7, 7') de transfert desservant les modules (2, 3 et 6) et par un moyen informatique (8) de contrôle et de commande des différents étapes de procédé, et d'acquisition, d'enregistrement, de traitement, d'évaluation et/ou de restitution de données, **caractérisé en ce que** le premier module (2) de réception et de stockage des supports de mesure (4 et/ou 5) frais et usagés comporte, en outre, au moins un moyen (59) de réception et de stockage de récipients primaires (1) contenant de l'inoculum bactérien initial et un dispositif de calibration (60, 61) de l'inoculum dans lesdits récipients primaires (1) et de préparation desdits supports de mesure (4 et/ou 5).

27. Appareil, selon la revendication 26, **caractérisé en ce que** le récipient primaire (1) de réception de l'inoculum se présente sous forme d'un récipient cylindrique muni sur son fond d'au moins un tube d'évent (9), d'une conduite d'évacuation de trop-plein (10) éventuellement d'une conduite (10') de mise à un niveau inférieur du volume d'inoculum, d'un moyen de prélèvement d'une quantité prédéterminée d'inoculum, monté de manière séparable, par arrachement ou par emboîtement, dans le fond du récipient (1) et excentré par rapport à l'axe dudit récipient (1), telle qu'une pipette (11), et d'un logement transversal (12) en forme d'auge s'étendant entre les parois du récipient (1), qui est pourvu à chaque extrémité dudit logement (12) d'une fenêtre de mesure (13), ce logement (12) étant muni, en outre, d'une partie surélevée (12') communiquant avec ledit logement (12), l'évent (9), la conduite de trop-plein (10), la conduite (10') de mise à un niveau inférieur et le moyen de prélèvement (11) étant fermés de manière étanche par un film arrachable (14), un couvercle de fermeture (15) coopérant par emboîtement conique étanche avec la partie supérieure conique (16) dudit récipient (1).

28. Appareil, selon la revendication 27, **caractérisé en ce que** la partie supérieure conique (16) du récipient primaire (1) est pourvue d'au moins une encoche (17) de faible profondeur et de deux encoches (18) de profondeur plus importante et de largeur différente, disposées en alignement entre-elles et à 90° par rapport à l'axe passant par la ou les encoches (17) de faible profondeur, cette ou ces encoches de faible profondeur (17) étant alignées dans l'axe du logement (12) du fond et les encoches (18) de profondeur plus importante coopérant avec des pattes (19) de section correspondante prévues sur le couvercle de fermeture étanche (15) qui est pourvu, d'une part, sur sa face supérieure, d'une surface (20) s'étendant dans l'axe des pattes (19) et, d'autre part, d'une paroi conique (21) de forme correspondante à celle de la partie supérieure conique (16) du récipient (1) et reliant les pattes (19).

29. Appareil, selon la revendication 26, mettant en oeuvre un support rotatif (4) pour antibiogramme, qui se présente sous forme d'un disque composite supérieur muni d'alvéoles périphériques (32) de réception de l'inoculum et de préparations antibiotiques, **caractérisé en ce que** ledit support de mesure rotatif (4) est muni d'un corps cylindrique concentrique, de plus petit diamètre que le disque et lié audit disque, formant une chambre centrale (33), de réception de l'inoculum pour la distribution par centrifugation, ladite chambre (33) étant alimentée à travers un orifice (23) de la face supérieure du disque composite supérieur et étant entourée par un manchon (35) de préhension et de rigidification de l'ensemble.

30. Appareil, selon l'une quelconque des revendications 26 et 29 **caractérisé en ce que** le disque composite supérieur du support rotatif pour antibiogramme (4) est constitué, d'une part, par une partie de couvercle circulaire (22) pourvue en son centre d'un orifice (23) et, d'autre part, par un plateau circulaire (31), dont la face supérieure tournée vers la face inférieure de la partie de couvercle (22) porte, en relief sur sa périphérie, les alvéoles (32) de réception de l'inoculum et de préparations antibiotiques, ce plateau circulaire (31) étant relié à la chambre concentrique (33) fermée par un fond (34), la partie de couvercle (22) étant pourvue d'un rebord (22') s'étendant concentriquement par-dessus une paroi circulaire (31') du plateau circulaire (31), délimitant les alvéoles en relief (32) vers l'extérieur, et fixé à ladite paroi circulaire (31') par collage ou soudage.

31. Appareil, selon la revendication 30, **caractérisé en ce que** le plateau circulaire (31) de la partie inférieure de corps est pourvu, entre les alvéoles en relief (32), d'au moins une alvéole de pré-incubation (37) reliée, par l'intermédiaire d'un capillaire (38), à une chambre concentrique (27) ménagée sous le plateau circulaire (22) de la partie de couvercle, dans la chambre concentrique (33) du plateau circulaire (31) formant la partie inférieure du corps

32. Appareil, selon l'une quelconque des revendications 30 et 31 **caractérisé en ce que** l'orifice (23), prévu au centre de la partie de couvercle (22), est disposée dans un puits (25) s'étendant concentriquement à l'intérieur de la chambre concentrique (27) qui présente une coupelle mobile (28) pourvue d'un milieu de culture sec, ladite coupelle mobile (28) présentant une paroi de guidage étanche (29) délimitant avec ladite coupelle mobile (28) une cuvette, le puits (25) étant fermé par un film étanche (26) recouvrant la partie supérieure du plateau circulaire de couvercle et délimitant simultanément le capillaire (38).

33. Appareil, selon la revendication 26, mettant en oeuvre un support rotatif (5) pour l'identification, se présentant sous forme d'un disque composite supérieur muni d'alvéoles périphériques (45) de réception de l'inoculum et de réactif de caractérisation, **caractérisé en ce que** ledit support rotatif (5) pour l'identification est muni d'un corps cylindrique concentrique, de plus petit diamètre que le disque et lié audit disque, formant un manchon concentrique (46) de réception du récipient primaire (1) en position retournée, pour la distribution par centrifugation de l'inoculum, ce manchon concentrique (46) s'étendant, au centre, sous la face inférieure du plateau circulaire (44), étant relié à cette dernière et étant muni à son extrémité libre d'une partie de paroi conique (47), s'étendant intérieurement audit manchon (46) et destinée à coopérer avec la paroi externe de la partie supérieure conique (16) du récipient primaire (1) et étant entouré par un manchon concentrique (48) de préhension et de rigidification de l'ensemble.

34. Appareil, selon la revendication 33, **caractérisé en ce que** le disque composite supérieur du support rotatif pour l'identification (5) est constitué, d'une part, par une partie de couvercle circulaire (43) et, d'autre part, par une partie inférieure sous forme d'un plateau circulaire (44) pourvu, sur sa face supérieure tournée vers la face inférieure de la partie de couvercle (43), en relief sur sa périphérie, d'alvéoles (45) de réception de l'inoculum et de réactifs de caractérisation, le couvercle (43) étant pourvu d'un rebord (43') s'étendant concentriquement par-dessus la paroi circulaire (44') du plateau circulaire (44), délimitant les alvéoles en relief (45) vers l'extérieur, et fixé à ladite paroi circulaire (44') par collage ou soudage.

35. Appareil, selon l'une quelconque des revendications 30 à 34, **caractérisé en ce que** des moyens de captage de l'inoculum (45"), lors de la centrifugation, prévus, vus en sens radial, devant chaque alvéole (32, 45), respectivement des supports (4 et 5), sont reliés chacun avec l'intérieur d'une alvéole correspondante (32, 45) par l'intermédiaire d'un capillaire (36, 49) et sont ouverts, par ailleurs, sur un espace intérieur (30, 43"), communiquant avec la chambre concentrique (33) du plateau circulaire (31) formant la partie inférieure de corps ou avec l'espace de réception du récipient primaire (1) dans le manchon concentrique (46) s'étendant sous la face inférieure du plateau circulaire (44) formant la partie inférieure du disque composite, les alvéoles en relief (32, 45) étant reliées, par ailleurs, chacune par l'intermédiaire d'un capillaire radial (39, 50) à un capillaire circulaire (40, 51) débouchant, par l'intermédiaire d'un autre capillaire (41, 52), à l'extérieur du plateau circulaire (22) de couvercle ou de la partie de couvercle circulaire (43), l'alvéole de pré-incubation (37) prévue sur le support de mesure (4) pour antibiogramme étant reliée directement, par un trou de passage correspondant, au capillaire circulaire (40), ces différents capillaires étant délimités dans la partie supérieure du plateau circulaire (22) et du couvercle (43) par le film étanche (26 ou 52'), les capillaires (41 et 52) communiquant en leur extrémité la plus proche de l'axe central des disques (31 et 44) avec les chambres intérieures (27 et 43") par des trous percés dans les plateaux circulaires (22 et 43).

36. Appareil, selon l'une quelconque des revendications 30 à 32, 34 et 35, **caractérisé en ce que** le plateau circulaire de couvercle (22) et le couvercle circulaire (43) sont munis, en outre, au-dessus de chaque alvéole (32, 45) et près de son bord, d'une fenêtre de mesure (42, 53), une fenêtre analogue (43, 54) étant prévue sous la face inférieure du plateau circulaire (31, 44) formant la partie inférieure du corps ou du disque composite, en regard de chaque fenêtre de mesure (42, 53) et au moins une zone réfléchissante d'indexation (42', 53'), formée sur la face externe de la paroi périphérique externe des supports (4, 5), étant associée à chaque alvéole (32, 45) avec un décalage angulaire fixe par rapport à cette dernière.

37. Appareil, selon la revendication 36, **caractérisé en ce que**, pour l'introduction automatique d'un inoculum bactérien initial dans un support de mesure pour antibiogramme ou analyse combinée simultanée, le récipient primaire (1') se présente sous forme d'un réceptacle muni, d'une part, d'une pipette (11) identique à celle associée au récipient primaire (1) et, d'autre part, d'une macropipette (73) de transfert de la totalité du contenu du récipient (1'), cette macropipette pouvant être détachée du récipient primaire (1'), utilisée pour vider son contenu dans le support (4), puis replacée dans le récipient primaire (1), la pipette (11) étant ensuite détachée et utilisée pour obturer le support (4) en étant déposée dans le puits (25).

## Patentansprüche

1. Verfahren zur Identifizierung von Bakterien und zur Bestimmung von deren Empfindlichkeit gegen Antibiotika in Form eines Antibiogramms, **dadurch gekennzeichnet, dass** es aus manuellem Zuführen eines bestimmten Volumens an Bakterienkolonie mittels eines Entnahmeinstrumentes zu einem Primärbehälter (1; 101) besteht, aus automatischem Vornehmen der Verteilung dieser Bakterienkolonie innerhalb einer Flüssigkeit, um eine mindestens vorkalibrierte Impfkultur in dem Primärbehälter (1; 101) herzustellen, aus ebenfalls automatischem Durchführen der gesamten oder teilweisen Übertragungen dieser vorkalibrierten Impfkultur zwischen dem Primärbehälter (1; 101) und mindestens einer oder mehreren Messvorrichtungen (4, 5; 104, 105), die dazu vorgesehen sind, dass jeweils entweder die Identifizierung oder das Antibiogramm oder sogar noch diese beiden Bestimmungen gleichzeitig in einem einzigen kombinierten Arbeitsgang erbracht werden, wobei die Übertragungen ausgeführt werden, ohne dass das vorkalibrierte Impfmittel mit einem anderen Element, als einerseits einem Entnahmeund Übertragungsinstrument (101'; 1, 101) und/oder dem Primärbehälter (1; 101), und andererseits seiner oder seinen endgültigen Messvorrichtungen (4, 5; 104, 105) in Berührung kommt, und auf solch eine Weise, dass die übertragenen Bakterienmengen den Mengen entsprechen, die zur Durchführung der Analysen erforderlich sind, aus automatischem Durchführen der Aufteilung der vorkalibrierten Impfkultur, möglicherweise nachdem sie zweckmäßigerweise verdünnt wurde, um eine endgültige Kalibrierung zu erhalten, auf ein oder mehrere Zellen (32, 37, 45; 132, 137, 145) der Messvorrichtungen (4, 5; 104, 105), von denen die meisten die geeigneten Reagenzien enthalten, aus Durchführen, in einigen Fällen insbesondere für das Antibiogramm, innerhalb einer Messvorrichtung (4; 104), eines Vorganges, der vorläufiges Wachsen genannt wird, der und dazu vorgesehen ist, die Bakterien in einen Zustand der schnellen Vermehrung zu versetzen, bevor sie analysiert werden und Messungen bezüglich des Inhaltes der Zellen (32, 37, 45; 132, 137, 145) während oder am Ende einer oder mehrerer Inkubationen, die die Impfkultur in den Messvorrichtungen (4, 5; 104, 105) durchlaufen hat, vorgenommen werden, dabei werden die abgelesenen Messungen mittels einer Computervorrichtung (8, 108) aufgezeichnet und hinsichtlich der Charakterisierung des Wachsens der Bakterien, die in der Impfkultur vorhanden sind, ihrer Identifizierung und/oder der Bestimmung ihrer Empfindlichkeit gegen verschiedene Antibiotika, verarbeitet.

2. Verfahren nach Anspruch 1, **dadurch gekennzeichnet, dass** das Entnehmen eines bestimmten Volumens an Bakterienkolonie und das Übertragen der Volumen an vorkalibrierter Impfkultur in die Messvorrichtungen (104, 105) mittels eines besonderen Entnahme- und Übertragungsinstrumentes (101') vorgenommen werden, das mit dem Primärbehälter (101) zusammenarbeitet, wobei die zu übertragenden Volumen an vorkalibrierter Impfkultur anhand optischer Messungen, auf bakterielle Konzentration. der Impfkultur hin bestimmt, die in dem Primärbehälter (101) vorgenommen werden, und in bezug auf die notwendigen Bakterienmengen berechnet werden.

3. Verfahren nach irgendeinem der Ansprüche 1 und 2, **dadurch gekennzeichnet, dass** das Entnehmen und das Zuführen eines bestimmten Volumens an Bakterienkolonie zu dem Primärbehälter (101) mittels des Entnahme- und Übertragungsinstrumentes (101') nach seiner Konfiguration hinsichtlich des Entnehmens des gewünschten Volumens durchgeführt werden, und die Übertragungen der vorkalibrierten Impfkultur aus dem Primärbehälter (101) zu den Messvorrichtungen (104, 105) per Pipettieren von vorbestimmten Impfkulturvolumen durchgeführt werden.

4. Verfahren nach irgendeinem der Ansprüche 1 bis 3, **dadurch gekennzeichnet, dass** das Übertragen der Impfkultur innerhalb der Messvorrichtungen (104, 105), insbesondere zu den Zellen (132, 145), dynamisch über Steuerung des Öffnens oder des dichten Absperrens von aufeinanderfolgenden Verbindungsgängen, die nach und nach zu den Zellen (132, 145) führen, geregelt wird.

5. Verfahren nach Anspruch 1, **dadurch gekennzeichnet, dass** es aus manuellem Zuführen eines bestimmten Volumens an bakterieller Starterimpfkultur mittels eines Entnahme- und Übertragungsinstrumentes zu einem Primärbehälter (1) besteht, aus automatischem Durchführen einer endgültigen Kalibrierung der Impfkultur in dem Primärbehälter (1), indem ihre bakterielle Konzentration und ihr Volumen auf vorgegebene Werte gebracht werden, aus ebenfalls automatischem Durchführen der gesamten oder teilweisen Übertragungen dieser kalibrierten Impfkultur zwischen dem Primärbehälter (1) und einem oder mehreren Messvorrichtungen (4, 5), die dazu vorgesehen sind, dass jeweils entweder die Identifizierung oder das Antibiogramm oder sogar noch diese beiden Bestimmungen gleichzeitig in einem einzigen kombinierten Arbeitsgang erbracht werden, wobei die Übertragungen ausgeführt werden, ohne dass die Starterimpfkultur mit einem anderen Element, als ihrem ursprünglichen Behälter und ihrem oder ihren endgültigen Behältern in Kontakt kommt, und auf eine Weise, dass die kalibrierte Impfkultur nach deren Beendigung innerhalb der Messvorrichtungen (4, 5) eingeschlossen bleibt, die daher verschlossen sind, aus automatischem Aufteilen der Impfkultur auf eine oder mehrere Zellen (32, 45) der Messvorrichtungen (4, 5), von denen die meisten geeignete Reagenzien enthalten, aus dem Durchführen, in einigen Fällen insbesondere für das Antibiogramm, innerhalb einer Messvorrichtung (4), eines Vorganges, der vorläufiges Wachsen genannt wird, das dazu vorgesehen ist, die Bakterien in einen Zustand der schnellen Vermehrung zu versetzen, bevor sie analysiert werden und photometrische Messungen bezüglich des Inhaltes der Zellen (32, 45) während oder am Ende einer oder mehrerer Inkubationen, die die Impfkultur in den Messvorrichtungen (4, 5) durchlaufen hat, vorgenommen werden, dabei werden die abgelesenen Messungen mittels einer Computervorrichtung (8) aufgezeichnet und hinsichtlich der Charakterisierung des Wachsens der Bakterien, die in der Impfkultur vorhanden sind, ihrer Identifizierung und/oder der Bestimmung ihrer Empfindlichkeit gegen verschiedene Antibiotika, verarbeitet.

6. Verfahren nach Anspruch 5, **dadurch gekennzeichnet, dass** das automatische Kalibrieren der Impfkultur in dem Primärbehälter (1) darin besteht, einer Starterimpfkultur, die im Verhältnis zu den Reaktionsbedürfnissen eine überschüssige Bakterienmenge enthält, dabei wird der Überschuss durch visuelle Beobachtung der Trübheit der Starterimpfkultur überprüft, eine Injektion Verdünnungswasser zu dem Primärbehälter (1) zuzuführen, so dass ein erhöhtes Volumen an verdünnter Impfkultur erzeugt wird, dann einen Anteil der so verdünnten Impfkultur durch Ansaugung zu eliminieren, bis das endgültige Volumen der kalibrierten, erforderlichen Impfkultur erreicht wird, wobei diese Arbeitsgänge, auf der Basis optischer Messungen, die durch den Primärbehälter (1) an der Impfkultur vorgenommen werden, um ihre Konzentration zu bestimmen, durchgeführt und eventuell wiederholt werden, bis gleichzeitig die gesuchte Konzentration und das angestrebte Volumen für die kalibrierte Impfkultur erreicht werden.

7. Verfahren nach irgendeinem der Ansprüche 5 und 6, **dadurch gekennzeichnet, dass** das Übertragen der kalibrierten Impfkultur zwischen dem Primärbehälter (1) und der oder den Messvorrichtungen (4, 5) durch Einpassen des gesamten oder eines Bereiches (11) des Primärbehälters (1), der die Impfkultur enthält, in eine Ausnehmung, die zu diesem Zweck in jeder Vorrichtung (4, 5) vorgesehen ist, so durchgeführt wird, dass die gewünschte Menge kalibrierter Impfkultur, die in dem eingepassten Bereich des Primärbehälters enthalten ist, dorthin übertragen wird, dabei garantiert dieses Einpassen die erforderliche Isolierung für die Einschließung der Impfkultur in der Einheit, die durch die Messvorrichtung (4, 5) und den Primärbehälter (1) oder des Bereiches (11) des Primärbehälters (1), der in die Vorrichtung (4, 5) eingepasst ist, gebildet wird, als auch das Verschließen dieser Einheit.

8. Verfahren nach irgendeinem der Ansprüche 1 und 5, **dadurch gekennzeichnet, dass** das vorläufige Wachsen der kalibrierten Impfkultur in den Messvorrichtungen, die zu der Bestimmung von Antibiogrammen dienen oder in einigen Messvorrichtungen, die zur Durchführung der kombinierten Bestimmungen von Antibiogramm und Identifizierung dienen, durch eine erste Inkubation ausgeführt wird, die Vorinkubation genannt wird, in deren Verlauf die in der Impfkultur vorhandenen Bakterien ohne dass sie Reagenzien ausgesetzt werden inkubiert werden, und dieses solange bis ein Anfang bakteriellen Wachsens messbar ist oder bis die bakterielle Konzentration einen vorbestimmten Grenzwert erreicht, wobei nach diesem vorläufigen Wachsen die Impfkultur mit den Reagenzien in Kontakt gebracht wird und eine endgültige Inkubation folgt, in deren Verlauf ihre Interaktionen beobachtet werden.

9. Verfahren nach irgendeinem der Ansprüche 1 und 5, **dadurch gekennzeichnet, dass** das Aufteilen der Impfkultur auf die Zellen (32, 37, 45; 132, 137, 145), die an der Peripherie der Messvorrichtungen (4, 5; 104, 105) angeordnet sind, mittels Zentrifugieren durchgeführt wird, dabei werden die Messungen und die Beobachtungen, die an den Zellen (32, 37, 45; 132, 137, 145) erfolgen, durchgeführt, indem die Messvorrichtungen (4, 5; 104, 105) um die zentrale Zentrifugierachse der Vorrichtungen in Drehbewegung versetzt werden.

10. Verfahren nach irgendeinem der Ansprüche 1, 5, 8 und 9, **dadurch gekennzeichnet, dass** während des vorläufigen Wachsens in einer Messvorrichtung (4; 104) für das Antibiogramm, die Messung der Konzentration der bakteriellen Impfkultur und die Bestimmung des Wachstumbeginnes durch photometrische Messungen ausgeführt werden, die an einem aliquoten Bereich der zu diesem Zweck übertragenen Impfkultur durchgeführt werden, durch ein erstes Zentrifugieren in einer speziellen Peripheriezelle (37; 137), die der Charakterisierung des Wachsens während der Vorinkubation gewidmet ist und die keine Reagenzien enthält, wobei der Rest der Impfkultur während der Vorinkubation in einer konzentrischen, mittleren Kammer (27; 127) der Vorrichtung (4; 104) eingeschlossen bleibt, bevor sie nach der Beendigung des vorläufigen Wachsens in die anderen Peripheriezellen (32; 132) durch ein zweites Zentrifugieren übertragen wird, dem ein Autorisierungsvorgang oder ein entsprechender Entsicherungsvorgang vorausgeht, der den Verbindungsgang für die Übertragung der verbliebenen Impfkultur zu den anderen Zellen (32; 132), die Reagenzien enthalten, freigibt.

11. Verfahren nach irgendeinem der Ansprüche 1, 5, 9 und 10, **dadurch gekennzeichnet, dass** der Übergang von der Vorinkubationsphase zur Inkubationsphase ohne Unterbrechung der Einschließung des Inneren der betreffenden, rotierenden Messvorrichtung (4; 104) im Verhältnis nach außen stattfindet, dabei verbleibt die Vorrichtung verschlossen und der Übergang geschieht durch einen Vorgang für die Vorrichtung (4), oder eine Unterbrechung eines Vorganges für die Vorrichtung (104), an einer Verschlusseinrichtung, die an der Ausführung dieser Einschließung beteiligt ist.

12. Verfahren nach irgendeinem der Ansprüche 1 bis 11, **dadurch gekennzeichnet, dass** die Messungen durch Trübungsanalyse für die Messvorrichtungen (4; 104), die für das Antibiogramm vorgesehen sind, und durch Lichtmessung oder Bestimmung des Absorptionskoeffizienten oder der optischen Dichte für die Messvorrichtungen (5; 105), die zur Identifizierung oder zu kombinierten Bestimmungen der Identifizierung/Antibiogramm dienen, oder sogar noch durch Fluoreszenzanalyse, nach vorheriger Zugabe einer fluorogenen Markierung zu dem Reagenzstoff, durchgeführt werden.

13. Messvorrichtung in Form einer Scheibe, die einen festen Hauptkörper umfasst, der, in Form von nicht kommunizierenden Abteilungen, eine mittlere Kammer und mehrere periphere Zellen begrenzt, die jeweils einer Auffangnische zugeordnet sind, **dadurch gekennzeichnet, dass** der Hauptkörper (126) eine obere Wand (128) aufweist, die mit mehreren, durchgehenden Öffnungseinheiten zur Übertragung (129, 130, 131, 133) versehen ist, welche jeweils in die mittlere, zylindrische Kammer (127), in jede Auffangnische (146), die jeder Zelle (132, 145) zugeordnet ist, und in jede der Zellen (132, 145) einmünden, und die mit Ausnahme eines zentralen Bereiches, der die Einfüllöffnung (134) der mittleren Kammer (127) und des Gebietes der Messfenster der Zellen (132, 145) umgibt, durch eine genau anliegende, dichte, schwimmende Membrane (135) abgedeckt wird, die örtlich und insbesondere an ihrer äußeren Peripherie und um die Einfüllöffnung (134) hermetisch befestigt ist, und die ganz eng gegen die Oberseite der oberen Wand (128) gedrückt werden kann, um vorübergehende, dichte Kontaktbereiche (136, 138, 142) auszubilden, die sich dazwischen erstreckt oder verschiedene der durchgehenden Öffnungseinheiten (129, 131, 131, 133) abdeckt, insbesondere während der Rotation der Messvorrichtung.

14. Messvorrichtung nach Anspruch 13, **dadurch gekennzeichnet, dass** die obere Wand (128), in Höhe ihrer Oberseite, Rillen oder konzentrische, ringförmige Rücksprünge (140, 140') aufweist, in die die durchgehenden Öffnungen (129, 130, 131, 133) einmünden, die nach konzentrischen, beabstandeten Kreisen angeordnet sind, wobei die innere Rille (140) eine Verstärkungsrippe (136) umfasst, die eine ringförmige Kontaktfäche ausbildet und die Ausgangsöffnungen (129) der mittleren Kammer (127) von den Eingangsöffnungen (130) der konzentrischen, ringförmigen Kammer (147), die die radialen Auffangnischen (146) umfasst, abtrennt, und die Ausgangsöffnungen (131) der Auffangnischen (146) münden in Höhe der Oberseite einer konzentrischen, kreisförmigen Verstärkungsrippe oder von deutlich vorstehenden Ausgestaltungen (138) ein, beispielsweise in Form von Kegeln, die in der äußeren Rille (140') angeordnet ist (sind), dabei sind die oberen Enden der Verstärkungsrippe (136) und der Ausgestaltungen (138) in einer Ebene oder in Ebenen angeordnet, die sich leicht zurückgesetzt von der Klebefläche der genau anliegenden, dichten Membrane (135) erstreckt.

15. Messvorrichtung nach irgendeinem der Ansprüche 13 und 14, **dadurch gekennzeichnet, dass** sie eine Zelle zur Vorinkubation (137) umfasst, die mit den anderen peripheren Zellen (132, 145) identisch und die kreisförmig mit diesen letzteren ausgerichtet ist, und die mit einer verschlossenen Vorkammer (143) kommuniziert, die in der konzentrischen Kammer (147) ausgebildet ist, die die Nischen zum Auffangen und zur gleichmäßigen Aufteilung (146) einschließt, und die von einer inneren Auffangnische (144) aus, die sich in Höhe eines seitlichen, peripheren Wandabschnittes (139) der mittleren Kammer (127) befindet, mittels eines durch die dichten Kontaktbereiche (136, 138, 142) nicht verschließbaren Verbindungsganges, die zwischen der genau anliegenden Membrane (135) und der oberen Wand (128) gebildet werden können, mit Impfkultur gespeist wird.

16. Messvorrichtung nach Anspruch 15, **dadurch gekennzeichnet, dass** der Versorgungsdurchgang der Vorkammer (143), die frei mit der Vorinkubationszelle (137) kommuniziert und ebenfalls einen Überlauf für die Auffangnischen (146) ausbildet, aus einer Leitung (157) besteht, die in dem oberen Bereich des seitlichen, peripheren Wandabschnitts (139) beginnt, der die innere Auffangnische (144) begrenzt, und die aus dem Zusammenwirken einer Rille, die in dem oberen Abschnitt einer radial vorstehenden Verlängerung (158) der seitlichen, peripheren Wand (139) der mittleren Kammer (127) und der oberen Wand (128) angeordnet ist, gebildet wird, und die in oder an dem Eingang einer Rille (148) mit radialer, zentripalen Ausdehnung einmündet, die in die Oberseite der oberen Wand (128) gerieft ist, und eine dichte Leitung durch Zusammenwirken mit der Membrane (135) ausbildet, die auf beiden Seiten der Rille (148) angeklebt ist und deren distales Ende in Höhe einer durchgehenden Öffnung (148') endet, die in die Vorkammer (143) einmündet.

17. Messvorrichtung nach irgendeinem der Ansprüche 13 bis 16, **dadurch gekennzeichnet, dass** die Einfüllöffnung (134) der mittleren Kammer (127) in einem Rücksprung (134') der oberen Wand (128) angeordnet und mittels eines Stopfens (150) verschließbar ist, dessen oberer Abschnitt im Wesentlichen in Höhe der Oberseite der oberen Wand (128) abschließt, dadurch dass der Boden der mittleren Kammer (127) von einer konzentrischen Hülse (151) zum Ergreifen und zur Verstärkung umgeben ist, die in Höhe der Unterseite der Vorrichtung (104, 105) vorsteht, und dadurch, dass eine durchgehende Öffnung (152), die identische Abmessungen wie diejenigen einer Zelle (132, 145) aufweist und in kreisförmiger Ausrichtung mit diesen letzteren angeordnet ist, in dem Hauptkörper (126) und in der oberen Wand (128) ausgebildet ist, wobei die Öffnung (152) vorzugsweise neben der Vorinkubationszelle (137) angeordnet ist.

18. Messvorrichtung nach irgendeinem der Ansprüche 13 bis 17, **dadurch gekennzeichnet, dass** die obere Wand (128) über jeder Zelle (132, 137, 145) und der Nähe ihres äußeren Randes mit einem Messfenster versehen ist, wobei ein analoges Fenster jeweils in den Wandbereichen des Hauptkörpers (126) vorgesehen ist, der jeweils die Böden der Zellen ausbildet, und das jedem vorgenannten Messfenster gegenüberliegt, und wobei mindestens ein reflektierendes Gebiet zur Indizierung, das auf der Außenseite der peripheren Außenwand des Hauptkörpers (126) ausgebildet ist, jeweils mit der Zelle (132, 137, 145) mit einem festen Winkelversatz im Verhältnis zu dieser letzten, assoziiert ist.

19. Gerät zur Anwendung des Verfahrens nach irgendeinem der Ansprüche 1 bis 12, im Wesentlichen gebildet aus einem ersten Modul (102) zur Halterung und zur Stapelung von frischen Messvorrichtungen (104, 105) nach irgendeinem der Ansprüche 13 bis 18, und von Primärbehältern (101), die jeweils mit einem entsprechenden Entnahme- und Übertragungsinstrument (101') zusammenarbeiten, aus einem zweiten Modul (103) zur Inkubation der Vorrichtungen (104 und 105), nach der Injektion der bakteriellen Impfkultur, aus einem dritten Modul (106) zur Zentrifugierung und zu Messungen per Trübungsanalysen und/oder Lichtmessungen und/oder Fluoreszenzanalysen, aus einem oder mehreren Einrichtungen (107, 107') zur Übertragung und zur Manipulation, die die Module (102, 103 und 106) versorgen und aus einer Computervorrichtung (108) zur Überwachung und zur Steuerung der verschiedenen Verfahrensschritte, und zur Erfassung, zur Speicherung, zur Verarbeitung, zur Auswertung und/oder zur Wiedergabe von Daten, wobei die zweiten und die dritten Module (103) und (106) in einem Einschließungsraum angeordnet sind, Gerät, **dadurch gekennzeichnet, dass** die Primärbehälter (101) auf einer kronenförmigen Halterungseinrichtung (109) gelagert sind, die sich konzentrisch um und unter einer Halterungseinrichtung (110) in Form einer Scheibe erstreckt, auf der die Messvorrichtungen (104, 105) gelagert werden, wobei die beiden Halterungseinrichtungen (109, 110) so befestigt sind, dass sie die Fähigkeit aufweisen, um ein und dieselbe Achse zu rotieren, und dass jeder Primärbehälter (101) durch einfache Rotation der kronenförmigen Halterungseinrichtung (109) in Höhe einer Manipulationsstation (111) des zugeordneten Entnahme- und Übertragungsinstrumentes (101') und zur Messung der optischen Dichte des Inhaltes des Primärbehälters (101) gebracht werden kann.

20. Gerät nach Anspruch 19, **dadurch gekennzeichnet, dass** jeder Primärbehälter (101), zum Aufnehmen eines bestimmten Volumens an Bakterienkolonie und zum Präparieren einer mindestens vorkalibrierten Impfkultur, sich in der Form eines zylindrischen Behälters, der in Höhe seines oberen Abschnittes offen ist, darstellt, welcher mit einem radialen Rand (112) versehen ist, um den Primärbehälter (101) in einer der entsprechenden Aufnahmen (109') zu halten, die in der beweglichen, kronenförmigen Halterungseinrichtung (109) ausgebildet ist (sind), und dass das Entnahme- und Übertragungsinstrument (101') aus einer Spritze besteht, deren Körper (113) einen vorderen sich verjüngenden Bereich zum Entnehmen und zum Ausspritzen (113') und einen hinteren im Wesentlichen zylindrischen Bereich (113'') zum Aufbewahren und zum Ergreifen umfasst, wobei der hintere Bereich (113'') in Höhe seines an die vordere Wand (113') angrenzenden Endes zum teilweisen Einpassen durch Blockierung des Körpers (113) in dem Primärbehälter (101) mindestens einen radialen, äußeren Absatz (114) aufweist, wobei das freie, vordere Ende des vorderen Bereiches (113') in geringer Entfernung von dem Boden des Primärbehälters (101) angeordnet ist.

21. Gerät nach Anspruch 20, **dadurch gekennzeichnet, dass** die Spritze (101') in Höhe ihres vorderen Endes Mittel (118, 118') zum Entnehmen, in Volumen gesehen in Abhängigkeit von der Stellung ihres Kolbens (115), einer reduzierten Menge oder einer normalen Menge von der Bakterienkolonie umfasst.

22. Gerät nach irgendeinem der Ansprüche 19 bis 21, **dadurch gekennzeichnet, dass** jeder Primärbehälter (101) in seiner Seitenwand in der Nähe seines Bodens, zwei sich gegenüberliegende Messfenster (119, 119') umfasst, die zur Messung der optischen Dichte des Inhaltes des Primärbehälters (101) in Höhe der Station (111) ausgebildet sind.

23. Gerät nach irgendeinem der Ansprüche 20 bis 22, **dadurch gekennzeichnet, dass** die Manipulationseinrichtung (121) der Spritze, die das Entnahmeinstrument (101') in Höhe der Station (111) darstellt, aus zwei Klemmbacken-Einheiten (121' und 121'') besteht, die zwei äußere Klemmbacken (121') umfassen, die dazu vorgesehen sind, mit einem radialen Rand (128) in Höhe der Öffnung des hinteren Bereiches (113'') des Körpers (113) starr und blockierend in Eingriff zu gelangen, und zwei innere Klemmbacken (121''), konzentrisch zwischen den zwei äußeren Klemmbacken (121') angeordnet, die dazu vorgesehen sind, mit einer Verstärkung (122') der Betätigungsstange (116) starr und blockierend in Eingriff zu gelangen, wobei das Schließen der beiden Klemmbacken-Einheiten (121', 121' ') gleichzeitig durch Gleiten einer Hülse (121''') um und entlang der äußeren Klemmbacken (121') ausgeführt wird, wobei die inneren Klemmbacken (121'') insbesondere in der Schließposition im Verhältnis zu den äußeren Klemmbacken (121') und zu der Hülse (121''') gleiten können und diese letztere kann sich auf der beweglichen, kronenförmigen Halterung (109) und auf dem radialen Rand (112) der oberen Öffnung des betreffenden Primärbehälters (101) abstützen, auf eine Weise, dass dieser letztere in der Position in seiner Aufnahme (109') während des Gleitens von irgendeiner der beiden Klemmbacken-Einheiten (121', 121'') blockiert wird.

24. Gerät nach irgendeinem der Ansprüche 19 bis 23, **dadurch gekennzeichnet, dass** es auf der einen Seite eine erste Einrichtung zur Manipulation und zur Übertragung (107) umfasst, die die Halterungen (109 und 110) versorgt und die sich in Form eines Manipulationsarmes darstellt, der rotationsfähig und gleitfähig an einer ersten vertikalen Achse (123) befestigt ist und zwei Klemmbacken zum Er-. greifen (124, 124') umfasst, von denen die erste (124) mit einem Greifinstrument (125) eines Primärbehälters (101) ausgestattet ist und von denen die zweite (124') eine gekrümmte Form oder einen Haken aufweist, der dazu vorgesehen ist, an zwei bestimmten Stellen einer Messvorrichtung (104, 105) einzugreifen, auf eine Weise, dass dieser letztere durch untere Abstützung und durch Einklemmung in Zusammenwirken mit der ersten Klemmbacke zum Ergreifen (124) gehalten wird, und, auf der anderen Seite eine zweite Einrichtung zur Manipulation und zur Übertragung (107'), die das Modul (103) zur Inkubation und das Modul (104) zur Zentrifugierung und zur Messung versorgt, und die Form eines Stützarmes aufweist, der rotationsfähig und gleitfähig an einer zweiten vertikalen Achse (123') befestigt ist, und an seinem freien Ende ein Annahmeinstrument (125') umfasst, das durch Zuordnung der Form mit mindestens einem Teil der Unterseite der Messvorrichtungen (104, 105) zusammenwirkt, wobei diese beiden Einrichtungen zur Manipulation und zur Übertragung (107, 107') einen Überlappungsbereich in ihren jeweils erreichbaren Arbeitsräumen oder Arbeitsgebieten aufweisen, in dessen Höhe insbesondere die Übertragung der Messvorrichtungen (104, 105) von einer zur anderen erfolgen kann.

25. Gerät nach irgendeinem der Ansprüche 19 bis 24, **dadurch gekennzeichnet, dass** das dritte Modul (106) mehrere Arbeitsstationen (153) umfasst, insbesondere zur Messung und zur Zentrifugierung, die jeweils zusätzlich zu den verschiedenen Messeinrichtungen durch Trübungsanalyse, durch Lichtmessung und/oder durch Fluoreszenzanalyse, einerseits eine scheibenförmige Stützbasis (154) umfasst, die von dem oberen Ende eines vertikalen Schaftes (154') abgestützt wird, der rotierend angetrieben wird und durch Verrasten oder mittels Aufrechterhaltung radialer Reibung in die konzentrische Hülse (151) einer Messvorrichtung (104, 105) eingreifen kann, und andererseits eine Druckvorrichtung (155), die im Wesentlichen scheibenförmig ist und zur Rotation um ihre Symmetrieachse durch Reibung auf der Messvorrichtung (104, 105) angetrieben wird, wobei die Druckvorrichtung (155) ein oder mehrere ringförmige Druckinstrumente (156, 156', 156'') umfasst, die von einer hohen Stellung, in welcher die Druckinstrumente nicht mit der Messvorrichtung (104, 105) in Kontakt sind, die auf der Stützbasis (154) angeordnet ist, zu verschiedenen, niedrigeren Stellungen und umgekehrt verschoben werden können, in denen sie aufeinanderfolgend auf der genau anliegenden, schwimmenden Membrane (135) in einer bestimmten Reihenfolge (156'', 156', 156) zum Aufliegen kommen, wobei diese letztere sich selbst in Höhe der dichten Kontaktbereiche, die kreisförmig oder ringförmig, konzentrisch oder in kreisförmigen oder in konzentrischen, ringförmigen Konfigurationen angeordnet sind, auf der oberen Wand (128) der betreffenden Messvorrichtung (104, 105) abstützt.

26. Gerät zur Anwendung des Verfahrens nach irgendeinem der Ansprüche 5 bis 12, im Wesentlichen ein erstes Modul zur Entgegennahme und zur Stapelung der frischen und gebrauchten Messvorrichtungen (4 und/oder 5) umfassend, ein zweites Modul (3) der Inkubation der Vorrichtungen (4 und/oder 5), ein drittes Modul (6) für Messungen durch Trübungsanalysen und/oder Lichtmessungen und/oder Fluoreszenzanalysen, ein oder mehrere Übertragungseinrichtungen (7, 7'), die die Module (2, 3 und 6) versorgen und eine Computervorrichtung (8) zur Überwachung und Steuerung der verschiedenen Verfahrensschritte, und zur Erfassung, zur Speicherung, zur Verarbeitung, zur Auswertung und/oder zur Wiedergabe von Daten, **dadurch gekennzeichnet, dass** das erste Modul (2) zur Entgegennahme und zur Stapelung der frischen und gebrauchten Messvorrichtungen (4 und/oder 5) außerdem mindestens eine Vorrichtung (59) zur Entgegennahme und zur Stapelung der Primärbehälter (1) umfasst, die die bakterielle Starterimpfkultur enthalten und eine Vorrichtung zur Kalibrierung (60, 61) der Impfkultur in den Primärbehältern (1) und zum Präparieren der Messvorrichtungen (4 und/oder 5).

27. Gerät nach Anspruch 26, **dadurch gekennzeichnet, dass** sich der Primärbehälter (1) zur Entgegennahme der Impfkultur in Form eines zylindrischen Behälters darstellt, der an seinem Boden mit mindestens einer Entlüftungsröhre (9), einer Überlaufablaufleitung (10), möglicherweise einer Leitung (10') zur Einstellung auf einen niedrigeren Stand des Impfkulturvolumens, eines Entnahmeinstrumentes einer vorbestimmten Impfkulturmenge, das abnehmbar durch Herausziehen oder Einpassen in den Boden des Behälters (1) befestigt und aus dem Zentrum im Verhältnis zur Achse des Behälters (1) versetzt ist, so wie eine Pipette (11), und einer querliegenden Abteilung (12), die sich als Mulde zwischen den Wänden des Behälters (1) erstreckt, der jeweils an dem Ende der Abteilung (12) mit einem Messfenster (13) versehen ist, wobei diese Abteilung (12) außerdem mit einem erhabenen Bereich (12') versehen ist, der mit der Abteilung (12) kommuniziert, wobei die Entlüftung (9), die Überlaufleitung (10), die Leitung (10') zur Einstellung auf einen niedrigeren Stand und das Entnahmeinstrument (11) durch einen wegreißbaren Film (14) dicht verschlossen sind, wobei ein Verschlussdeckel (15) durch konische, dichte Einpassung mit dem oberen, konischen Bereich (16) des Behälters (1) zusammenwirkt.

28. Gerät nach Anspruch 27, **dadurch gekennzeichnet, dass** der obere, konische Bereich (16) des Primärbehälters (1) mit mindestens einer Ausnehmung (17) von geringer Tiefe und mit zwei Ausnehmungen (18) von größerer Tiefe und unterschiedlicher Breite versehen ist, die untereinander ausgerichtet und im Winkel von 90° im Verhältnis zu der Achse angeordnet sind, die durch die Ausnehmung oder die Ausnehmungen (17) von geringer Tiefe verläuft, diese Ausnehmung oder diese Ausnehmungen von geringer Tiefe (17) sind in der Achse der Abteilung (12) des Bodens ausgerichtet und die Ausnehmungen (18) von größerer Tiefe, die mit Laschen (19) von entsprechendem Querschnitt zusammenwirken, die an dem dichten Verschlussdeckel (15) vorgesehen sind, der einerseits auf seiner Oberseite mit einer Fläche (20), die sich in der Achse der Laschen (19) erstreckt und andererseits, mit einer konischen Wand (21) versehen ist, die eine entsprechende Form zu der des oberen, konischen Bereiches (16) des Behälters (1) aufweist und die Laschen (19) verbindet.

29. Gerät nach Anspruch 26, das eine rotierende Vorrichtung (4) für das Antibiogramm anwendet, die sich in Form einer zusammengesetzten, oberen Scheibe darstellt, die mit peripheren Zellen (32) zur Entgegennahme der Impfkultur und der antibiotischen Präparate ausgestattet ist, **dadurch gekennzeichnet, dass** die rotierende Messvorrichtung (4) mit einem zylindrischen, konzentrischen Körper, von kleinerem Durchmesser als die Scheibe und mit der Scheibe verbunden, versehen ist, und eine mittlere Kammer (33) zur Entgegennahme der Impfkultur ausbildet, für die Verteilung durch Zentrifugieren, wobei die Kammer (33) durch eine Öffnung (23) in der Oberseite der oberen, zusammengesetzten Scheibe gespeist wird und von einer Hülse (35) zum Ergreifen und zur Verstärkung der Einheit umgeben ist.

30. Gerät nach irgendeinem der Ansprüche 26 und 29, **dadurch gekennzeichnet, dass** die obere, zusammengesetzte Scheibe der rotierenden Vorrichtung für das Antibiogramm (4), einerseits einen kreisförmigen Deckelabschnitt (22) umfasst, der in seiner Mitte mit einer Öffnung (23) versehen ist, und andererseits eine kreisförmige Plattform (31), deren Oberseite, die zu der Unterseite des Deckelabschnitts (22) ausgerichtet ist, im Relief an ihrer Peripherie die Zellen (32) zur Entgegennahme der Impfkultur und der antibiotischen Präparate aufweist, wobei diese kreisförmige Plattform (31) an der konzentrischen Kammer (33) angeschlossen ist, die von einem Boden (34) verschlossen wird, wobei der Deckelabschnitt (22) mit einem Rand (22') versehen ist, der sich konzentrisch über eine kreisförmige Wand (31') der kreisförmigen Plattform (31) erstreckt, die die Zellen im Relief (32) nach der Außenseite begrenzt, und die durch Verkleben oder Verschweißen an der kreisförmigen Wand (31') befestigt ist.

31. Gerät nach Anspruch 30, **dadurch gekennzeichnet, dass** die kreisförmige Plattform (31) des unteren Körperbereiches zwischen den Zellen im Relief (32) mit mindestens einer Zelle zur Vorinkubation (37) versehen ist, die mittels einer Kapillare (38) mit einer konzentrischen Kammer (27) verbunden ist, die unter der kreisförmigen Plattform (22) des Deckelabschnitts, in der konzentrischen Kammer (33) der kreisförmigen Plattform (31), die den unteren Körperbereich ausbildet, angeordnet ist.

32. Gerät nach irgendeinem der Ansprüche 30 und 31, **dadurch gekennzeichnet, dass** die Öffnung (23), die in der Mitte des Deckelabschnitts (22) vorgesehen ist in einer Vertiefung (25) angeordnet ist, die sich konzentrisch im Innern der konzentrischen Kammer (27) erstreckt, die eine bewegliche Schale (28) aufweist, die mit einem Trockennährboden versehen ist, wobei die bewegliche Schale (28) eine dichte Führungswand (29) aufweist, die mit der beweglichen Schale (28) eine Schüssel begrenzt, wobei die Vertiefung (25) durch einen dichten Film (26) verschlossen ist, der den oberen Teil der kreisförmigen Plattform des Deckels bedeckt und gleichzeitig die Kapillare (38) begrenzt.

33. Gerät nach Anspruch 26, das eine rotierende Vorrichtung (5) zur Identifizierung anwendet, die sich in Form einer oberen, zusammengesetzten Scheibe darstellt, die mit peripheren Zellen (45) zur Entgegennahme von Impfkultur und Reagenzstoff zur Charakterisierung versehen ist,
**dadurch gekennzeichnet, dass** die rotierende Vorrichtung (5) zur Identifizierung mit einem zylindrischen, konzentrischen Körper, von kleinerem Durchmesser als die Scheibe und mit der Scheibe verbunden, versehen ist, dabei eine konzentrische Hülse (46) zur Entgegennahme des Primärbehälters (1) in umgedrehter Stellung ausbildet, für die Verteilung der Impfkultur durch Zentrifugierung, wobei sich diese konzentrische Hülse (46) in der Mitte unter der Unterseite der kreisförmigen Plattform (44) erstreckt, dabei mit dieser letzteren verbunden ist und an ihrem freien Ende mit einem Abschnitt der konischen Wand (47) versehen ist, der sich innerhalb der Hülse (46) erstreckt, und der dazu vorgesehen ist, mit der Außenwand des oberen konischen Bereiches (16) des Primärbehälters (1) zusammenzuarbeiten und von einer konzentrischen Hülse (48) zum Ergreifen und zur Verstärkung der Einheit umgeben ist.

34. Gerät nach Anspruch 33, **dadurch gekennzeichnet, dass** die obere, zusammengesetzte Scheibe der rotierenden Vorrichtung zur Identifizierung (5) einerseits einen kreisförmigen Deckelabschnitt (43) und andererseits einen unteren Abschnitt in Form einer kreisförmigen Plattform (44) umfasst, die auf ihrer Oberseite, die zu der Unterseite des Deckelabschnitts (43) ausgerichtet ist, im Relief an ihrer Peripherie Zellen (45) zur Entgegennahme der Impfkultur und der Reagenzien zur Charakterisierung aufweist, wobei der Deckel (43) mit einem Rand (43') versehen ist, der sich konzentrisch über die kreisförmige Wand (44') der kreisförmigen Plattform (44) erstreckt, und die Zellen im Relief (45) nach der Außenseite begrenzt, und die durch Verkleben oder Verschweißen an der kreisförmigen Wand (44') befestigt ist.

35. Gerät nach irgendeinem der Ansprüche 30 bis 34, **dadurch gekennzeichnet, dass** Mittel zum Auffangen der Impfkultur (45'') während der Zentrifugierung, die in radialer Richtung gesehen vor jeder Zelle (32, 45), jeweils der Vorrichtungen (4 und 5) vorgesehen sind, jeweils mit dem Inneren einer entsprechenden Zelle (32, 45) mittels einer Kapillare (36, 49) verbunden sind, und ansonsten über einem inneren Raum (30, 43'') offen sind, der mit der konzentrischen Kammer (33) der kreisförmigen Plattform (31), die den unteren Körperbereich ausbildet oder mit dem Raum zur Entgegennahme des Primärbehälters (1) in der konzentrischen Hülse (46) kommuniziert, die sich unter der Unterseite der kreisförmigen Plattform (44) erstreckt, die den unteren Abschnitt der zusammengesetzten Scheibe ausbildet, wobei die Zellen im Relief (32, 45) ansonsten jeweils mittels einer radialen Kapillare (39, 50) an eine kreisförmige Kapillare (40, 51) angeschlossen sind, die mittels einer anderen Kapillare (41, 52), an der Außenseite der kreisförmigen Plattform (22) des Deckels oder des kreisförmigen Deckelabschnitts (43) einmündet, wobei die Zelle zur Vorinkubation (37), die in der Messvorrichtung (4) für das Antibiogramm vorgesehen ist, direkt durch eine entsprechende Durchgangslochung an die kreisförmigen Kapillaren (40) angeschlossen ist, wobei diese verschiedenen Kapillaren an dem oberen Bereich der kreisförmigen Plattform (22) und des Deckels (43) durch einen dichten Film (26 oder 52') begrenzt sind, wobei die Kapillaren (41 und 52) an ihrem Endbereich, der der zentralen Achse der Scheiben (31 und 44) am nächsten liegt, mit den inneren Kammern (27 und 43'') über durchgestoßene Lochungen in den kreisförmigen Plattformen (22 und 43) kommunizieren.

36. Gerät nach irgendeinem der Ansprüche 30 bis 32, 34 und 35, **dadurch gekennzeichnet, dass** die kreisförmige Plattform des Deckels (22) und der kreisförmige Deckel (43) außerdem oberhalb jeder Zelle (32, 45) und in der Nähe des Randes mit einem Messfenster (42, 53) versehen sind, wobei ein analoges Fenster (43, 54) unter der Unterseite der kreisförmigen Plattform (31, 44), die den unteren Körperbereich oder den Bereich der zusammengesetzten Scheibe ausbildet, jedem Messfenster (42, 53) gegenüberliegend vorgesehen ist, und mindestens ein reflektierendes Gebiet zur Indizierung (42', 53'), das auf der Außenseite der peripheren, äußeren Wand der Vorrichtungen (4, 5) ausgebildet ist, ist jeweils einer Zelle (32, 45) mit einem festen, winkelförmigen Versatz im Verhältnis zu dieser letzteren zugeordnet.

37. Gerät nach Anspruch 36, **dadurch gekennzeichnet, dass** für das automatische Zuführen einer bakteriellen Starterimpfkultur zu einer Messvorrichtung für das Antibiogramm oder für gleichzeitige, kombinierte Analyse, der Primärbehälter (1') sich in Form eines Gefäßes darstellt, das einerseits mit einer Pipette (11), die mit der identisch ist, die zu dem Primärbehälter (1) gehört, und andererseits mit einer Makropipette (73) zum Übertragen der Gesamtheit des Behälterinhaltes (1') ausgestattet ist, wobei diese Makropipette von dem Primärbehälter (1') abgenommen werden kann, um zum Ausleeren ihres Inhaltes in die Vorrichtung (4) verwendet zu werden, dann in den Primärbehälter (1) wieder eingesetzt werden kann, wobei die Pipette (11) anschließend abgenommen und verwendet werden kann, um die Vorrichtung (4) zu Verschließen, indem sie in die Vertiefung (25) eingesetzt wird.

## Claims

1. Process for bacteria identification and determination of the sensitivity of bacteria to antibiotics in antibiogram form, **characterized in that** it involves manually introducing, by means of a sampling tool, a given volume of bacterial colony into a primary receiver (1; 101), automatically dispersing this bacterial colony within a liquid to form an at least precalibrated inoculum in said primary receiver (1; 101), also automatically carrying out total or partial transfer of this precalibrated inoculum between said primary receiver (1; 101) and one or more measuring supports (4, 5; 104, 105) intended to carry out respectively the identification, the antibiogram or these two determinations simultaneously in a single combined operation, said transfers being carried out without the precalibrated inoculum being placed in contact with an element other than, on the one hand, a sampling and transfer tool (101'; 1, 101) and/or the primary receiver (1; 101) and, on the other hand, its final measuring support or supports (4, 5; 104, 105) and in such a way that the transferred quantities of bacteria correspond to the quantities required for the analyses to be carried out, automatically distributing the precalibrated inoculum, optionally after having appropriately diluted it to end up with a definitive calibration in one or more compartments (32, 37, 45; 132, 137, 145) of said measuring supports (4, 5; 104, 105) mainly containing appropriate reagents, carrying out in certain cases, in particular for the antibiogram, within a measuring support (4; 104) a so-called pre-growth operation intended to enable the bacteria to multiply rapidly before being subjected to analysis and taking measurements on the content of said compartments (32, 37, 45; 132, 137, 145) during or at the end of one or more incubations undergone by the inoculum in said measuring supports (4, 5; 104, 105), the measurements taken being registered by a computer (8; 108) and being processed in order to characterize the growth of the bacteria present in the inoculum, to identify them and/or to determine their sensitivity to various antibiotics.

2. Process according to Claim 1, **characterized in that** the sampling of a given volume of bacterial colony and the transfers of the volumes of precalibrated inoculum into the measuring supports (104, 105) are carried out by means of a single sampling and transfer tool (101') cooperating with the primary receiver (101), the volumes of precalibrated inoculum to be transferred being determined by optical measurement of the bacterial concentration of said inoculum carried out in the primary receiver (101) and calculated as a function of the quantities of bacteria required.

3. Process according to any one of Claims 1 and 2, **characterized in that** the sampling and introduction into the primary receiver (101) of a given volume of bacterial colony are carried out by means of the sampling and transfer tool (101') after the shaping thereof in order to sample the desired volume, and the transfers of precalibrated inoculum from the primary receiver (101) to the measuring supports (104, 105) are carried out by pipetting predetermined volumes of inoculum.

4. Process according to any one of Claims 1 to 3, **characterized in that** the transfer of the inoculum within the measuring supports (104, 105), in particular to the compartments (132, 145), is monitored dynamically by controlling the opening or the sealed blockage of successive passages leading progressively to said compartments (132, 145).

5. Process according to Claim 1, **characterized in that** it involves manually introducing a given volume of initial bacterial inoculum into a primary receiver (1) using a sampling and transfer tool, automatically carrying out definitive calibration of said inoculum in said primary receiver (1) by bringing its bacterial concentration and its volume to set values, also automatically carrying out total or partial transfer of this calibrated inoculum between said primary receiver (1) and one or more measuring supports (4, 5) intended to carry out respectively the identification, the antibiogram or these two determinations simultaneously in a single combined operation, said transfers being carried out without the initial inoculum being placed in contact with an element other than its initial receiver and its final receiver or receivers and in such a way that, at the outlet thereof, the calibrated inoculum remains confined within said measuring supports (4, 5) which are thus closed, automatically distributing the inoculum in one or more compartments (32, 45) of said measuring supports (4, 5) mainly containing appropriate reagents, carrying out, in certain cases, in particular for the antibiogram, within a measuring support (4), a so-called pre-growth operation intended to enable the bacteria to multiply rapidly before they are subjected to analysis and taking photometric measurements on the content of said compartments (32, 45) during or at the end of one or more incubations undergone by the inoculum in said measuring supports (4, 5), the measurements taken being registered by a computer (8) and being processed in order to characterize the growth of the bacteria present in the inoculum, to identify them and/or to determine their sensitivity to various antibiotics.

6. Process according to Claim 5, **characterized in that** the automatic calibration of the inoculum in the primary receiver (1) involves injecting, from an initial inoculum containing a quantity of bacteria in excess relative to the reaction requirements, the excess being checked by visual observation of the turbidity of the initial inoculum, diluting water into said primary receiver (1) so as to create an increased volume of more dilute inoculum, then eliminating a proportion of the inoculum diluted in this way by aspiration until the required final volume of calibrated inoculum is obtained, these operations being carried out and optionally being repeated on the basis of optical measurements taken through the primary receiver (1) on the inoculum in order to determine the concentration thereof until the desired concentration and volume for the calibrated inoculum are both obtained.

7. Process according to any one of Claims 5 and 6, **characterized in that** the transfer of the calibrated inoculum between the primary receiver (1) and the measuring support or supports (4, 5) is carried out by fitting all or part (11) of the primary receiver (1) containing the inoculum into a cavity provided for this purpose in each support (4, 5) so as to transfer to it the desired calibrated quantity of inoculum contained in the fitted portion of said primary receiver, this fitting ensuring the isolation necessary for the confinement of the inoculum in the unit formed by the measuring support (4, 5) and the primary receiver (1) or the part (11) of the primary receiver (1) fitted in said support (4, 5) as well as the closure of this unit.

8. Process according to any one of Claims 1 and 5, **characterized in that** the pre-growth of the calibrated inoculum is carried out in the measuring supports used for the determination of the antibiograms or in certain measuring supports used for carrying out combined determination of antibiogram and identification, by a first incubation known as pre-incubation, during which the bacteria present in the inoculum are incubated without being placed in the presence of reagents until a beginning of bacterial growth is measurable or until the bacterial concentration reaches a predefined level, this pre-growth being followed by putting the inoculum in contact with the reagents and final incubation during which their interactions are observed.

9. Process according to any one of Claims 1 and 5, **characterized in that** the distribution of the inoculum in the compartments (32, 37, 45; 132, 137, 145) situated at the periphery of the measuring supports (4, 5; 104, 105) is carried out by centrifugation, the measurements and observations made in the region of said compartments (32, 37, 45; 132, 137, 145) being carried out by rotating the measuring supports (4, 5; 104, 105) round the central axis of centrifugation of said supports.

10. Process according to any one of Claims 1, 5, 8 and 9, **characterized in that**, during the pre-growth in an antibiogram measuring support (4; 104), the measurement of the concentration of bacterial inoculum and the determination of the beginning of growth are carried out by photometric measurement carried out on an aliquot part of the inoculum transferred for this purpose, by a first centrifugation in a specific peripheral compartment (37; 137) dedicated to the characterization of the growth during pre-incubation and not containing reagents, the remainder of inoculum remaining blocked during pre-incubation in a central concentric chamber (27; 127) of said support (4; 104), before being transferred, after completion of pre-growth, into the other peripheral compartments (32; 132) by a second centrifugation preceded by an appropriate action of authorization or unblocking, opening the passage for the transfer of the remainder of the inoculum to said other compartments (32; 132) containing reagents.

11. Process according to any one of Claims 1, 5, 9 and 10, **characterized in that** the passage from the pre-incubation stage to the incubation stage is carried out without breaking the confinement of the interior of the rotating measuring support concerned (4; 104) relative to the exterior, said support remaining closed and said passage being carried out by an action in the case of the support (4) or an interruption in an action in the case of the support (104) on a blocking element participating in the production of this confinement.

12. Process according to any one of Claims 1 to 11, **characterized in that** the measurements are taken by nephelometry in the case of measuring supports (4; 104) used for the antibiogram and by photometry or determination of absorbency or of optical density in the case of measuring supports (5; 105) used for the identification or for the combined identification/ antibiogram determinations, or by fluorimetry after prior addition of a fluorogenic marker to the reagent.

13. Disc-shaped measuring support comprising a rigid main body limiting, in the form of non-communicating enclosures, a central chamber and several associated peripheral compartments each having a latching niche, **characterized in that** said main body (126) has an upper wall (128) provided with several sets of traversing transfer orifices (129, 130, 131, 133) respectively opening into the cylindrical central chamber (127), in each latching niche (146) allocated to each compartment (132,145) and in each of said compartments (132, 145) and covered, with the exception of a central portion surrounding the charging orifice (134) of said central chamber (127) and of the zone of the measurement windows of the compartments (132, 145), by a snugly fitting sealed floating membrane (135) fixed locally and hermetically in particular at its external periphery and round the charging orifice (134) and being able to be pressed intimately against the upper surface of said upper wall (128) to form sealed temporary zones of contact (136, 138, 142) extending between or covering some of said sets of traversing orifices (129, 130, 131, 133), in particular during the rotation of said measuring support.

14. Measuring support according to Claim 13, **characterized in that** the upper wall (128) comprises, in the region of its upper face, concentric annular grooves or indentations (140, 140') in which there open the traversing orifices (129, 130, 131, 133) arranged in spaced concentric circles, the internal groove (140) comprising a rib (136) forming an annular contact surface and separating the outlet orifices (129) of the central chamber (127) and the inlet orifices (130) of the annular concentric chamber (147) comprising the radial, latching niches (146), and the outlet orifices (131) of the latching niches (146) opening in the region of the upper face of a concentric circular rib or of distinct protruding structures (138), for example in the form of domes, arranged in the external groove (140'), the upper ends of the rib (136) and of the structures (138) being located in a plane or planes extending slightly back from the plane of adhesion of the snugly fitting sealed membrane (135).

15. Measuring support according to any one of Claims 13 and 14, **characterized in that** it comprises a pre-incubation compartment (137) identical to the other peripheral compartments (132, 145), aligned circularly therewith and communicating with a closed pre-chamber (143) formed in the concentric chamber (147) enclosing the latching and equi-distribution niches (146) and supplied with inoculum from an internal latching niche (144) located in the region of a portion of the peripheral lateral wall (139) of the central chamber (127), by means of a passage not blockable by the sealed zones of contact (136, 138, 142) capable of being formed between the snugly fitting membrane (135) and the upper wall (128).

16. Measuring support according to Claim 15, **characterized in that** the supply passage of the pre-chamber (143), communicating freely with the pre-incubation compartment (137) and also forming an overflow for the latching niches (146), consists of a conduit (157) beginning in the upper part of the portion of peripheral lateral wall (139) limiting the internal latching niche (144) formed by cooperation between a groove made in the upper part of a radial projecting extension (158) of the peripheral lateral wall (139) of the central chamber (127) and of the upper wall (128), and opening into or at the inlet of a groove (148) with a centripetal radial extension dug in the upper face of the upper wall (128), forming a sealed conduit in cooperation with the membrane (135) stuck on either side of said groove (148) and of which the distal end terminates in the region of a traversing orifice (148) opening in the pre-chamber (143).

17. Measuring support according to any one of Claims 13 to 16, **characterized in that** the charging orifice (134) of the central chamber (127) is located in an indentation (134) in the upper wall (128) and is blockable by means of a stopper (150) of which the upper part is substantially level with the upper face of the upper wall (128), **in that** the bottom of the central chamber (127) is surrounded by a concentric gripping and rigidifying sleeve (151) projecting in the region of the lower face of the support (104, 105) and **in that** a traversing aperture (152) having dimensions identical to those of a compartment (132,145) and located in circular alignment with them, is made in the main body (126) and in the upper wall (128), said aperture (152) preferably being located adjacently to the pre-incubation compartment (137).

18. Measuring support according to any one of Claims 13 to 17, **characterized in that** the upper wall (128) is equipped, above each compartment (132, 137, 145) and close to its external edge, with a measuring window, a similar window being provided in each of the wall portions of the main body (126) respectively constituting the bottoms of said compartments opposite each aforementioned measuring window and at least one indexing reflecting zone formed on the external face of the external peripheral wall of the main body (126) being associated with each compartment (132, 137, 145) with an angular offset which is fixed relative thereto.

19. Apparatus for carrying out the process according to any one of Claims 1 to 12, essentially consisting of a first fresh module (102) for support and for storage of measuring supports (104, 105) according to any one of Claims 13 to 18 and of primary receivers (101) each cooperating with a corresponding sampling and transfer tool (101'), of a second module (103) for the incubation of the supports (104 and 105), after injection of the bacterial inoculum, of a third module (106) for centrifugation and nephelometric and/or photometric and/or fluorimetric measurement, of one or more transfer and handling devices (107, 107') serving said modules (102, 103 and 106) and of a computer (108) for monitoring and controlling the various stages of the process and acquiring, registering, processing, evaluating and/or restoring data, the second and third modules (103 and 106) being arranged in a confinement enclosure, the apparatus being **characterized in that** the primary receivers (101) are carried by a crown-shaped supporting means (109) extending concentrically round and beneath a disc-shaped supporting means (110) bearing the measuring supports (104, 105), the two supporting means (109, 110) being mounted with the ability to rotate round the same axis and **in that** each primary receiver (101) can be brought, by mere rotation of said crown-shaped supporting means (109), in the region of a station (111) for handling of the associated sampling and transfer tool (101') and for optical density measurement of the content of said primary receiver (101).

20. Apparatus according to Claim 19, **characterized in that** each primary receiver (101) for receiving a given volume of bacterial colony and for preparation of an at least precalibrated inoculum has the form of a cylindrical receiver which is open in the region of its upper part equipped with a radial rim (112) for holding said primary receiver (101) in one of the corresponding recesses (109) made in the crown-shaped moving support (109) and **in that** the sampling and transfer tool (101') consists of a syringe of which the body (113) comprises a frontal tapered part for sampling and ejection (113') and a substantially cylindrical rear part (113") for storage and gripping, said rear part (113") having at least one external radial offset (114) in the region of its end adjacent to the front part (113') for partial fitting with blocking of said body (113) in the primary receiver (101), the frontal free end of the frontal part (113') thus being located at a short distance from the bottom of said primary receiver (101).

21. Apparatus according to Claim 20, **characterized in that** the syringe (101) comprises, in the region of its front end, means (118, 118') for sampling a reduced quantity or a normal quantity of bacterial colony, by volume, as a function of the positioning of its piston (115).

22. Apparatus according to any one of Claims 19 to 21, **characterized in that** each primary receiver (101) comprises, in its lateral wall in the vicinity of its bottom, two opposing measuring windows (119, 119') adapted to measure the optical density of the content of said primary receiver (101) at the station (111).

23. Apparatus according to any one of Claims 20 to 22, **characterized in that** the handling means (121) of the syringe constituting the sampling tool (101') in the region of the station (111) consists of two sets of jaws (121' and 121") comprising two external jaws (121) intended to engage in a rigid, blocking manner with a radial rim (128) in the region of the aperture of the rear part (113") of the body (113) and two internal jaws (121") arranged concentrically between the two external jaws (121) intended to engage in a rigid and blocking manner with a swelling (122) of the actuating rod (116), the two sets of jaws (121', 121") being closed simultaneously by sliding a sleeve (121''') round and along the external jaws (121'), the internal jaws (121") being able to slide relative to the external jaws (121), in particular in the closure position, and to the sleeve (121''') and the latter being able to rest on the crown-shaped moving support (109) and on the radial edge (112) of the upper aperture of the considered primary receiver (101) so as to block it in position in its recess (109') during the sliding of any one of the two sets of jaws (121', 121").

24. Apparatus according to any one of Claims 19 to 23, **characterized in that** it comprises, on the one hand, a first handling and transfer device (107) serving the supports (109 and 110) and having the form of a handling arm mounted with the ability to rotate and slide on a first vertical shaft (123) and comprising two gripping jaws (124, 124) of which the first (124) is equipped with a means (125) for engagement of a primary receiver (101) and of which the second (124) has a curved shape or a hook intended to engage with two distinct points of a measuring support (104, 105) so as to hold the measuring support (104, 105) by propping it from below and by pinching in cooperation with the first gripping jaw (124) and, on the other hand, a second handling and transfer device (107) serving the incubation module (103) and the centrifugation and measuring module (106), having the form of a supporting arm mounted with the ability to rotate and slide on a second vertical shaft (123) and comprising at its free end a receiving means (125) mating with at least a part of the lower face of the measuring supports (104, 105), the two handling and transfer devices (107, 107) having an overlap zone for their respectively accessible spaces or manoeuvring zones in the region of which the measuring supports (104, 105) can in fact be transferred from one to the other.

25. Apparatus according to any one of Claims 19 to 24, **characterized in that** the third module (106) comprises several work stations (153), in particular for measurement and centrifugation, each comprising, in addition to the various means of measurement by nephelometry, by photometry and/or by fluorimetry, on the one hand, a disc-shaped supporting base (154) carried by the upper end of a rotating vertical shaft (154) capable of engaging with interlocking or frictional holding in the concentric sleeve (151) of a measuring support (104, 105) and, on the other hand, a substantially discoidal upper pressing device (155) set into rotation round its axis of symmetry by friction on the measuring support (104, 105), said pressing device (155) comprising one or more annular pressing members (156, 156', 156") and being able to be displaced reversibly from a high position in which said pressing members are not in contact with the measuring support (104, 105) arranged on the supporting base (154) to several lower positions in which they are successively brought to rest on the snugly fitting floating membrane (135) in a predetermined order (156", 156', 156), this membrane itself resting in the region of concentric circular or annular sealed contact zones or zones arranged in concentric circular or annular configurations with the upper wall (128) of the measuring support (104, 105) under consideration.

26. Apparatus for carrying out the process according to any one of Claims 5 to 12, essentially consisting of a first module for receiving and storing fresh and worn measuring supports (4 and/or 5), of a second module (3) for incubation of the supports (4 and/or 5), of a third module (6) for nephelometric and/or photometric and/or fluorimetric measurement, of one or more transfer devices (7, 7') serving the modules (2, 3 and 6) and of a computer (8) for monitoring and controlling the various stages of the process and for acquiring, registering, processing, evaluating and/or restoring data, **characterized in that** the first module (2) for receiving and storing fresh and worn measuring supports (4 and/or 5) also comprises at least one means (59) for receiving and storing primary receivers (1) containing the initial bacterial inoculum and a device (60, 61) for calibration of the inoculum in said primary receivers (1) and for preparation of said measuring supports (4 and/or 5).

27. Apparatus according to Claim 26, **characterized in that** the primary receiver (1) for receiving the inoculum has the form of a cylindrical receiver equipped on its bottom with at least one vent tube (9), with an overflow evacuation conduit (10), optionally with a conduit (10') for setting a lower volumetric level of inoculum, with a means for sampling a predetermined quantity of inoculum mounted separably by tearing or fitting in the bottom of the receiver (1) and eccentric relative to the axis of said receiver (1) such as a pipette (11) and with a trough-shaped transverse recess (12) extending between the walls of the receiver (1), which is provided at each end of said recess (12) with a measuring window (13), this recess (12) also being equipped with a raised part (12') communicating with said recess (12), the vent (9), the overflow conduit (10), the lowering conduit (10') and the sampling means (11) being closed in a sealed manner by a tearable film (14), a closure cover (15) cooperating by sealed conical fitting with the upper conical part (16) of said receiver (1).

28. Apparatus according to Claim 27, **characterized in that** the conical upper part (16) of the primary receiver (1) is provided with at least one shallow notch (17) and with two deeper notches (18) of different widths arranged in alignment with one another and at 90° to the axis passing through the shallow notch or notches (17), this or these shallow notches (17) being aligned in the axis of the recess (12) of the bottom and the deeper notches (18) cooperating with tabs (19) of corresponding section provided on the sealed closure cover (15) provided, on the one hand, on its upper face with a surface (20) extending in the axis of the tabs (19) and, on the other hand, with a conical wall (21) having a shape corresponding to that of the conical upper part (16) of the receiver (1) and connecting the tabs (19).

29. Apparatus according to Claim 26 employing a rotating support (4) for antibiogram, which has the form of an upper composite disc provided with peripheral compartments (32) for receiving the inoculum and antibiotic preparations, **characterized in that** said rotating measuring support (4) is provided with a concentric cylindrical body having a smaller diameter than the disc and connected to said disc, forming a central chamber (33) for receiving the inoculum for distribution by centrifugation, said chamber (33) being supplied through an orifice (23) in the upper face of the upper composite disc and being surrounded by a sleeve (39) for gripping and rigidifying the unit.

30. Apparatus according to any one of Claims 26 and 29, **characterized in that** the upper composite disc of the rotating support for antibiogram (4) consists, on the one hand, of a circular cover part (22) provided with an orifice (23) in its centre and, on the other hand, of a circular plate (31) of which the upper face turned toward the lower face of the cover part (22) carries, in relief on its periphery, compartments (32) for receiving the inoculum and antibiotic preparations, this circular plate (31) being connected to the concentric chamber (33) closed by a bottom (34), the cover part (22) being provided with a rim (22') extending concentrically above a circular wall (31') of the circular plate (31), limiting compartments in relief (32) toward the exterior and fixed to said circular wall (31') by adhesion or welding.

31. Apparatus according to Claim 30, **characterized in that** the circular plate (31) of the lower body part is provided, between the compartments in relief (32), with at least one pre-incubation compartment (37) connected, by means of a capillary tube (38), to a concentric chamber (27) made beneath the circular plate (22) of the cover part in the concentric chamber (33) of the circular plate (31) forming the lower body part.

32. Apparatus according to either of Claims 30 and 31, **characterized in that** the orifice (23) provided in the centre of the cover part (22) is arranged in a pit (25) extending concentrically within the concentric chamber (27) having a moving dish (28) provided with a dry culture medium, said moving dish (28) having a sealed guide wall (29) limiting a basin with said moving dish (28), the pit (25) being closed by a sealed film (26) covering the upper part of the circular cover plate and simultaneously limiting the capillary tube (38).

33. Apparatus according to Claim 26, employing a rotating support (5) for identification, having the form of an upper composite disc provided with peripheral compartments (45) for receiving the inoculum and characterizing reagent, **characterized in that** said rotating support (5) for identification is provided with a concentric cylindrical body of smaller diameter than the disc and connected to said disc, forming a concentric sleeve (46) for receiving the primary receiver (1) in the inverted position for the distribution by centrifugation of the inoculum, this concentric sleeve (46) extending, at the centre, beneath the lower face of the circular plate (44), being connected thereto and being equipped at its free end with a conical wall part (47) extending internally to said sleeve (46) and intended to cooperate with the external wall of the conical upper part (16) of the primary receiver (1) and being surrounded by a concentric sleeve (48) for gripping and rigidifying of the unit.

34. Apparatus according to Claim 33, **characterized in that** the upper composite disc of the rotating support for identification (5) consists, on the one hand, of a circular cover part (43) and, on the other hand, of a lower part in the form of a circular plate (44) provided, on its upper face turned toward the lower face of the cover part (43) in relief on its periphery, with compartments (45) for receiving the inoculum and characterizing reagents, the cover (43) being provided with a rim (43) extending concentrically above the circular wall (44') of the circular plate (44) limiting compartments in relief (45) toward the exterior and fixed to said circular wall (44) by adhesion or welding.

35. Apparatus according to any one of Claims 30 to 34, **characterized in that** means for sensing the inoculum (45") during centrifugation provided, as viewed in the radial direction, in front of each compartment (32, 45) respectively with supports (4 and 5) are each connected to the interior of a corresponding compartment (32, 45) by means of a capillary tube (36, 49) and are open furthermore into an internal space (30, 43") communicating with the concentric chamber (33) of the circular plate (31) forming the lower body part or with the space for receiving the primary receiver (1) in the concentric sleeve (46) extending beneath the lower face of the circular plate (44) forming the lower part of the composite disc, the compartments in relief (32, 45) being connected furthermore, each by means of a radial capillary tube (39, 50), to a circular capillary tube (40, 51) opening, via a further capillary tube (41, 52), to the exterior of the circular cover plate (22) or of the circular cover part (43), the pre-incubation compartment (37) provided on the measuring support (4) for antibiogram being connected directly, by a corresponding passage hole, to the circular capillary tube (40), these various capillary tubes being limited in the upper part of the circular plate (22) and of the cover (43) by the sealed film (26 or 52), the capillaries (41 and 52) communicating at their end closest to the central axis of the discs (31 and 44) with the internal chambers (27 and 43") by holes pierced in the circular plates (22 and 43).

36. Apparatus according to any one of Claims 30 to 32, 34 and 35, **characterized in that** the circular cover plate (22) and the circular cover (43) are also equipped, above each compartment (32, 45) and dose to its edge, with a measuring window (42, 53), a similar window (43, 54) being provided beneath the lower face of the circular plate (31, 44) forming the lower part of the body or of the composite disc opposite each measuring window (42, 53) and at least one indexing reflective zone (42', 53'), formed on the external face of the external peripheral wall of the supports (4,5), being associated with each compartment (32,45) with an angular offset which is fixed relative thereto.

37. Apparatus according to Claim 36, **characterized in that**, for the automatic introduction of an initial bacterial inoculum into a measuring support for antibiogram or simultaneous combined analysis, the primary receiver (1') has the form of a container provided, on the one hand, with a pipette (11) identical to that associated with the primary receiver (1) and, on the other hand, with a macropipette (73) for transferring the entire content of the receiver (1'), this macropipette being able to be detached from the primary receiver (1') used to empty its content into the support (4) then replaced into the primary receiver (1), the pipette (11) then being detached and used to block the support (4) while being deposited in the pit (25).
